# FASCICULE DE BREVET EUROPEEN

(11) **EP 0 839 194 B1**
(45) Date de publication et mention de la délivrance du brevet: **26.04.2006**
(21) Numéro de dépôt: 96925799.7
(22) Date de dépôt: 17.07.1996
(51) Int. Cl.: C12N 15/12, C07K 14/82, C12N 15/62, C12N 15/86, C07K 19/00, A61K 38/16, A61K 31/70, A61K 48/00

(54) **VARIANTS DE LA PROTEINE P53 ET UTILISATIONS THERAPEUTIQUES**
VARIANTEN DES P53-PROTEINS UND DEREN THERAPEUTISCHEN VERWENDUNGEN
PROTEIN P53 VARIANTS AND THEIR THERAPEUTIC USES

(30) Priorité: 19.07.1995 FR 9508729
(43) Date de publication de la demande: 06.05.1998
(73) Titulaire: Aventis Pharma S.A., 92160 Antony (FR)
(72) Inventeur: CONSEILLER, Emmanuel, F-75015 Paris (FR); BRACCO, Laurent, F-75013 Paris (FR)
(74) Mandataire: Savina, Jacques
(86) Numéro de dépôt international: PCT/FR1996/001111
(87) Numéro de publication internationale: WO 1997/004092

(56) Documents cités:
- WO-A-93/03160
- WO-A-94/12202
- WO-A-95/09916
- WO-A-95/16771
- WO-A-95/17213
- WO-A-96/16989
- PROCEEDINGS OF THE NATIONAL ACADEMY OF SCIENCES OF USA, vol. 91, WASHINGTON US, pages 1998-2002, XP002019922 PIETENPOL, J. ET AL.: "Sequence-specific transcriptional activation is essential for growth suppression by p53" cité dans la demande
- MOLECULAR AND CELLULAR BIOLOGY, vol. 13, no. 11, WASHINGTON US, pages 6849-6857, XP002019923 BROWN, D. ET AL.: "The tumor suppressor p53 and the oncoprotein simian virus 40 T antigen bind to overlapping domains on the MDM2 protein"
- MOLECULAR AND CELLULAR BIOLOGY, (1995 JAN) 15 (1) 497-504, XP000568946 WU, L. ET AL.: "Alternatively spliced forms in the carboxy-terminal domain of the p53 protein regulate its ability to promote annealing of complementary single strands of nucleic acids."
- GENE EXPRESSION, (1993) 3 (1) 95-107, XP000611818 REED, M. ET AL.: "p53 domains: suppression, transformation, and transactivation."
- EMBO JOURNAL, 15 (14) 3693-701., 15 Juillet 1996, XP002019925 ATTARDI, L. D. ET AL.: "Transcriptional activation by p53, but not induction of the p21 gene, is essential for oncogene-mediated apoptosis."
- SCIENCE, vol. 250, US, pages 1400-1403, XP002019926 HU, J. ET AL.: "Sequence requirements for coiled-coils: analysis with lambda repressor-GCN4 leucine zipper fusions"

### LISTE DE SEQUENCES

(1) INFORMATIONS GENERALES:
   (i) DEPOSANT:
      (A) NOM: RHONE POULENC RORER S.A.
      (B) RUE: 20, AVENUE RAYMOND ARON
      (C) VILLE: ANTONY
      (E) PAYS: FRANCE
      (F) CODE POSTAL: 92165
      (G) TELEPHONE: (1) 40.91.69.22
      (H) TELECOPIE: (1) 40.91.72.91
   (ii) TITRE DE L' INVENTION: Variants de la protéine p53 et utilisations thérapeutiques
   (iii) NOMBRE DE SEQUENCES: 46
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 112 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 1:
   AGATCTGAAG GCCCTCAAGG AGAAGCTGAA GGCCCTGGAG GAGAAGCTGA AGGCCCTGGA     60
   GGAGAAGCTG AAGGCACTAG TGGGGGAGCG ATGATGAATC GATATCGCGG CC            112
(2) INFORMATIONS POUR LA SEQ ID NO: 2:
   (i) CARACTERISTIQUES DE LA SEQUENCE:
      (A) LONGUEUR: 266 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 2:
   AAGCTTGAAT TCGTTAACAT GTCCACGGCC CCCCCGACCG ATGTCAGCCT GGGGGACGAG    60
   CTCCACTTAG ACGGCGAGGA CGTGGCGATG GCGCATGCCG ACGCGCTAGA CGATTTCGAT   120
   CTGGACATGT TGGGGGACGG GGATTCCCCG GGGCCGGGAT TTACCCCCCA CGACTCCGCC   180
   CCCTACGGCG CTCTGGATAT GGCCGACTTC GAGTTTGAGC AGATGTTTAC CGATGCCCTT   240
   GGAATTGACG AGTACGGTGG TCGACC                                        266
(2) INFORMATIONS POUR LA SEQ ID NO: 3:
   (i) CARACTERISTIQUES DE LA SEQUENCE:
      (A) LONGUEUR: 76 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 3:
   TCGAGCCTGC AGCCTAGAGC CTTCCAAGCC CTCATGAAGG AGGAAAGCCC AAACTGCTAG    60
   TGAGGATCCG CGGCCG                                                    76
(2) INFORMATIONS POUR LA SEQ ID NO: 4:
   (i) CARACTERISTIQUES DE LA SEQUENCE:
      (A) LONGUEUR: 788 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 4:
   GGGAAGCTTG GGCCGGGTCG ACCTGCACCA GCAGCTCCTA CACCGGCGGC CCCTGCACCA 60
   GCCCCCTCCT GGCCCCTGTC ATCTTCTGTC CCTTCCCAGA AAACCTACCA GGGCAGCTAC 120
   GGTTTCCGTC TGGGCTTCTT GCATTCTGGG ACAGCCAAGT CTGTGACTTG CACGTACTCC 180
   CCTGCCCTCA ACAAGATGTT TTGCCAACTG GCCAAGACCT GCCCTGTGCA GCTGTGGGTT 240
   GATTCCACAC CCCCGCCCGG CACCCGCGTC CGCGCCATGG CCATCTACAA GCAGTCACAG 300
   CACATGACGG AGGTTGTGAG GCGCTGCCCC CACCATGAGC GCTGCTCAGA TAGCGATGGT 360
   CTGGCCCCTC CTCAGCATCT TATCCGAGTG GAAGGAAATT TGCGTGTGGA GTATTTGGAT 420
   GACAGAAACA CTTTTCGACA TAGTGTGGTG GTGCCCTATG AGCCGCCTGA GGTTGGCTCT 480
   GACTGTACCA CCATCCACTA CAACTACATG TGTAACAGTT CCTGCATGGG CGGCATGAAC 540
   CGGAGGCCCA TCCTCACCAT CATCACACTG GAAGACTCCA GTGGTAATCT ACTGGGACGG 600
   AACAGCTTTG AGGTGCGTGT TTGTGCCTGT CCTGGGAGAG ACCGGCGCAC AGAGGAAGAG 660
   AATCTCCGCA AGAAAGGGGA GCCTCACCAC GAGCTGCCCC CAGGGAGCAC TAAGCGAGCA 720
   CTGCCCAACA ACACCAGCTC CTCTCCCCAG CCAAAGAAGA AACCACTGGA TGGGGATCCG 780
   CGGCCGCC 788
(2) INFORMATIONS POUR LA SEQ ID NO: 5:
   (i) CARACTERISTIQUES DE LA SEQUENCE:
      (A) LONGUEUR: 821 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 5:
   GGGAAGCTTG GGCCGGGTCG ACCTGCACCA GCAGCTCCTA CACCGGCGGC CCCTGCACCA 60
   GCCCCCTCCT GGCCCCTGTC ATCTTCTGTC CCTTCCCAGA AAACCTACCA GGGCAGCTAC 120
   GGTTTCCGTC TGGGCTTCTT GCATTCTGGG ACAGCCAAGT CTGTGACTTG CACGTACTCC 180
   CCTGCCCTCA ACAAGATGTT TTGCCAACTG GCCAAGACCT GCCCTGTGCA GCTGTGGGTT  240
   GATTCCACAC CCCCGCCCGG CACCCGCGTC CGCGCCATGG CCATCTACAA GCAGTCACAG  300
   CACATGACGG AGGTTGTGAG GCGCTGCCCC CACCATGAGC GCTGCTCAGA TAGCGATGGT  360
   CTGGCCCCTC CTCAGCATCT TATCCGAGTG GAAGGAAATT TGCGTGTGGA GTATTTGGAT  420
   GACAGAAACA CTTTTCGACA TAGTGTGGTG GTGCCCTATG AGCCGCCTGA GGTTGGCTCT  480
   GACTGTACCA CCATCCACTA CAACTACATG TGTAACAGTT CCTGCATGGG CGGCATGAAC  540
   CGGAGGCCCA TCCTCACCAT CATCACACTG GAAGACTCCA GTGGTAATCT ACTGGGACGG  600
   AACAGCTTTG AGGTGCGTGT TTGTGCCTGT CCTGGGAGAG ACCGGCGCAC AGAGGAAGAG  660
   AATCTCCGCA AGAAAGGGGA GCCTCACCAC GAGCTGCCCC CAGGGAGCAC TAAGCGAGCA  720
   CTGCCCAACA ACACCAGCTC CTCTCCCCAG CCAAAGAAGA AACCACTGGA TGGAGAATAT  780
   TTCACCCTTC AGATCCGTGG GCGTGAGGAT CCGCGGCCGC C                      821
(2) INFORMATIONS POUR LA SEQ ID NO: 6:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 6:
   ATGGAGGAGC CGCAG  15
(2) INFORMATIONS POUR LA SEQ ID NO: 7:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 7:
   GGCGGCCGCG ATATCGATTC ATCAGTCTGA GTCAGGCCCT TC  42
(2) INFORMATIONS POUR LA SEQ ID NO: 8:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 8:
   GGCGGCCGCG ATATCGATTC ATCAGCTCGA GTGAGC 36
(2) INFORMATIONS POUR LA SEQ ID NO: 9:
   (i) CARACTERISTIQUES DE LA SEQUENCE:
      (A) LONGUEUR: 43 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 9:
   TCGAGCCTGC AGCCTAGAGC CTTCCAAGCC CTCATGAAGG AGG 43
(2) INFORMATIONS POUR LA SEQ ID NO: 10:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 10:
   AAAGCCCAAA CTGCTGATGA ATCGATATCG C 31
(2) INFORMATIONS POUR LA SEQ ID NO: 11:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 11:
   TGAGGGCTTG GAAGGCTCTA GGCTGCAGGC 30
(2) INFORMATIONS POUR LA SEQ ID NO: 12:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 12:
   GGCCGCGATA TCGATTCATC AGCAGTTTGG GCTTTCCTCC TTCA 44
(2) INFORMATIONS POUR LA SEQ ID NO: 13:
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 13:
   GGGAAGCTTG GGCCGGGTCG ACCTGCACCA GCAGCTCCT 39
(2) INFORMATIONS POUR LA SEQ ID NO: 14:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 14:
   GGCGGCCGCG GATCCCCATC CAGTGGTTTC TT 32
(2) INFORMATIONS POUR LA SEQ ID NO: 15:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 15:
   ATCTGAATGG CGCTC 15
(2) INFORMATIONS POUR LA SEQ ID NO: 16:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 16:
   GGCGGCCGCG GATCCTCACG CCCACGGATC TG 32
(2) INFORMATIONS POUR LA SEQ ID NO: 17:
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 17:
   AAGCTTGAAT TCGTTAACAT GTCCACGGCC CCCCCGACC 39
(2) INFORMATIONS POUR LA SEQ ID NO: 18:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 18:
   GGTCGACCAC CGTACTCGTC AAT 23
(2) INFORMATIONS POUR LA SEQ ID NO: 19:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 19:
   GATCTGAAGG CCCTCAAGGA GAAGCTGAAG GCC 33
(2) INFORMATIONS POUR LA SEQ ID NO: 20:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 20:
   CTGGAGGAGA AGCTGAAGGC CCTGGAGGAG AAGCTG 36
(2) INFORMATIONS POUR LA SEQ ID NO: 21:
   (i) CARACTERISTIQUES DE LA SEQUENCE:
      (A) LONGUEUR: 38 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 21:
   AAGGCACTAG TGGGGGAGCG ATGATGAATC GATATCGC 38
(2) INFORMATIONS POUR LA SEQ ID NO: 22:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 22:
(2) INFORMATIONS POUR LA SEQ ID NO: 23:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 23:
(2) INFORMATIONS POUR LA SEQ ID NO: 24:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 24:
   GGCCGCGATA TCGATTCATC ATCGCTCCCC CAC                                    33
(2) INFORMATIONS POUR LA SEQ ID NO: 25:
   (i) CARACTERISTIQUES DE LA SEQUENCE:
      (A) LONGUEUR: 1095 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1095
   (xi) DESCRIPTION DE LA SEQUENCE: SEQ ID NO: 25:
   ATG TCC ACG GCC CCC CCG ACC GAT GTC AGC CTG GGG GAC GAG CTC CAC      48
   Met Ser Thr Ala Pro Pro Thr Asp Val Ser Leu Gly Asp Glu Leu His
     1               5                  10                  15
   TTA GAC GGC GAG GAC GTG GCG ATG GCG CAT GCC GAC GCG CTA GAC GAT      96
   Leu Asp Gly Glu Asp Val Ala Met Ala His Ala Asp Ala Leu Asp Asp
                20                  25                  30
   TTC GAT CTG GAC ATG TTG GGG GAC GGG GAT TCC CCG GGG CCG GGA TTT     144
   Phe Asp Leu Asp Met Leu Gly Asp Gly Asp Ser Pro Gly Pro Gly Phe
           35                  40                  45
   ACC CCC CAC GAC TCC GCC CCC TAC GGC GCT CTG GAT ATG GCC GAC TTC      192
   Thr Pro His Asp Ser Ala Pro Tyr Gly Ala Leu Asp Met Ala Asp Phe
       50                  55                  60
   GAG TTT GAG CAG ATG TTT ACC GAT GCC CTT GGA ATT GAC GAG TAC GGT      240
   Glu Phe Glu Gln Met Phe Thr Asp Ala Leu Gly Ile Asp Glu Tyr Gly
   65                  70                  75                  80
   GGT CGA CCT GCA CCA GCA GCT CCT ACA CCG GCG GCC CCT GCA CCA GCC      288
   Gly Arg Pro Ala Pro Ala Ala Pro Thr Pro Ala Ala Pro Ala Pro Ala
                   85                  90                  95
   CCC TCC TGG CCC CTG TCA TCT TCT GTC CCT TCC CAG AAA ACC TAC CAG      336
   Pro Ser Trp Pro Leu Ser Ser Ser Val Pro Ser Gln Lys Thr Tyr Gln
               100                 105                 110
   GGC AGC TAC GGT TTC CGT CTG GGC TTC TTG CAT TCT GGG ACA GCC AAG      384
   Gly Ser Tyr Gly Phe Arg Leu Gly Phe Leu His Ser Gly Thr Ala Lys
           115                 120                 125
   TCT GTG ACT TGC ACG TAC TCC CCT GCC CTC AAC AAG ATG TTT TGC CAA      432
   Ser Val Thr Cys Thr Tyr Ser Pro Ala Leu Asn Lys Met Phe Cys Gln
       130                 135                 140
   CTG GCC AAG ACC TGC CCT GTG CAG CTG TGG GTT GAT TCC ACA CCC CCG      480
   Leu Ala Lys Thr Cys Pro Val Gln Leu Trp Val Asp Ser Thr Pro Pro
   145                 150                 155                 160
   CCC GGC ACC CGC GTC CGC GCC ATG GCC ATC TAC AAG CAG TCA CAG CAC      528
   Pro Gly Thr Arg Val Arg Ala Met Ala Ile Tyr Lys Gln Ser Gln His
                   165                 170                 175
   ATG ACG GAG GTT GTG AGG CGC TGC CCC CAC CAT GAG CGC TGC TCA GAT      576
   Met Thr Glu Val Val Arg Arg Cys Pro His His Glu Arg Cys Ser Asp
               180                 185                 190
   AGC GAT GGT CTG GCC CCT CCT CAG CAT CTT ATC CGA GTG GAA GGA AAT      624
   Ser Asp Gly Leu Ala Pro Pro Gln His Leu Ile Arg Val Glu Gly Asn
           195                 200                 205
   TTG CGT GTG GAG TAT TTG GAT GAC AGA AAC ACT TTT CGA CAT AGT GTG      672
   Leu Arg Val Glu Tyr Leu Asp Asp Arg Asn Thr Phe Arg His Ser Val
       210                 215                 220
   GTG GTG CCC TAT GAG CCG CCT GAG GTT GGC TCT GAC TGT ACC ACC ATC      720
   Val Val Pro Tyr Glu Pro Pro Glu Val Gly Ser Asp Cys Thr Thr Ile
   225                 230                 235                 240
   CAC TAC AAC TAC ATG TGT AAC AGT TCC TGC ATG GGC GGC ATG AAC CGG      768
   His Tyr Asn Tyr Met Cys Asn Ser Ser Cys Met Gly Gly Met Asn Arg
                   245                 250                 255
   AGG CCC ATC CTC ACC ATC ATC ACA CTG GAA GAC TCC AGT GGT AAT CTA      816
   Arg Pro Ile Leu Thr Ile Ile Thr Leu Glu Asp Ser Ser Gly Asn Leu
               260                 265                 270
   CTG GGA CGG AAC AGC TTT GAG GTG CGT GTT TGT GCC TGT CCT GGG AGA      864
   Leu Gly Arg Asn Ser Phe Glu Val Arg Val Cys Ala Cys Pro Gly Arg
           275                 280                 285
   GAC CGG CGC ACA GAG GAA GAG AAT CTC CGC AAG AAA GGG GAG CCT CAC      912
   Asp Arg Arg Thr Glu Glu Glu Asn Leu Arg Lys Lys Gly Glu Pro His
       290                 295                 300
   CAC GAG CTG CCC CCA GGG AGC ACT AAG CGA GCA CTG CCC AAC AAC ACC      960
   His Glu Leu Pro Pro Gly Ser Thr Lys Arg Ala Leu Pro Asn Asn Thr
   305                 310                 315                 320
   AGC TCC TCT CCC CAG CCA AAG AAG AAA CCA CTG GAT GGG GAT CTG AAG     1008
   Ser Ser Ser Pro Gln Pro Lys Lys Lys Pro Leu Asp Gly Asp Leu Lys
                   325                 330                 335
   GCC CTC AAG GAG AAG CTG AAG GCC CTG GAG GAG AAG CTG AAG GCC CTG     1056
   Ala Leu Lys Glu Lys Leu Lys Ala Leu Glu Glu Lys Leu Lys Ala Leu
               340                 345                 350
   GAG GAG AAG CTG AAG GCA CTA GTG GGG GAG CGA TGA TGA                 1095
   Glu Glu Lys Leu Lys Ala Leu Val Gly Glu Arg  *   *
           355                 360                 365
(2) INFORMATIONS POUR LA SEQ ID NO: 26:
   (i) CARACTERISTIQUES DE LA SEQUENCE:
      (A) LONGUEUR: 1128 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1128
   (xi) DESCRIPTION DE LA SEQUENCE: SEQ ID NO: 26:
   ATG TCC ACG GCC CCC CCG ACC GAT GTC AGC CTG GGG GAC GAG CTC CAC       48
   Met Ser Thr Ala Pro Pro Thr Asp Val Ser Leu Gly Asp Glu Leu His
                   370                 375                 380
   TTA GAC GGC GAG GAC GTG GCG ATG GCG CAT GCC GAC GCG CTA GAC GAT       96
   Leu Asp Gly Glu Asp Val Ala Met Ala His Ala Asp Ala Leu Asp Asp
               385                 390                 395
   TTC GAT CTG GAC ATG TTG GGG GAC GGG GAT TCC CCG GGG CCG GGA TTT      144
   Phe Asp Leu Asp Met Leu Gly Asp Gly Asp Ser Pro Gly Pro Gly Phe
           400                 405                 410
   ACC CCC CAC GAC TCC GCC CCC TAC GGC GCT CTG GAT ATG GCC GAC TTC      192
   Thr Pro His Asp Ser Ala Pro Tyr Gly Ala Leu Asp Met Ala Asp Phe
       415                 420                     425
   GAG TTT GAG CAG ATG TTT ACC GAT GCC CTT GGA ATT GAC GAG TAC GGT      240
   Glu Phe Glu Gln Met Phe Thr Asp Ala Leu Gly Ile Asp Glu Tyr Gly
   430                 435                     440                 445
   GGT CGA CCT GCA CCA GCA GCT CCT ACA CCG GCG GCC CCT GCA CCA GCC      288
   Gly Arg Pro Ala Pro Ala Ala Pro Thr Pro Ala Ala Pro Ala Pro Ala
                   450                     455                 460
   CCC TCC TGG CCC CTG TCA TCT TCT GTC CCT TCC CAG AAA ACC TAC CAG      336
   Pro Ser Trp Pro Leu Ser Ser Ser Val Pro Ser Gln Lys Thr Tyr Gln
               465                 470                     475
   GGC AGC TAC GGT TTC CGT CTG GGC TTC TTG CAT TCT GGG ACA GCC AAG      384
   Gly Ser Tyr Gly Phe Arg Leu Gly Phe Leu His Ser Gly Thr Ala Lys
           480                 485                     490
   TCT GTG ACT TGC ACG TAC TCC CCT GCC CTC AAC AAG ATG TTT TGC CAA      432
   Ser Val Thr Cys Thr Tyr Ser Pro Ala Leu Asn Lys Met Phe Cys Gln
       495                 500                     505
   CTG GCC AAG ACC TGC CCT GTG CAG CTG TGG GTT GAT TCC ACA CCC CCG      480
   Leu Ala Lys Thr Cys Pro Val Gln Leu Trp Val Asp Ser Thr Pro Pro
   510                 515                     520                 525
   CCC GGC ACC CGC GTC CGC GCC ATG GCC ATC TAC AAG CAG TCA CAG CAC      528
   Pro Gly Thr Arg Val Arg Ala Met Ala Ile Tyr Lys Gln Ser Gln His
                   530                     535                 540
   ATG ACG GAG GTT GTG AGG CGC TGC CCC CAC CAT GAG CGC TGC TCA GAT      576
   Met Thr Glu Val Val Arg Arg Cys Pro His His Glu Arg Cys Ser Asp
               545                 550                     555
   AGC GAT GGT CTG GCC CCT CCT CAG CAT CTT ATC CGA GTG GAA GGA AAT      624
   Ser Asp Gly Leu Ala Pro Pro Gln His Leu Ile Arg Val Glu Gly Asn
           560                 565                     570
   TTG CGT GTG GAG TAT TTG GAT GAC AGA AAC ACT TTT CGA CAT AGT GTG      672
   Leu Arg Val Glu Tyr Leu Asp Asp Arg Asn Thr Phe Arg His Ser Val
       575                 580                     585
   GTG GTG CCC TAT GAG CCG CCT GAG GTT GGC TCT GAC TGT ACC ACC ATC      720
   Val Val Pro Tyr Glu Pro Pro Glu Val Gly Ser Asp Cys Thr Thr Ile
   590                 595                     600                 605
   CAC TAC AAC TAC ATG TGT AAC AGT TCC TGC ATG GGC GGC ATG AAC CGG      768
   His Tyr Asn Tyr Met Cys Asn Ser Ser Cys Met Gly Gly Met Asn Arg
                   610                     615                 620
   AGG CCC ATC CTC ACC ATC ATC ACA CTG GAA GAC TCC AGT GGT AAT CTA      816
   Arg Pro Ile Leu Thr Ile Ile Thr Leu Glu Asp Ser Ser Gly Asn Leu
               625                 630                     635
   CTG GGA CGG AAC AGC TTT GAG GTG CGT GTT TGT GCC TGT CCT GGG AGA      864
   Leu Gly Arg Asn Ser Phe Glu Val Arg Val Cys Ala Cys Pro Gly Arg
           640                 645                     650
   GAC CGG CGC ACA GAG GAA GAG AAT CTC CGC AAG AAA GGG GAG CCT CAC      912
   Asp Arg Arg Thr Glu Glu Glu Asn Leu Arg Lys Lys Gly Glu Pro His
       655                 660                 665
   CAC GAG CTG CCC CCA GGG AGC ACT AAG CGA GCA CTG CCC AAC AAC ACC      960
   His Glu Leu Pro Pro Gly Ser Thr Lys Arg Ala Leu Pro Asn Asn Thr
   670                 675                 680                 685
   AGC TCC TCT CCC CAG CCA AAG AAG AAA CCA CTG GAT GGA GAA TAT TTC     1008
   Ser Ser Ser Pro Gln Pro Lys Lys Lys Pro Leu Asp Gly Glu Tyr Phe
                   690                 695                 700
   ACC CTT CAG ATC CGT GGG CGT GAG GAT CTG AAG GCC CTC AAG GAG AAG     1056
   Thr Leu Gln Ile Arg Gly Arg Glu Asp Leu Lys Ala Leu Lys Glu Lys
               705                 710                 715
   CTG AAG GCC CTG GAG GAG AAG CTG AAG GCC CTG GAG GAG AAG CTG AAG     1104
   Leu Lys Ala Leu Glu Glu Lys Leu Lys Ala Leu Glu Glu Lys Leu Lys
           720                 725                 730
   GCA CTA GTG GGG GAG CGA TGA TGA                                     1128
   Ala Leu Val Gly Glu Arg  *   *
       735                 740
(2) INFORMATIONS POUR LA SEQ ID NO: 27:
   (i) CARACTERISTIQUES DE LA SEQUENCE:
      (A) LONGUEUR: 765 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..765
   (xi) DESCRIPTION DE LA SEQUENCE: SEQ ID NO: 27:
   ATG TCC ACG GCC CCC CCG ACC GAT GTC AGC CTG GGG GAC GAG CTC CAC       48
   Met Ser Thr Ala Pro Pro Thr Asp Val Ser Leu Gly Asp Glu Leu His
               380                 385                 390
   TTA GAC GGC GAG GAC GTG GCG ATG GCG CAT GCC GAC GCG CTA GAC GAT       96
   Leu Asp Gly Glu Asp Val Ala Met Ala His Ala Asp Ala Leu Asp Asp
           395                 400                 405
   TTC GAT CTG GAC ATG TTG GGG GAC GGG GAT TCC CCG GGG CCG GGA TTT      144
   Phe Asp Leu Asp Met Leu Gly Asp Gly Asp Ser Pro Gly Pro Gly Phe
       410                 415                 420
   ACC CCC CAC GAC TCC GCC CCC TAC GGC GCT CTG GAT ATG GCC GAC TTC      192
   Thr Pro His Asp Ser Ala Pro Tyr Gly Ala Leu Asp Met Ala Asp Phe
   425                 430                 435                 440
   GAG TTT GAG CAG ATG TTT ACC GAT GCC CTT GGA ATT GAC GAG TAC GGT      240
   Glu Phe Glu Gln Met Phe Thr Asp Ala Leu Gly Ile Asp Glu Tyr Gly
                   445                 450                 455
   GGT CGA CCT GCA CCA GCA GCT CCT ACA CCG GCG GCC CCT GCA CCA GCC      288
   Gly Arg Pro Ala Pro Ala Ala Pro Thr Pro Ala Ala Pro Ala Pro Ala
               460                 465                 470
   CCC TCC TGG CCC CTG TCA TCT TCT GTC CCT TCC CAG AAA ACC TAC CAG      336
   Pro Ser Trp Pro Leu Ser Ser Ser Val Pro Ser Gln Lys Thr Tyr Gln
           475                 480                 485
   GGC AGC TAC GGT TTC CGT CTG GGC TTC TTG CAT TCT GGG ACA GCC AAG      384
   Gly Ser Tyr Gly Phe Arg Leu Gly Phe Leu His Ser Gly Thr Ala Lys
       490                 495                 500
   TCT GTG ACT TGC ACG TAC TCC CCT GCC CTC AAC AAG ATG TTT TGC CAA      432
   Ser Val Thr Cys Thr Tyr Ser Pro Ala Leu Asn Lys Met Phe Cys Gln
   505                 510                 515                 520
   CTG GCC AAG ACC TGC CCT GTG CAG CTG TGG GTT GAT TCC ACA CCC CCG      480
   Leu Ala Lys Thr Cys Pro Val Gln Leu Trp Val Asp Ser Thr Pro Pro
                   525                 530                 535
   CCC GGC ACC CGC GTC CGC GCC ATG GCC ATC TAC AAG CAG TCA CAG CAC      528
   Pro Gly Thr Arg Val Arg Ala Met Ala Ile Tyr Lys Gln Ser Gln His
               540                 545                 550
   ATG ACG GAG GTT GTG AGG CGC TGC CCC CAC CAT GAG CGC TGC TCA GAT      576
   Met Thr Glu Val Val Arg Arg Cys Pro His His Glu Arg Cys Ser Asp
           555                 560                 565
   AGC GAT GGT CTG GCC CCT CCT CAG CAT CTT ATC CGA GTG GAA GGA AAT      624
   Ser Asp Gly Leu Ala Pro Pro Gln His Leu Ile Arg Val Glu Gly Asn
       570                 575                 580
   TTG CGT GTG GAG TAT TTC ACC CTT CAG ATC CGT GGG CGT GAG CGC TTC      672
   Leu Arg Val Glu Tyr Phe Thr Leu Gln Ile Arg Gly Arg Glu Arg Phe
   585                 590                 595                 600
   GAG ATG TTC CGA GAG CTG AAT GAG GCC TTG GAA CTC AAG GAT GCC CAG      720
   Glu Met Phe Arg Glu Leu Asn Glu Ala Leu Glu Leu Lys Asp Ala Gln
                   605                 610                 615
   GCT GGG AAG GAG CCA GGG GGG AGC AGG GCT CAC TCG AGC TGA TGA          765
   Ala Gly Lys Glu Pro Gly Gly Ser Arg Ala His Ser Ser  *   *
               620                 625                 630
(2) INFORMATIONS POUR LA SEQ ID NO: 28:
   (i) CARACTERISTIQUES DE LA SEQUENCE:
      (A) LONGUEUR: 816 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..816
   (xi) DESCRIPTION DE LA SEQUENCE: SEQ ID NO: 28:
   ATG TCC ACG GCC CCC CCG ACC GAT GTC AGC CTG GGG GAC GAG CTC CAC      48
   TTA GAC GGC GAG GAC GTG GCG ATG GCG CAT GCC GAC GCG CTA GAC GAT      96
   TTC GAT CTG GAC ATG TTG GGG GAC GGG GAT TCC CCG GGG CCG GGA TTT     144
   ACC CCC CAC GAC TCC GCC CCC TAC GGC GCT CTG GAT ATG GCC GAC TTC     192
   GAG TTT GAG CAG ATG TTT ACC GAT GCC CTT GGA ATT GAC GAG TAC GGT     240
   GGT CGA CCT GCA CCA GCA GCT CCT ACA CCG GCG GCC CCT GCA CCA GCC     288
   CCC TCC TGG CCC CTG TCA TCT TCT GTC CCT TCC CAG AAA ACC TAC CAG     336
   GGC AGC TAC GGT TTC CGT CTG GGC TTC TTG CAT TCT GGG ACA GCC AAG     384
   TCT GTG ACT TGC ACG TAC TCC CCT GCC CTC AAC AAG ATG TTT TGC CAA     432
   CTG GCC AAG ACC TGC CCT GTG CAG CTG TGG GTT GAT TCC ACA CCC CCG     480
   CCC GGC ACC CGC GTC CGC GCC ATG GCC ATC TAC AAG CAG TCA CAG CAC     528
   ATG ACG GAG GTT GTG AGG CGC TGC CCC CAC CAT GAG CGC TGC TCA GAT        576
   AGC GAT GGT CTG GCC CCT CCT CAG CAT CTT ATC CGA GTG GAA GGA AAT        624
   TTG CGT GTG GAG TAT TTC ACC CTT CAG ATC CGT GGG CGT GAG CGC TTC        672
   GAG ATG TTC CGA GAG CTG AAT GAG GCC TTG GAA CTC AAG GAT GCC CAG        720
   GCT GGG AAG GAG CCA GGG GGG AGC AGG GCT CAC TCG AGC CTG CAG CCT        768
   AGA GCC TTC CAA GCC CTC ATG AAG GAG GAA AGC CCA AAC TGC TGA TGA        816
(2) INFORMATIONS POUR LA SEQ ID NO: 29:
   (i) CARACTERISTIQUES DE LA SEQUENCE:
      (A) LONGUEUR: 1209 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1209
   (xi) DESCRIPTION DE LA SEQUENCE: SEQ ID NO: 29:
   ATG TCC ACG GCC CCC CCG ACC GAT GTC AGC CTG GGG GAC GAG CTC CAC         48
   Met Ser Thr Ala Pro Pro Thr Asp Val Ser Leu Gly Asp Glu Leu His
           275                     280                     285
   TTA GAC GGC GAG GAC GTG GCG ATG GCG CAT GCC GAC GCG CTA GAC GAT         96
   Leu Asp Gly Glu Asp Val Ala Met Ala His Ala Asp Ala Leu Asp Asp
       290                     295                     300
   TTC GAT CTG GAC ATG TTG GGG GAC GGG GAT TCC CCG GGG CCG GGA TTT        144
   Phe Asp Leu Asp Met Leu Gly Asp Gly Asp Ser Pro Gly Pro Gly Phe
   305                 310                 315                 320
   ACC CCC CAC GAC TCC GCC CCC TAC GGC GCT CTG GAT ATG GCC GAC TTC      192
   Thr Pro His Asp Ser Ala Pro Tyr Gly Ala Leu Asp Met Ala Asp Phe
                   325                 330                 335
   GAG TTT GAG CAG ATG TTT ACC GAT GCC CTT GGA ATT GAC GAG TAC GGT      240
   Glu Phe Glu Gln Met Phe Thr Asp Ala Leu Gly Ile Asp Glu Tyr Gly
               340                 345                 350
   GGT CGA CCT GCA CCA GCA GCT CCT ACA CCG GCG GCC CCT GCA CCA GCC      288
   Gly Arg Pro Ala Pro Ala Ala Pro Thr Pro Ala Ala Pro Ala Pro Ala
           355                 360                 365
   CCC TCC TGG CCC CTG TCA TCT TCT GTC CCT TCC CAG AAA ACC TAC CAG      336
   Pro Ser Trp Pro Leu Ser Ser Ser Val Pro Ser Gln Lys Thr Tyr Gln
       370                 375                 380
   GGC AGC TAC GGT TTC CGT CTG GGC TTC TTG CAT TCT GGG ACA GCC AAG      384
   Gly Ser Tyr Gly Phe Arg Leu Gly Phe Leu His Ser Gly Thr Ala Lys
   385                 390                 395                 400
   TCT GTG ACT TGC ACG TAC TCC CCT GCC CTC AAC AAG ATG TTT TGC CAA      432
   Ser Val Thr Cys Thr Tyr Ser Pro Ala Leu Asn Lys Met Phe Cys Gln
                   405                 410                 415
   CTG GCC AAG ACC TGC CCT GTG CAG CTG TGG GTT GAT TCC ACA CCC CCG      480
   Leu Ala Lys Thr Cys Pro Val Gln Leu Trp Val Asp Ser Thr Pro Pro
               420                 425                 430
   CCC GGC ACC CGC GTC CGC GCC ATG GCC ATC TAC AAG CAG TCA CAG CAC      528
   Pro Gly Thr Arg Val Arg Ala Met Ala Ile Tyr Lys Gln Ser Gln His
           435                 440                 445
   ATG ACG GAG GTT GTG AGG CGC TGC CCC CAC CAT GAG CGC TGC TCA GAT      576
   Met Thr Glu Val Val Arg Arg Cys Pro His His Glu Arg Cys Ser Asp
       450                 455                 460
   AGC GAT GGT CTG GCC CCT CCT CAG CAT CTT ATC CGA GTG GAA GGA AAT      624
   Ser Asp Gly Leu Ala Pro Pro Gln His Leu Ile Arg Val Glu Gly Asn
   465                 470                 475                 480
   TTG CGT GTG GAG TAT TTG GAT GAC AGA AAC ACT TTT CGA CAT AGT GTG      672
   Leu Arg Val Glu Tyr Leu Asp Asp Arg Asn Thr Phe Arg His Ser Val
                   485                 490                 495
   GTG GTG CCC TAT GAG CCG CCT GAG GTT GGC TCT GAC TGT ACC ACC ATC      720
   Val Val Pro Tyr Glu Pro Pro Glu Val Gly Ser Asp Cys Thr Thr Ile
               500                 505                 510
   CAC TAC AAC TAC ATG TGT AAC AGT TCC TGC ATG GGC GGC ATG AAC CGG      768
   His Tyr Asn Tyr Met Cys Asn Ser Ser Cys Met Gly Gly Met Asn Arg
           515                 520                 525
   AGG CCC ATC CTC ACC ATC ATC ACA CTG GAA GAC TCC AGT GGT AAT CTA      816
   Arg Pro Ile Leu Thr Ile Ile Thr Leu Glu Asp Ser Ser Gly Asn Leu
       530                 535                 540
   CTG GGA CGG AAC AGC TTT GAG GTG CGT GTT TGT GCC TGT CCT GGG AGA      864
   Leu Gly Arg Asn Ser Phe Glu Val Arg Val Cys Ala Cys Pro Gly Arg
   545                 550                 555                 560
   GAC CGG CGC ACA GAG GAA GAG AAT CTC CGC AAG AAA GGG GAG CCT CAC      912
   Asp Arg Arg Thr Glu Glu Glu Asn Leu Arg Lys Lys Gly Glu Pro His
                   565                 570                 575
   CAC GAG CTG CCC CCA GGG AGC ACT AAG CGA GCA CTG CCC AAC AAC ACC      960
   His Glu Leu Pro Pro Gly Ser Thr Lys Arg Ala Leu Pro Asn Asn Thr
               580                 585                 590
   AGC TCC TCT CCC CAG CCA AAG AAG AAA CCA CTG GAT GGA GAA TAT TTC     1008
   Ser Ser Ser Pro Gln Pro Lys Lys Lys Pro Leu Asp Gly Glu Tyr Phe
           595                 600                 605
   ACC CTT CAG ATC CGT GGG CGT GAG CGC TTC GAG ATG TTC CGA GAG CTG     1056
   Thr Leu Gln Ile Arg Gly Arg Glu Arg Phe Glu Met Phe Arg Glu Leu
       610                 615                 620
   AAT GAG GCC TTG GAA CTC AAG GAT GCC CAG GCT GGG AAG GAG CCA GGG     1104
   Asn Glu Ala Leu Glu Leu Lys Asp Ala Gln Ala Gly Lys Glu Pro Gly
   625                 630                 635                 640
   GGG AGC AGG GCT CAC TCC AGC CAC CTG AAG TCC AAA AAG GGT CAG TCT     1152
   Gly Ser Arg Ala His Ser Ser His Leu Lys Ser Lys Lys Gly Gln Ser
                   645                 650                 655
   ACC TCC CGC CAT AAA AAA CTC ATG TTC AAG ACA GAA GGG CCT GAC TCA     1200
   Thr Ser Arg His Lys Lys Leu Met Phe Lys Thr Glu Gly Pro Asp Ser
               660                 665                 670
   GAC TGA TGA                                                         1209
   Asp  *   *
           675
(2) INFORMATIONS POUR LA SEQ ID NO: 30:
   (i) CARACTERISTIQUES DE LA SEQUENCE:
      (A) LONGUEUR: 1149 paires de bases
      (B) TYPE: nucl,otide
      (C) NOMBRE DE BRINS: simple
      (D) CONFIGURATION: lin,aire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1149
   (xi) DESCRIPTION DE LA SEQUENCE: SEQ ID NO: 30:
   ATG GCC ACG GCC CCC CCG ACC GAT GTC AGC CTG GGG GAC GAG CTC CAC       48
   Met Ala Thr Ala Pro Pro Thr Asp Val Ser Leu Gly Asp Glu Leu His
    1               5                  10                  15
   TTA GAC GGC GAG GAC GTG GCG ATG GCG CAT GCC GAC GCG CTA GAC GAT      96
   Leu Asp Gly Glu Asp Val Ala Met Ala His Ala Asp Ala Leu Asp Asp
               20                  25                  30
   TTC GAT CTG GAC ATG TTG GGG GAC GGG GAT TCC CCG GGG CCG GGA TTT     144
   Phe Asp Leu Asp Met Leu Gly Asp Gly Asp Ser Pro Gly Pro Gly Phe
           35                  40                  45
   ACC CCC CAC GAC TCC GCC CCC TAC GGC GCT CTG GAT ATG GCC GAC TTC     192
   Thr Pro His Asp Ser Ala Pro Tyr Gly Ala Leu Asp Met Ala Asp Phe
       50                  55                  60
   GAG TTT GAG CAG ATG TTT ACC GAT GCC CTT GGA ATT GAC GAG TAC GGT     240
   Glu Phe Glu Gln Met Phe Thr Asp Ala Leu Gly Ile Asp Glu Tyr Gly
   65                  70                  75                  80
   GGT CGA CCT GCA CCA GCA GCT CCT ACA CCG GCG GCC CCT GCA CCA GCC     288
   Gly Arg Pro Ala Pro Ala Ala Pro Thr Pro Ala Ala Pro Ala Pro Ala
                   85                  90                  95
   CCC TCC TGG CCC CTG TCA TCT TCT GTC CCT TCC CAG AAA ACC TAC CAG     336
   Pro Ser Trp Pro Leu Ser Ser Ser Val Pro Ser Gln Lys Thr Tyr Gln
               100                 105                 110
   GGC AGC TAC GGT TTC CGT CTG GGC TTC TTG CAT TCT GGG ACA GCC AAG     384
   Gly Ser Tyr Gly Phe Arg Leu Gly Phe Leu His Ser Gly Thr Ala Lys
           115                 120                 125
   TCT GTG ACT TGC ACG TAC TCC CCT GCC CTC AAC AAG ATG TTT TGC CAA     432
   Ser Val Thr Cys Thr Tyr Ser Pro Ala Leu Asn Lys Met Phe Cys Gln
       130                 135                 140
   CTG GCC AAG ACC TGC CCT GTG CAG CTG TGG GTT GAT TCC ACA CCC CCG     480
   Leu Ala Lys Thr Cys Pro Val Gln Leu Trp Val Asp Ser Thr Pro Pro
   145                 150                 155                 160
   CCC GGC ACC CGC GTC CGC GCC ATG GCC ATC TAC AAG CAG TCA CAG CAC     528
   Pro Gly Thr Arg Val Arg Ala Met Ala Ile Tyr Lys Gln Ser Gln His
                   165                 170                 175
   ATG ACG GAG GTT GTG AGG CGC TGC CCC CAC CAT GAG CGC TGC TCA GAT     576
   Met Thr Glu Val Val Arg Arg Cys Pro His His Glu Arg Cys Ser Asp
               180                 185                 190
   AGC GAT GGT CTG GCC CCT CCT CAG CAT CTT ATC CGA GTG GAA GGA AAT     624
   Ser Asp Gly Leu Ala Pro Pro Gln His Leu Ile Arg Val Glu Gly Asn
           195                 200                 205
   TTG CGT GTG GAG TAT TTG GAT GAC AGA AAC ACT TTT CGA CAT AGT GTG     672
   Leu Arg Val Glu Tyr Leu Asp Asp Arg Asn Thr Phe Arg His Ser Val
       210                 215                 220
   GTG GTG CCC TAT GAG CCG CCT GAG GTT GGC TCT GAC TGT ACC ACC ATC     720
   Val Val Pro Tyr Glu Pro Pro Glu Val Gly Ser Asp Cys Thr Thr Ile
   225                 230                 235                 240
   CAC TAC AAC TAC ATG TGT AAC AGT TCC TGC ATG GGC GGC ATG AAC CGG     768
   His Tyr Asn Tyr Met Cys Asn Ser Ser Cys Met Gly Gly Met Asn Arg
                   245                 250                 255
   AGG CCC ATC CTC ACC ATC ATC ACA CTG GAA GAC TCC AGT GGT AAT CTA     816
   Arg Pro Ile Leu Thr Ile Ile Thr Leu Glu Asp Ser Ser Gly Asn Leu
               260                 265                 270
   CTG GGA CGG AAC AGC TTT GAG GTG CGT GTT TGT GCC TGT CCT GGG AGA     864
   Leu Gly Arg Asn Ser Phe Glu Val Arg Val Cys Ala Cys Pro Gly Arg
           275                 280                 285
   GAC CGG CGC ACA GAG GAA GAG AAT CTC CGC AAG AAA GGG GAG CCT CAC     912
   Asp Arg Arg Thr Glu Glu Glu Asn Leu Arg Lys Lys Gly Glu Pro His
       290                 295                 300
   CAC GAG CTG CCC CCA GGG AGC ACT AAG CGA GCA CTG CCC AAC AAC ACC     960
   His Glu Leu Pro Pro Gly Ser Thr Lys Arg Ala Leu Pro Asn Asn Thr
   305                 310                 315                 320
   AGC TCC TCT CCC CAG CCA AAG AAG AAA CCA CTG GAT GGA GAA TAT TTC    1008
   Ser Ser Ser Pro Gln Pro Lys Lys Lys Pro Leu Asp Gly Glu Tyr Phe
                   325                 330                 335
   ACC CTT CAG ATC CGT GGG CGT GAG CGC TTC GAG ATG TTC CGA GAG GAT    1056
   Thr Leu Gln Ile Arg Gly Arg Glu Arg Phe Glu Met Phe Arg Glu Asp
               340                 345                 350
   CTG AAG GCC CTC AAG GAG AAG CTG AAG GCC CTG GAG GAG AAG CTG AAG    1104
   Leu Lys Ala Leu Lys Glu Lys Leu Lys Ala Leu Glu Glu Lys Leu Lys
           355                 360                 365
   GCC CTG GAG GAG AAG CTG AAG GCA CTA GTG GGG GAG CGA TGA TGA        1149
   Ala Leu Glu Glu Lys Leu Lys Ala Leu Val Gly Glu Arg  *   *
       370                 375                 380
(2) INFORMATIONS POUR LA SEQ ID NO: 31:
   (I) CARACTERISTIQUES DE LA SEQUENCE:
      (A) LONGUEUR: 1611 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (IX) CARACTERISTIQUE:
      (A) NOM/CLE: CDS
      (B) EMPLACEMENT:1..1611
   (XI) DESCRIPTION DE LA SEQUENCE: SEQ ID NO: 31:
   ATG GCC CAG GTG CAG CTG CAG GAG TCA GGG GCA GAG CTT GTG GGG TCA      48
   MET ALA GLN VAL GLN LEU GLN GLU SER GLY ALA GLU LEU VAL GLY SER
    1               5                  10                  15
   GGG GCC TCA GTC AAG TTG TCC TGC ACA GCT TCT GGC TTC AAC ATT AAA      96
   GLY ALA SER VAL LYS LEU SER CYS THR ALA SER GLY PHE ASN ILE LYS
               20                  25                  30
   GAC TAC TAT ATG CAC TGG GTG AAG CAG AGG CCT GAA CAG GGC CTG GAG     144
   ASP TYR TYR MET HIS TRP VAL LYS GLN ARG PRO GLU GLN GLY LEU GLU
           35                  40                  45
   TGG ATT GGA TGG ATT GAT CCT GAG AAT GGT GAT ACT GAA TAT GCC CCG     192
   TRP ILE GLY TRP ILE ASP PRO GLU ASN GLY ASP THR GLU TYR ALA PRO
       50                  55                  60
   AAG TTC CAG GGC AAG GCC ACT ATG ACT GCA GAC ACA TCC TCC AAT ACA     240
   LYS PHE GLN GLY LYS ALA THR MET THR ALA ASP THR SER SER ASN THR
   65                  70                  75                  80
   GCC TAC CTG CAG CTC AGC AGC CTG GCA TCT GAG GAC ACT GCC GTC TAT     288
   ALA TYR LEU GLN LEU SER SER LEU ALA SER GLU ASP THR ALA VAL TYR
                   85                  90                  95
   TAT TGT AAT TTT TAC GGG GAT GCT TTG GAC TAC TGG GGC CAA GGG ACC     336
   TYR CYS ASN PHE TYR GLY ASP ALA LEU ASP TYR TRP GLY GLN GLY THR
               100                 105                 110
   ACG GTC ACC GTC TCC TCA GGT GGA GGC GGT TCA GGC GGA GGT GGC TCT     384
   THR VAL THR VAL SER SER GLY GLY GLY GLY SER GLY GLY GLY GLY SER
           115                 120                 125
   GGC GGT GGC GGA TCG GAT GTT TTG ATG ACC CAA ACT CCA CTC ACT TTG     432
   GLY GLY GLY GLY SER ASP VAL LEU MET THR GLN THR PRO LEU THR LEU
       130                 135                 140
   TCG GTT ACC ATT GGA CAA CCA GCC TCC ATC TCT TGC AAG TCA AGT CAG     480
   SER VAL THR ILE GLY GLN PRO ALA SER ILE SER CYS LYS SER SER GLN
   145                 150                 155                 160
   AGC CTC TTG GAT AGT GAT GGA AAG ACA TAT TTG AAT TGG TTG TTA CAG     528
   SER LEU LEU ASP SER ASP GLY LYS THR TYR LEU ASN TRP LEU LEU GLN
                   165                 170                 175
   AGG CCA GGC CAG TCT CCA AAG CGC CTA ATC TAT CTG GTG TCT AAA CTG     576
   ARG PRO GLY GLN SER PRO LYS ARG LEU ILE TYR LEU VAL SER LYS LEU
               180                 185                 190
   GAC TCT GGA GTC CCT GAC AGG TTC ACT GGC AGT GGA TCA GGG ACA GAT     624
   ASP SER GLY VAL PRO ASP ARG PHE THR GLY SER GLY SER GLY THR ASP
           195                 200                 205
   TTC ACA CTG AAA ATC AAC AGA GTG GAG GCT GAG GAT TTG GGA GTT TAT     672
   PHE THR LEU LYS ILE ASN ARG VAL GLU ALA GLU ASP LEU GLY VAL TYR
       210                 215                 220
   TAT TGC TGG CAA GGT ACA CAT TCT CCG CTC ACG TTC GGT GCT GGG ACC     720
   TYR CYS TRP GLN GLY THR HIS SER PRO LEU THR PHE GLY ALA GLY THR
   225                 230                 235                 240
   AAG CTG GAG CTG AAA CGG GCG GCC GCA TTG CAG ACG CGT CGA CCT GCA     768
   LYS LEU GLU LEU LYS ARG ALA ALA ALA LEU GLN THR ARG ARG PRO ALA
                   245                 250                 255
   CCA GCA GCT CCT ACA CCG GCG GCC CCT GCA CCA GCC CCC TCC TGG CCC     816
   PRO ALA ALA PRO THR PRO ALA ALA PRO ALA PRO ALA PRO SER TRP PRO
               260                 265                 270
   CTG TCA TCT TCT GTC CCT TCC CAG AAA ACC TAC CAG GGC AGC TAC GGT     864
   LEU SER SER SER VAL PRO SER GLN LYS THR TYR GLN GLY SER TYR GLY
           275                 280                 285
   TTC CGT CTG GGC TTC TTG CAT TCT GGG ACA GCC AAG TCT GTG ACT TGC     912
   PHE ARG LEU GLY PHE LEU HIS SER GLY THR ALA LYS SER VAL THR CYS
       290                 295                 300
   ACG TAC TCC CCT GCC CTC AAC AAG ATG TTT TGC CAA CTG GCC AAG ACC     960
   THR TYR SER PRO ALA LEU ASN LYS MET PHE CYS GLN LEU ALA LYS THR
   305                 310                 315                 320
   TGC CCT GTG CAG CTG TGG GTT GAT TCC ACA CCC CCG CCC GGC ACC CGC    1008
   CYS PRO VAL GLN LEU TRP VAL ASP SER THR PRO PRO PRO GLY THR ARG
                   325                 330                 335
   GTC CGC GCC ATG GCC ATC TAC AAG CAG TCA CAG CAC ATG ACG GAG GTT    1056
   VAL ARG ALA MET ALA ILE TYR LYS GLN SER GLN HIS MET THR GLU VAL
               340                 345                 350
   GTG AGG CGC TGC CCC CAC CAT GAG CGC TGC TCA GAT AGC GAT GGT CTG    1104
   VAL ARG ARG CYS PRO HIS HIS GLU ARG CYS SER ASP SER ASP GLY LEU
           355                 360                 365
   GCC CCT CCT CAG CAT CTT ATC CGA GTG GAA GGA AAT TTG CGT GTG GAG    1152
   ALA PRO PRO GLN HIS LEU ILE ARG VAL GLU GLY ASN LEU ARG VAL GLU
       370                 375                 380
   TAT TTG GAT GAC AGA AAC ACT TTT CGA CAT AGT GTG GTG GTG CCC TAT    1200
   TYR LEU ASP ASP ARG ASN THR PHE ARG HIS SER VAL VAL VAL PRO TYR
   385                 390                 395                 400
   GAG CCG CCT GAG GTT GGC TCT GAC TGT ACC ACC ATC CAC TAC AAC TAC    1248
   GLU PRO PRO GLU VAL GLY SER ASP CYS THR THR ILE HIS TYR ASN TYR
                   405                 410                 415
   ATG TGT AAC AGT TCC TGC ATG GGC GGC ATG AAC CGG AGG CCC ATC CTC    1296
   MET CYS ASN SER SER CYS MET GLY GLY MET ASN ARG ARG PRO ILE LEU
               420                 425                 430
   ACC ATC ATC ACA CTG GAA GAC TCC AGT GGT AAT CTA CTG GGA CGG AAC    1344
   THR ILE ILE THR LEU GLU ASP SER SER GLY ASN LEU LEU GLY ARG ASN
           435                 440                 445
   AGC TTT GAG GTG CGT GTT TGT GCC TGT CCT GGG AGA GAC CGG CGC ACA    1392
   SER PHE GLU VAL ARG VAL CYS ALA CYS PRO GLY ARG ASP ARG ARG THR
       450                 455                 460
   GAG GAA GAG AAT CTC CGC AAG AAA GGG GAG CCT CAC CAC GAG CTG CCC    1440
   GLU GLU GLU ASN LEU ARG LYS LYS GLY GLU PRO HIS HIS GLU LEU PRO
   465                 470                 475                 480
   CCA GGG AGC ACT AAG CGA GCA CTG CCC AAC AAC ACC AGC TCC TCT CCC    1488
   PRO GLY SER THR LYS ARG ALA LEU PRO ASN ASN THR SER SER SER PRO
                   485                 490                 495
   CAG CCA AAG AAG AAA CCA CTG GAT GGG GAT CTG AAG GCC CTC AAG GAG    1536
   GLN PRO LYS LYS LYS PRO LEU ASP GLY ASP LEU LYS ALA LEU LYS GLU
               500                 505                 510
   AAG CTG AAG GCC CTG GAG GAG AAG CTG AAG GCC CTG GAG GAG AAG CTG    1584
   LYS LEU LYS ALA LEU GLU GLU LYS LEU LYS ALA LEU GLU GLU LYS LEU
           515                 520                 525
   AAG GCA CTA GTG GGG GAG CGA TGA TGA                                1611
   LYS ALA LEU VAL GLY GLU ARG  *   *
       530                 535
(2) INFORMATIONS POUR LA SEQ ID NO: 32:
   (I) CARACTERISTIQUES DE LA SEQUENCE:
      (A) LONGUEUR: 1065 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (IX) CARACTERISTIQUE:
      (A) NOM/CLE: CDS
      (B) EMPLACEMENT:1..1065
   (XI) DESCRIPTION DE LA SEQUENCE: SEQ ID NO: 32:
   ATG GGA GAA TAT TTC ACC CTT CAG ATC CGT GGG CGT GAG CGC TTC GAG    48
   MET GLY GLU TYR PHE THR LEU GLN ILE ARG GLY ARG GLU ARG PHE GLU
    1               5                  10                  15
   ATG TTC CGA GAG CTG AAT GAG GCC TTG GAA CTC AAG GAT GCC CAG GCT    96
   MET PHE ARG GLU LEU ASN GLU ALA LEU GLU LEU LYS ASP ALA GLN ALA
               20                  25                  30
   GGG AAG GAG CCA GGG GGG AGC AGG GCT CAC TCC AGC CAC CTG AAG TCC    144
   GLY LYS GLU PRO GLY GLY SER ARG ALA HIS SER SER HIS LEU LYS SER
           35                  40                  45
   AAA AAG GGT CAG TCT ACC TCC CGC CAT AAA AAA CTC ATG TTC AAG ACA    192
   LYS LYS GLY GLN SER THR SER ARG HIS LYS LYS LEU MET PHE LYS THR
       50                  55                  60
   GAA GGG CCT GAC TCA GAC GGT CGA CCT GCA CCA GCA GCT CCT ACA CCG    240
   GLU GLY PRO ASP SER ASP GLY ARG PRO ALA PRO ALA ALA PRO THR PRO
   65                       70                       75                       80
   GCG GCC CCT GCA CCA GCC CCC TCC TGG CCC CTG TCA TCT TCT GTC CCT         288
   ALA ALA PRO ALA PRO ALA PRO SER TRP PRO LEU SER SER SER VAL PRO
                       85                       90                       95
   TCC CAG AAA ACC TAC CAG GGC AGC TAC GGT TTC CGT CTG GGC TTC TTG         336
   SER GLN LYS THR TYR GLN GLY SER TYR GLY PHE ARG LEU GLY PHE LEU
                  100                      105                      110
   CAT TCT GGG ACA GCC AAG TCT GTG ACT TGC ACG TAC TCC CCT GCC CTC         384
   HIS SER GLY THR ALA LYS SER VAL THR CYS THR TYR SER PRO ALA LEU
             115                      120                      125
   AAC AAG ATG TTT TGC CAA CTG GCC AAG ACC TGC CCT GTG CAG CTG TGG         432
   ASN LYS MET PHE CYS GLN LEU ALA LYS THR CYS PRO VAL GLN LEU TRP
        130                      135                      140
   GTT GAT TCC ACA CCC CCG CCC GGC ACC CGC GTC CGC GCC ATG GCC ATC         480
   VAL ASP SER THR PRO PRO PRO GLY THR ARG VAL ARG ALA MET ALA ILE
   145                      150                      155                      160
   TAC AAG CAG TCA CAG CAC ATG ACG GAG GTT GTG AGG CGC TGC CCC CAC         528
   TYR LYS GLN SER GLN HIS MET THR GLU VAL VAL ARG ARG CYS PRO HIS
                       165                      170                      175
   CAT GAG CGC TGC TCA GAT AGC GAT GGT CTG GCC CCT CCT CAG CAT CTT         576
   HIS GLU ARG CYS SER ASP SER ASP GLY LEU ALA PRO PRO GLN HIS LEU
                  180                      185                      190
   ATC CGA GTG GAA GGA AAT TTG CGT GTG GAG TAT TTG GAT GAC AGA AAC         624
   ILE ARG VAL GLU GLY ASN LEU ARG VAL GLU TYR LEU ASP ASP ARG ASN
             195                      200                      205
   ACT TTT CGA CAT AGT GTG GTG GTG CCC TAT GAG CCG CCT GAG GTT GGC         672
   THR PHE ARG HIS SER VAL VAL VAL PRO TYR GLU PRO PRO GLU VAL GLY
        210                      215                      220
   TCT GAC TGT ACC ACC ATC CAC TAC AAC TAC ATG TGT AAC AGT TCC TGC         720
   SER ASP CYS THR THR ILE HIS TYR ASN TYR MET CYS ASN SER SER CYS
   225                      230                      235                      240
   ATG GGC GGC ATG AAC CGG AGG CCC ATC CTC ACC ATC ATC ACA CTG GAA         768
   MET GLY GLY MET ASN ARG ARG PRO ILE LEU THR ILE ILE THR LEU GLU
                       245                      250                      255
   GAC TCC AGT GGT AAT CTA CTG GGA CGG AAC AGC TTT GAG GTG CGT GTT         816
   ASP SER SER GLY ASN LEU LEU GLY ARG ASN SER PHE GLU VAL ARG VAL
                  260                      265                      270
   TGT GCC TGT CCT GGG AGA GAC CGG CGC ACA GAG GAA GAG AAT CTC CGC         864
   CYS ALA CYS PRO GLY ARG ASP ARG ARG THR GLU GLU GLU ASN LEU ARG
             275                      280                      285
   AAG AAA GGG GAG CCT CAC CAC GAG CTG CCC CCA GGG AGC ACT AAG CGA         912
   LYS LYS GLY GLU PRO HIS HIS GLU LEU PRO PRO GLY SER THR LYS ARG
        290                      295                      300
   GCA CTG CCC AAC AAC ACC AGC TCC TCT CCC CAG CCA AAG AAG AAA CCA         960
   ALA LEU PRO ASN ASN THR SER SER SER PRO GLN PRO LYS LYS LYS PRO
   305                 310                 315                 320
   CTG GAT GGG GAT CTG AAG GCC CTC AAG GAG AAG CTG AAG GCC CTG GAG    1008
   LEU ASP GLY ASP LEU LYS ALA LEU LYS GLU LYS LEU LYS ALA LEU GLU
                   325                 330                 335
   GAG AAG CTG AAG GCC CTG GAG GAG AAG CTG AAG GCA CTA GTG GGG GAG    1056
   GLU LYS LEU LYS ALA LEU GLU GLU LYS LEU LYS ALA LEU VAL GLY GLU
               340                 345                 350
   CGA TGA TGA                                                        1065
   ARG  *   *
           355
(2) INFORMATIONS POUR LA SEQ ID NO: 33:
   (I) CARACTERISTIQUES DE LA SEQUENCE:
      (A) LONGUEUR: 963 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (IX) CARACTERISTIQUE:
      (A) NOM/CLE: CDS
      (B) EMPLACEMENT:1..963
   (XI) DESCRIPTION DE LA SEQUENCE: SEQ ID NO: 33:
   ATG GGA GAA TAT TTC ACC CTT CAG ATC CGT GGG CGT GAG CGC TTC GAG      48
   MET GLY GLU TYR PHE THR LEU GLN ILE ARG GLY ARG GLU ARG PHE GLU
    1               5                  10                  15
   ATG TTC CGA GAG CTG AAT GAG GCC TTG GAA CTC AAG GAT GCC CAG GCT      96
   MET PHE ARG GLU LEU ASN GLU ALA LEU GLU LEU LYS ASP ALA GLN ALA
               20                  25                  30
   GGG AAG GAG CCA GGT CGA CCT GCA CCA GCA GCT CCT ACA CCG GCG GCC     144
   GLY LYS GLU PRO GLY ARG PRO ALA PRO ALA ALA PRO THR PRO ALA ALA
           35                  40                  45
   CCT GCA CCA GCC CCC TCC TGG CCC CTG TCA TCT TCT GTC CCT TCC CAG     192
   PRO ALA PRO ALA PRO SER TRP PRO LEU SER SER SER VAL PRO SER GLN
       50                  55                  60
   AAA ACC TAC CAG GGC AGC TAC GGT TTC CGT CTG GGC TTC TTG CAT TCT     240
   LYS THR TYR GLN GLY SER TYR GLY PHE ARG LEU GLY PHE LEU HIS SER
    65                 70                  75                  80
   GGG ACA GCC AAG TCT GTG ACT TGC ACG TAC TCC CCT GCC CTC AAC AAG     288
   GLY THR ALA LYS SER VAL THR CYS THR TYR SER PRO ALA LEU ASN LYS
                   85                  90                  95
   ATG TTT TGC CAA CTG GCC AAG ACC TGC CCT GTG CAG CTG TGG GTT GAT     336
   MET PHE CYS GLN LEU ALA LYS THR CYS PRO VAL GLN LEU TRP VAL ASP
               100                 105                 110
   TCC ACA CCC CCG CCC GGC ACC CGC GTC CGC GCC ATG GCC ATC TAC AAG     384
   SER THR PRO PRO PRO GLY THR ARG VAL ARG ALA MET ALA ILE TYR LYS
           115                 120                 125
   CAG TCA CAG CAC ATG ACG GAG GTT GTG AGG CGC TGC CCC CAC CAT GAG     432
   GLN SER GLN HIS MET THR GLU VAL VAL ARG ARG CYS PRO HIS HIS GLU
       130                 135                 140
   CGC TGC TCA GAT AGC GAT GGT CTG GCC CCT CCT CAG CAT CTT ATC CGA     480
   ARG CYS SER ASP SER ASP GLY LEU ALA PRO PRO GLN HIS LEU ILE ARG
   145                 150                 155                 160
   GTG GAA GGA AAT TTG CGT GTG GAG TAT TTG GAT GAC AGA AAC ACT TTT     528
   VAL GLU GLY ASN LEU ARG VAL GLU TYR LEU ASP ASP ARG ASN THR PHE
                   165                 170                 175
   CGA CAT AGT GTG GTG GTG CCC TAT GAG CCG CCT GAG GTT GGC TCT GAC     576
   ARG HIS SER VAL VAL VAL PRO TYR GLU PRO PRO GLU VAL GLY SER ASP
               180                 185                 190
   TGT ACC ACC ATC CAC TAC AAC TAC ATG TGT AAC AGT TCC TGC ATG GGC     624
   CYS THR THR ILE HIS TYR ASN TYR MET CYS ASN SER SER CYS MET GLY
           195                 200                 205
   GGC ATG AAC CGG AGG CCC ATC CTC ACC ATC ATC ACA CTG GAA GAC TCC     672
   GLY MET ASN ARG ARG PRO ILE LEU THR ILE ILE THR LEU GLU ASP SER
       210                 215                 220
   AGT GGT AAT CTA CTG GGA CGG AAC AGC TTT GAG GTG CGT GTT TGT GCC     720
   SER GLY ASN LEU LEU GLY ARG ASN SER PHE GLU VAL ARG VAL CYS ALA
   225                 230                 235                 240
   TGT CCT GGG AGA GAC CGG CGC ACA GAG GAA GAG AAT CTC CGC AAG AAA     768
   CYS PRO GLY ARG ASP ARG ARG THR GLU GLU GLU ASN LEU ARG LYS LYS
                   245                 250                 255
   GGG GAG CCT CAC CAC GAG CTG CCC CCA GGG AGC ACT AAG CGA GCA CTG     816
   GLY GLU PRO HIS HIS GLU LEU PRO PRO GLY SER THR LYS ARG ALA LEU
               260                 265                 270
   CCC AAC AAC ACC AGC TCC TCT CCC CAG CCA AAG AAG AAA CCA CTG GAT     864
   PRO ASN ASN THR SER SER SER PRO GLN PRO LYS LYS LYS PRO LEU ASP
           275                 280                 285
   GGG GAT CTG AAG GCC CTC AAG GAG AAG CTG AAG GCC CTG GAG GAG AAG     912
   GLY ASP LEU LYS ALA LEU LYS GLU LYS LEU LYS ALA LEU GLU GLU LYS
       290                 295                 300
   CTG AAG GCC CTG GAG GAG AAG CTG AAG GCA CTA GTG GGG GAG CGA TGA     960
   LEU LYS ALA LEU GLU GLU LYS LEU LYS ALA LEU VAL GLY GLU ARG  *
   305                 310                 315                 320
   TGA                                                                963
    *
(2) INFORMATIONS POUR LA SEQ ID NO: 34:
   (I) CARACTERISTIQUES DE LA SEQUENCE:
      (A) LONGUEUR: 1011 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (IX) CARACTERISTIQUE:
      (A) NOM/CLE: CDS
      (B) EMPLACEMENT:1..1011
   (XI) DESCRIPTION DE LA SEQUENCE: SEQ ID NO: 34:
   ATG GGA GAA TAT TTC ACC CTT CAG ATC CGT GGG CGT GAG CGC TTC GAG       48
   MET GLY GLU TYR PHE THR LEU GLN ILE ARG GLY ARG GLU ARG PHE GLU
    1               5                  10                  15
   ATG TTC CGA GAG CTG AAT GAG GCC TTG GAA CTC AAG GAT GCC CAG GCT       96
   MET PHE ARG GLU LEU ASN GLU ALA LEU GLU LEU LYS ASP ALA GLN ALA
               20                  25                  30
   GGG AAG GAG CCA GGT CGA GGA GGT GGT GGC TCT GGA GGC GGA GGA TCC      144
   GLY LYS GLU PRO GLY ARG GLY GLY GLY GLY SER GLY GLY GLY GLY SER
           35                  40                  45
   GGC GGT GGA GGT TCT CGA CCT GCA CCA GCA GCT CCT ACA CCG GCG GCC      192
   GLY GLY GLY GLY SER ARG PRO ALA PRO ALA ALA PRO THR PRO ALA ALA
       50                  55                  60
   CCT GCA CCA GCC CCC TCC TGG CCC CTG TCA TCT TCT GTC CCT TCC CAG      240
   PRO ALA PRO ALA PRO SER TRP PRO LEU SER SER SER VAL PRO SER GLN
   65                  70                  75                  80
   AAA ACC TAC CAG GGC AGC TAC GGT TTC CGT CTG GGC TTC TTG CAT TCT      288
   LYS THR TYR GLN GLY SER TYR GLY PHE ARG LEU GLY PHE LEU HIS SER
                   85                  90                  95
   GGG ACA GCC AAG TCT GTG ACT TGC ACG TAC TCC CCT GCC CTC AAC AAG      336
   GLY THR ALA LYS SER VAL THR CYS THR TYR SER PRO ALA LEU ASN LYS
               100                 105                 110
   ATG TTT TGC CAA CTG GCC AAG ACC TGC CCT GTG CAG CTG TGG GTT GAT      384
   MET PHE CYS GLN LEU ALA LYS THR CYS PRO VAL GLN LEU TRP VAL ASP
           115                 120                 125
   TCC ACA CCC CCG CCC GGC ACC CGC GTC CGC GCC ATG GCC ATC TAC AAG     432
   SER THR PRO PRO PRO GLY THR ARG VAL ARG ALA MET ALA ILE TYR LYS
       130                 135                 140
   CAG TCA CAG CAC ATG ACG GAG GTT GTG AGG CGC TGC CCC CAC CAT GAG     480
   GLN SER GLN HIS MET THR GLU VAL VAL ARG ARG CYS PRO HIS HIS GLU
   145                 150                 155                 160
   CGC TGC TCA GAT AGC GAT GGT CTG GCC CCT CCT CAG CAT CTT ATC CGA     528
   ARG CYS SER ASP SER ASP GLY LEU ALA PRO PRO GLN HIS LEU ILE ARG
                   165                 170                 175
   GTG GAA GGA AAT TTG CGT GTG GAG TAT TTG GAT GAC AGA AAC ACT TTT     576
   VAL GLU GLY ASN LEU ARG VAL GLU TYR LEU ASP ASP ARG ASN THR PHE
               180                 185                 190
   CGA CAT AGT GTG GTG GTG CCC TAT GAG CCG CCT GAG GTT GGC TCT GAC     624
   ARG HIS SER VAL VAL VAL PRO TYR GLU PRO PRO GLU VAL GLY SER ASP
           195                 200                 205
   TGT ACC ACC ATC CAC TAC AAC TAC ATG TGT AAC AGT TCC TGC ATG GGC     672
   CYS THR THR ILE HIS TYR ASN TYR MET CYS ASN SER SER CYS MET GLY
       210                 215                 220
   GGC ATG AAC CGG AGG CCC ATC CTC ACC ATC ATC ACA CTG GAA GAC TCC     720
   GLY MET ASN ARG ARG PRO ILE LEU THR ILE ILE THR LEU GLU ASP SER
   225                 230                 235                 240
   AGT GGT AAT CTA CTG GGA CGG AAC AGC TTT GAG GTG CGT GTT TGT GCC     768
   SER GLY ASN LEU LEU GLY ARG ASN SER PHE GLU VAL ARG VAL CYS ALA
                   245                 250                 255
   TGT CCT GGG AGA GAC CGG CGC ACA GAG GAA GAG AAT CTC CGC AAG AAA     816
   CYS PRO GLY ARG ASP ARG ARG THR GLU GLU GLU ASN LEU ARG LYS LYS
               260                 265                 270
   GGG GAG CCT CAC CAC GAG CTG CCC CCA GGG AGC ACT AAG CGA GCA CTG     864
   GLY GLU PRO HIS HIS GLU LEU PRO PRO GLY SER THR LYS ARG ALA LEU
           275                 280                 285
   CCC AAC AAC ACC AGC TCC TCT CCC CAG CCA AAG AAG AAA CCA CTG GAT     912
   PRO ASN ASN THR SER SER SER PRO GLN PRO LYS LYS LYS PRO LEU ASP
       290                 295                 300
   GGG GAT CTG AAG GCC CTC AAG GAG AAG CTG AAG GCC CTG GAG GAG AAG     960
   GLY ASP LEU LYS ALA LEU LYS GLU LYS LEU LYS ALA LEU GLU GLU LYS
   305                 310                 315                 320
   CTG AAG GCC CTG GAG GAG AAG CTG AAG GCA CTA GTG GGG GAG CGA TGA    1008
   LEU LYS ALA LEU GLU GLU LYS LEU LYS ALA LEU VAL GLY GLU ARG  *
                   325                 330                 335
   TGA                                                                1011
    *
(2) INFORMATIONS POUR LA SEQ ID NO: 35:
   (I) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 35:
   CGGATCCTCT CGGAACATCT CGAA 24
(2) INFORMATIONS POUR LA SEQ ID NO: 36:
   (I) CARACTERISTIQUES DE LA SEQUENCE:
      (A) LONGUEUR: 749 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 36:
   GCCATGGCCC AGGTGCAGCT GCAGGAGTCA GGGGCAGAGC TTGTGGGGTC AGGGGCCTCA    60
   GTCAAGTTGT CCTGCACAGC TTCTGGCTTC AACATTAAAG ACTACTATAT GCACTGGGTG   120
   AAGCAGAGGC CTGAACAGGG CCTGGAGTGG ATTGGATGGA TTGATCCTGA GAATGGTGAT   180
   ACTGAATATG CCCCGAAGTT CCAGGGCAAG GCCACTATGA CTGCAGACAC ATCCTCCAAT   240
   ACAGCCTACC TGCAGCTCAG CAGCCTGGCA TCTGAGGACA CTGCCGTCTA TTATTGTAAT   300
   TTTTACGGGG ATGCTTTGGA CTACTGGGGC CAAGGGACCA CGGTCACCGT CTCCTCAGGT   360
   GGAGGCGGTT CAGGCGGAGG TGGCTCTGGC GGTGGCGGAT CGGATGTTTT GATGACCCAA   420
   ACTCCACTCA CTTTGTCGGT TACCATTGGA CAACCAGCCT CCATCTCTTG CAAGTCAAGT   480
   CAGAGCCTCT TGGATAGTGA TGGAAAGACA TATTTGAATT GGTTGTTACA GAGGCCAGGC   540
   CAGTCTCCAA AGCGCCTAAT CTATCTGGTG TCTAAACTGG ACTCTGGAGT CCCTGACAGG   600
   TTCACTGGCA GTGGATCAGG GACAGATTTC ACACTGAAAA TCAACAGAGT GGAGGCTGAG   660
   GATTTGGGAG TTTATTATTG CTGGCAAGGT ACACATTCTC CGCTCACGTT CGGTGCTGGG   720
   ACCAAGCTGG AGCTGAAACG GGCGGCCGC                                     749
(2) INFORMATIONS POUR LA SEQ ID NO: 37:
   (I) CARACTERISTIQUES DE LA SEQUENCE:
      (A) LONGUEUR: 45 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 37:
   AAGCTTGAAT TCGTTAACGC CACCATGGGA GAATATTTCA CCCTT 45
(2) INFORMATIONS POUR LA SEQ ID NO: 38:
   (I) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 38:
   GGGTCGACCT GGCTCCTTCC CAGC 24
(2) INFORMATIONS POUR LA SEQ ID NO: 39:
   (I) CARACTERISTIQUES DE LA SEQUENCE:
      (A) LONGUEUR: 749 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 39:
(2) INFORMATIONS POUR LA SEQ ID NO: 40:
   (I) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 40:
   GGGTCGACCG TCTGAGTCAG GCCCTTC                                    27
(2) INFORMATIONS POUR LA SEQ ID NO: 41:
   (I) CARACTERISTIQUES DE LA SEQUENCE:
      (A) LONGUEUR: 749 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 41:
(2) INFORMATIONS POUR LA SEQ ID NO: 42:
   (I) CARACTERISTIQUES DE LA SEQUENCE:
      (A) LONGUEUR: 48 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 42:
   TCGAGGAGGT GGTGGCTCTG GAGGCGGAGG ATCCGGCGGT GGAGGTTC               48
(2) INFORMATIONS POUR LA SEQ ID NO: 43:
   (I) CARACTERISTIQUES DE LA SEQUENCE:
      (A) LONGUEUR: 48 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 43:
   TCGAGAACCC CTACCGCCGG ATCCTCCGCC TCCAGAGCCA CCACCTCC 48
(2) INFORMATIONS POUR LA SEQ ID NO:44:
   (i) CARACTERISTIQUES DE LA SEQUENCE:
      (A) LONGUEUR: 16 acides aminés
      (B) TYPE: acide aminé
      (C) NOMBRE DE BRINS: simple
      (D) CONFIGURATION: linéaire
   (ii) TYPE DE MOLECULE: peptide
   (iii) HYPOTHETIQUE: non
   (v) TYPE DE FRAGMENT: interne
   (xi) DESCRIPTION DE LA SEQUENCE: SEQ ID NO: 44:
   Gly Gly Gly Ser Gly Gly Gly Ser Gly Gly Gly Ser Gly Gly Gly Ser
   1               5                   10                  15
(2) INFORMATIONS POUR LA SEQ ID NO: 45:
   (I) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: lin,aire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 45:
   GATCCGAACA TGTCCCAACA TGTTGA 26
(2) INFORMATIONS POUR LA SEQ ID NO: 46:
   (I) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (II) TYPE DE MOLECULE: ADNC
   (III) HYPOTHETIQUE: NON
   (IV) ANTI-SENS: NON
   (XI) DESCRIPTION DE LA SEQUENCE: SEQ ID NO: 46:
   AGCTTCAACA TGTTGGGACA TGGTCG                                          26

## Description

La présente invention concerne des protéines dérivées du produit du gène suppresseur de tumeurs p53, possédant des fonctions améliorées en vue d'une utilisation thérapeutique. Elle concerne avantageusement des protéines possédant des fonctions suppresseur de tumeurs et inducteur de mort cellulaire programmée supérieures à celle de la protéine p53 de type sauvage, plus particulièrement dans des contextes pathologiques de prolifération dans lesquels la protéine p53 de type sauvage est inactivée. Elle concerne également les acides nucléiques codant pour ces molécules, les vecteurs les contenant et leurs utilisations thérapeutiques, notamment en thérapie génique. Les produits de l'invention sont particulièrement adaptés à la restauration des fonctions de p53 dans des contextes pathologiques tels que notamment les cancers.

La protéine p53 sauvage intervient dans la régulation du cycle cellulaire et dans le maintien de l'intégrité du génome de la cellule. Cette protéine, dont la fonction principale est d'être un activateur de la transcription de certains gènes, est susceptible, par un processus non encore bien défini, de bloquer la cellule en phase G1 du cycle cellulaire lors de l'apparition de mutations au cours de la réplication du génome, et d'enclencher un certains nombre de processus de réparation de l'ADN. De plus, en cas de mauvais fonctionnement de ces processus de réparation ou en cas d'apparition d'évènements mutationnels trop nombreux pour être corrigés, cette protéine est capable d'induire le phénomène de mort cellulaire programmée, appelé apoptose.

De cette façon, la protéine p53 agit comme un supresseur de tumeur, en éliminant les cellules anormalement différenciées ou dont le génome a été endommagé.

Cette principale fonction de la p53 dépend de sa fonction de facteur de transcription, soit en d'autre termes de sa double capacité à reconnaître des séquences spécifiques au niveau de l'ADN génomique et à recruter la machinerie générale de transcription.

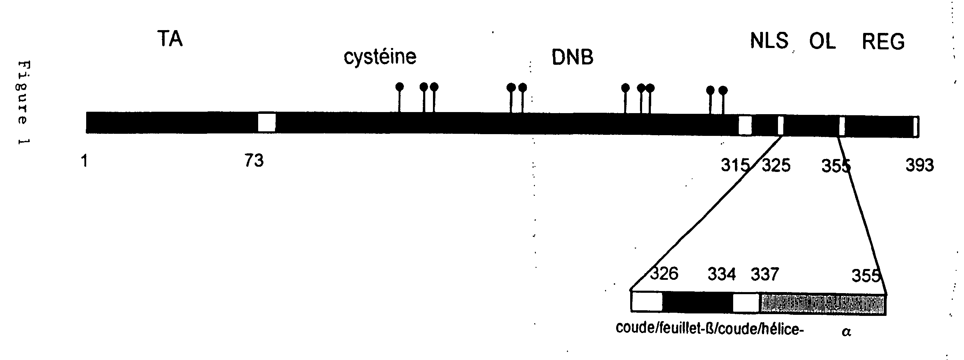

La protéine p53 comporte 393 acides aminés, qui définissent 5 domaines fonctionnels (voir Figure 1) :
- le domaine activateur de la transcription, constitué par les acides aminés 1 à 73, capable de lier certains facteurs de la machinerie générale de transcription comme la protéine TBP. Ce domaine est aussi le siège d'un certain nombre de modifications post-traductionnelles. Il est également le siège d'interactions nombreuses de la protéine p53 avec de nombreuses autres protéines et notamment avec la protéine cellulaire mdm2
ou la protéine EBNA5 du virus d'Epstein-Barr (EBV), capables de bloquer la fonction de la protéine sauvage. De plus, ce domaine possède des séquences d'acides aminés dites PEST de susceptibilité à la dégradation protéolytique.
- le domaine de liaison à l'ADN, localisé entre les acides aminés 73 et 315. La conformation de ce domaine central de p53 régule la reconnaissance de séquences d'ADN spécifiques de la protéine p53. Ce domaine est le siège de deux types d'altérations affectant la fonction de la protéine sauvage :
   (i) l'interaction avec des protéines bloquant la fonction de la p53 comme l'antigène 'grand T' du virus SV40 ou les protéines virales E6 des virus HPV16 et HPV18 capables de provoquer sa dégradation par le système de l'ubiquitine. Cette dernière interaction ne peut se faire qu'en présence de la protéine cellulaire E6ap (enzyme E3 de la cascade de l'ubiquitinilation).
   (ii) les mutations ponctuelles qui affectent la fonction de la p53 et dont la quasi-totalité sont localisées dans cette région.
- le signal de localisation nucléaire, constitué des acides aminés 315 à 325, indispensable au bon adressage de la protéine dans le compartiment où elle va exercer sa principale fonction.
- le domaine d'oligomérisation, constitué des acides aminés 325 à 355. Cette région 325 à 355 forme une structure de type; feuillet β (326-334)-coude (335-336)-hélice α (337-355). Les altérations de fonctions localisées dans cette région sont essentiellement dues à l'interaction de la protéine sauvage avec les différentes formes mutantes qui peuvent conduire à des effets variables sur la fonction de la protéine sauvage.
- le domaine de régulation, constitué des acides aminés 365 à 393, qui est le siège d'un certain nombre de modifications post-traductionnelles (glycosylations, phosphorylations, fixation d'ARN,...) qui modulent la fonction de la protéine p53 de façon positive ou négative. Ce domaine joue un rôle extrèmement important dans la modulation de l'activité de la protéine sauvage.

Le fonctionnement de la protéine p53 peut être perturbé de différentes façons.
- blocage de sa fonction par un certain nombre de facteurs comme par exemple l'antigène 'grand T' du virus SV40, la protéine EBNA5 du virus d'Epstein-Barr, ou la protéine cellulaire mdm2.
- déstabilisation de la protéine par augmentation de sa susceptibilité à la protéolyse, notamment par interaction avec la protéine E6 des virus du papillome humain HPV16 et HPV18, qui favorise l'entrée de la p53 dans le cycle d'ubiquitinilation. Dans ce cas l'interaction entre ces deux protéines ne peut se faire que par la fixation préalable d'une protéine cellulaire, la protéine E6ap dont le site de fixation est mal connu.
- mutations ponctuelles au niveau du gène de la p53.
- délétion d'un ou des deux allèles de la p53

Les deux derniers types de modifications sont retrouvés dans environ 50% des différents types de cancer. A cet égard, les mutations du gène de la p53 repertoriées dans les cellules cancéreuses touchent une très grande partie du gène codant pour cette protéine, et ont pour résultats des modifications variables du fonctionnement de cette protéine. On peut cependant noter que ces mutations sont en grande majorité localisées dans la partie centrale de la protéine p53 dont on sait qu'elle est la région de contact avec les séquences génomiques spécifiques de la protéine p53.

Ceci explique pourquoi la plupart des mutants de la protéine p53 ont comme principale caractéristique de ne plus pouvoir se fixer aux séquences d'ADN que reconnait la protéine sauvage et ainsi de ne plus pouvoir exercer leur rôle de facteur de transcription. Par ailleurs, certains mutants semblent avoir acquis de nouvelles fonctions telles que l'activation de certains gènes au niveau transcriptionnel.

On regroupe actuellement l'ensemble de ces modifications dans trois catégories:
- les mutants dits faibles, dont le produit est une protéine non-fonctionnelle, qui, dans le cas de mutation sur un seul des deux allèles, n'affecte pas le fonctionnement de la protéine sauvage codée par l'autre allèle. Les principaux représentants de cette catégorie sont les mutants H273 et W248, ce dernier étant spécifique du syndrome familial de Li-Fraumeni d'hypersensibilité aux affections cancéreuses.
- les mutants dominant-négatifs, dont le produit est une protéine non-fonctionnelle, qui, dans le cas de mutation sur un seul des deux allèles et par intéraction avec la protéine sauvage, est capable de bloquer le fonctionnement de celle-ci par formation d'oligomères mixtes non-actifs qui ne peuvent plus se fixer aux séquences d'ADN spécifiques de la protéine sauvage. Le principal représentant de cette catégorie est le mutant G281.
- les mutants dominant-oncogéniques, dont le produit est une protéine qui est capable d'une part de bloquer la fonction de la protéine sauvage comme les mutants de la catégorie précédente, et d'autre part, de favoriser par des mécanismes mal connus le développement tumoral, présentant ainsi un gain de fonction. Le principal représentant de cette catégorie est le mutant H175.

Compte tenu de ses propriétés anti-tumorales et apoptotiques et de son implication dans nombreuses pathologies de type hyperprolifératives, le gène p53 sauvage a été utilisé dans des approches de thérapie génique et cellulaire. II a en particulier été proposé de traiter certaines pathologies hyperprolifératives, et notament des cancers, par administration in vivo du gène p53 sauvage, par restauration des fonctions de p53. L'administration peut être réalisée préférentiellement par des vecteurs viraux et notamment adénoviraux (WO94/24297) ou rétroviraux (WO94/06910).

Il a ainsi été montré que l'introduction d'un acide nucléique codant pour la protéine p53 sauvage permettait de restaurer partiellement une régulation normale de la croissance cellulaire. Cependant, si ces résultats sont encourageants, l'efficacité de ces approches est limitée par l'efficacité thérapeutique de la protéine p53 après transfert et expression in vivo dans les cellules hyperprolifératives. En effet, les situations pathologiques hyperproliférative telles que les cancers proviennent du déréglement de l'équilibre qui s'établit dans un réseau de contrôles négatifs et positifs de la croissance cellulaire. L'inactivation du contrôle négatif exercé par la protéine p53 sauvage par l'apparition d'un mutant p53 dominant négatif à partir de l'un des deux allèles, la surexpression d'un partenaire cellulaire inactivant p53 comme par exemple mdm2, voir la présence d'un inactivateur viral suite à une infection, constituent un contexte non favorable pour une thérapie basée sur la réintroduction d'une protéine p53 sauvage qui risque fort d'être également inactivée.

Il est donc particulièrement important de pouvoir disposer de protéines de type p53 ayant des propriétés thérapeutiques accrues. Notamment, il serait particulièrement avantageux de disposer de molécules p53 actives constitutivement et non sensibles aux effets inactivateurs des mutants dominant-négatifs et oncogéniques ou d'autres protéines cellulaires ou virales telles que E6 de HPV18 et HPV16, MDM2, EBNA5 de EBV, etc rencontrés dans les cellules tumorales.

Certaines modifications de la protéine p53 ont été décrites dans l'art antérieur. Ainsi, la demande WO95/06661 décrit des modifications sur certains résidus des régions homologues de la protéine p53, c'est-à-dire dans les régions 343-351, 372-380 et 381-393. Cependant, ces modifications sont très mineures et ne permettent pas aux produits résultant d'échapper aux mécanismes d'inactivation de la protéine p53 in vivo. De plus, ces protéines ne semblent pas présenter d'activité améliorée par rapport à la protéine p53 sauvage.

Hupp et al. (Cell Vol 71 (1992) 875) ont décrit un dérivé de p53 comprenant une délétion des 30 résidus C-terminaux (p53ΔC-ter30). Toutefois, si cette protéine conserve une capacité à lier l'ADN, ses propriétés apoptotiques ne sont pas démontrées. De plus, elle n'est pas résistante à l'inactivation par les mutants dominants négatifs.

Pietenpol et al (PNAS 91 (1994) 1998) ont décrit des molécules chimériques dérivées de la protéine p53, notamment une protéine VP16-p53 (80-343)-GCN4. Cependant, cette molécule possède une capacité de liaison à l'ADN et de transactivation nettement diminuées par rapport à la protéine p53 sauvage (40%). Par ailleurs, elle possède une région d'oligomérisation non sélective, risquant d'interagir avec d'autres composant cellulaires et ainsi d'induire une réponse cellulaire non spécifique. Par ailleurs, ses propriétés de résistance aux mécanismes d'inactivation ne sont pas indiquées.

La présente invention décrit de nouveaux variants de la protéine p53 présentant des propriétés thérapeutiques améliorées. Elle décrit en particulier des variants adaptés à une utilisation en thérapie génique, notamment anti-cancéreuse. Les variants de l'invention dérivent de la protéine p53 par modification(s) structurale(s), conservent une activité de type p53 et, exprimés dans des cellules hyperprolifératives, présentent au moins une propriété accrue par rapport à la protéine p53. Il peut s'agir en particulier de l'activité anti-proliférative et/ou apoptotique. Les variants de l'invention possèdent avantageusement une activité anti-proliférative et/ou apoptotique accrue, ou plus spécifique des cellules hyperprolifératives, ou moins sensible aux différentes altérations auxquelles est sujette la p53 sauvage.

Un premier objet de l'invention concerne plus particulièrement un variant de la protéine p53 dans lequel tout ou partie du domaine d'oligomérisation est délété et remplacé par un domaine leucine zipper artificiel. Comme indiqué ci-avant, la protéine p53 est inactivée par certains mutants, et notamment les mutants dominant-négatifs et oncogéniques, rencontrés dans les cellules tumorales. Cette inactivation est le résultat de la formation d'oligomères mixtes non actifs entre la protéine p53 sauvage et le mutant, qui ne peuvent plus se fixer aux séquences spécifiques reconnues par la protéine p53 sauvage. La présente invention décrit maintenant des variants de la protéine p53 résistant à l'effet dominant négatif de certains mutants, c'est-à-dire des variants actifs dans un contexte cellulaire présentant un ou deux allèles mutés, ce qui est le cas de près de 90% des cancers humains p53 dépendants.

Dans les variants selon l'invention, tout ou partie du domaine d'oligomérisation naturel de la protéine, qui ne fait pas la distinction entre les formes sauvage et mutante, est ainsi remplacé par un domaine équivalent possédant une capacité d'oligomérisation spécifique. Cette modification est effectuée en utilisant un leucine-zipper artificiel optimisé pour former un dimère. Les molécules selon l'invention comportant un tel leucine-zipper artificiel sont particulièrement avantageuses car forment des oligomères uniquement avec d'autres molécules portant le même leucine-zipper. Elles ne forment donc pas d'oligomères avec les mutants dominant négatifs ou oncogéniques de la protéine p53, susceptibles de les inactiver. Elles ne forment pas non plus d'oligomères avec d'autres protéines cellulaires portant des domaines d'oligomérisation, susceptibles également de les inactiver ou d'induire des effets indésirables. Elles ne peuvent former que des homo-oligomères et possèdent donc une sélectivité importante, assurant une meilleure activité dans un contexte de pathologie hyperproliférative.

Selon la présente invention, le domaine leucine zipper artificiel est donc avantageusement un domaine non présent à l'état naturel, assurant une sélectivité d'oligomérisation. Tout préférentiellement, le domaine d'oligomérisation est représenté par la séquence SEQ ID n° 1.

Dans un mode de réalisation préféré de l'invention, les variants comportent une délétion de tout ou partie du domaine d'oligomérisation et de tout ou partie du domaine de régulation. Comme indiqué précédemment, le domaine d'oligomérisation est localisé entre les résidus 325-355 inclus et le domaine de régulation entre les résidus 365-393 inclus. Ce type de variant est tout à fait avantageux car est dépourvu de tout ou partie des effets de régulation négative exercée par l'intermédiaire de la partie C-terminale (aa 365-393). Ces variants constituent des protéines potentiellement constitutivement actives, présentant une activité non modulable et éventuellement accrue. La région de régulation est avantageusement supprimée dans sa totalité. Les variants préférés selon l'invention comportent une délétion de la partie C-terminale de la protéine p53, à partir du résidu 326 ou 337 inclus.

Des exemples d'intermédiaires utilisés pour la construction de ces variants sont notamment :
. pEC107 (75-325-lz) possédant une délétion de la partie C-terminale de la protéine p53, à partir du résidu 326, substituée par un domaine d'oligomérisation artificiel de séquence SEQ ID n°1.
. pEC110 (75-336-lz) possédant une délétion de la partie C-terminale de la protéine p53, à partir du résidu 337, substituée par un domaine d'oligomérisation artificiel de séquence SEQ ID n°1.

Selon un mode de réalisation avantageux, dans les variants de l'invention, le résidu cystéine en position 182 de la protéine p53 est remplacé par une histidine. Cette mutation permet avantageusement d'augmenter l'affinité du variant pour les séquences nucléotidiques spécifiques de liaison. L'introduction de cette modification supplémentaire permet donc d'obtenir une molécule ayant en plus un potentiel transactivateur augmenté.

Des exemples précis de constructions intermédiaires pour la préparation de variants selon l'invention combinant ces différentes modifications sont notamment :
pEC139 (75-325(H182)-lz) possédant une délétion de la partie C-terminale de la protéine p53, à partir du résidu 326, substituée par un domaine d'oligomérisation artificiel de séquence SEQ ID n°1, et une histidine en position 182.
pEC140 (75-336(H182)-Iz) possédant une délétion de la partie C-terminale de la protéine p53, à partir du résidu 337, substituée par un domaine d'oligomérisation artificiel de séquence SEQ ID n°1, et une histidine en position 182.

Avantageusement, dans les variants selon l'invention, tout ou partie du domaine transactivateur est également délété et remplacé par un domaine transactivateur hétérologue. Il a été indiqué ci-avant que les fonctions transactivatrices de p53 sont essentielles à son activité de suppresseur de tumeur ou d'inducteur d'apoptose. De manière à augmenter le potentiel thérapeutique des variants selon l'invention, il est particulièrement avantageux de substituer le domaine transactivateur naturel par un domaine transactivateur hétérologue puissant. Ces variants présentent ainsi de nombreux avantages. Ils possèdent bien entendu une activité transactivateur élevée. Mais ils sont également rendus insensibles aux effets de régulation négative exercés par l'intermédiaire de la partie N-terminale (aa 1-73). En effet cette région contient les séquence PEST responsables de sa dégradation protéolytique. La substitution de cette région par un domaine transactivateur hétérologue dépourvu de séquences PEST permet de diminuer cette régulation négative. Ces variants sont également caractérisés par la diminution, voire la suppression, de toute interaction avec la protéine E6 du virus du papillome humain (HPV) qui est susceptible d'induire leur dégradation. Ils sont également moins sensibles aux interactions avec d'autres protéines cellulaires telles que MDM2 et EBNA qui affectent l'activité de la protéine p53 sauvage. Les variants ainsi obtenus possèdent donc une stabilité accrue. La suppression des domaines sensibles à une régulation négative (domaines régulateur et transactivateur) conduit de manière particulièrement avantageuse à des molécules qui ne sont plus la cible de protéines induisant leur protéolyse ou leur inactivation.

Avantageusement, dans les variants de l'invention, le domaine transactivateur est supprimé par délétion des résidus 1 à 74. Les constructions intermédiaires utilisées pour la réalisation de telles molécules sont notamment pEC107 (75-325-lz), pEC110 (75-336-lz), pEC139 (75-325(H182)-Iz) et pEC140 (75-336(H182)-Iz).

Selon un premier mode de réalisation, le domaine transactivateur hétérologue est le domaine transactivateur de VP16. Il est avantageusement constitué des résidus 411 à 490 de VP16, dont la séquence est donnée SEQ ID n° 2. Des exemples précis de variants selon l'invention combinant ces différents modifications sont notamment :
⇒ pEC114 (VP16-75-325-lz) possédant une délétion de la partie N-terminale de la protéine p53 comprenant les résidus 1-74, substituée par le domaine transactivateur de VP16 de séquence SEQ ID n° 2 et une délétion de la partie C-terminale de la protéine p53, à partir du résidu 326, substituée par un domaine d'oligomérisation artificiel de séquence SEQ ID n°1. La séquence complète du variant pEC114 est représentée SEQ ID n° 25.
⇒ pEC116 (VP16-75-336-lz) possédant une délétion de la partie N-terminale de la protéine p53 comprenant les résidus 1-74, substituée par le domaine transactivateur de VP16 de séquence SEQ ID n° 2 et une délétion de la partie C-terminale de la protéine p53, à partir du résidu 337, substituée par un domaine d'oligomérisation artificiel de séquence SEQ ID n°1. La séquence complète du variant pEC116 est représentée SEQ ID n° 26.
⇒ pEC147 (VP16-75-325(H182)-Iz) possédant une délétion de la partie N-terminale de la protéine p53 comprenant les résidus 1-74, substituée par le domaine transactivateur de VP16 de séquence SEQ ID n° 2; une délétion de la partie C-terminale de la protéine p53, à partir du résidu 326, substituée par un domaine d'oligomérisation artificiel de séquence SEQ ID n°1, et une histidine en position 182.
⇒ pEC149 (VP16-75-336(H182)-lz) possédant une délétion de la partie N-terminale de la protéine p53 comprenant les résidus 1-74, substituée par le domaine transactivateur de VP16 de séquence SEQ ID n° 2; une délétion de la partie C-terminale de la protéine p53, à partir du résidu 337, substituée par un domaine d'oligomérisation artificiel de séquence SEQ ID n°1, et une histidine en position 182.

En raison des modifications mentionnées ci-avant, les variants de l'invention ont également des propriétés 'tueuses' que sont l'arrêt du cycle cellulaire et l'apoptose potentiellement améliorées. La combinaison des modification mentionnées, incluant la présence d'un domaine d'oligomérisation sélectif et un pouvoir transactivateur amélioré par substitution du domaine d'origine et par la présence d'une histidine en 182 confèrent en effet aux variants de l'invention des potentialités thérapeutiques nettement améliorées. En outre, les variants selon l'invention permettent d'éviter l'apparition de certains mutants (dominants oncogéniques). Les gains de fonction de certains mutants de p53 sont encore mal définis tant au niveau de leur mécanismes qu'au niveau des domaines de la protéine p53 impliqués. Il est fort probable que certaines de ces nouvelles fonctions vont dépendre de l'association à certains partenaires cellulaires effecteurs. L'élimination des domaines impliqués dans ces interactions, et dont les propriétés transformantes ont été mises en évidence, au sein des molécules décrites dans la présente demande sont de nature à empêcher l'apparition de ces gains de fonctions oncogéniques. Ainsi les mutations qui apparaitraient de façon aléatoire lors de la préparation des lots cliniques de plasmides codant pour les polypeptides décrits ou lors de la production de lots cliniques de vecteurs viraux ou chimiques codant pour ces mêmes polypeptides ne créeraient pas une sous-population de molécules oncogéniques.

Par ailleurs, en raison de la suppression de certains domaines de p53 indispensables pour la fixation de certaines molécules inhibitrices de sa fonction, les variants de l'invention présentent également une activité thérapeutique plus élevée et plus stable. Finalement, l'existence de motifs étrangers dans les différentes constructions de l'invention (protéine AS murine par exemple, domaine d'oligomérisation artificiel, etc) est susceptible de déclencher une réaction immunitaire lors de la mort des cellules transfectées et du relargage dans le milieu extracellulaire des ces différents fragments, augmentant ainsi la capacité du système immunitaire à lutter contre les cellules tumorales.

Selon un autre mode de réalisation, le domaine transactivateur hétérologue est un domaine transactivateur actif préférentiellement dans les cellules transformées et non dans les cellules saines avoisinantes. La présente invention décrit en effet également des molécules dont la fonction s'exerce essentiellement dans des cellules transformées et non dans les cellules saines avoisinantes. Bien qu'il semble que l'expression exogène d'une p53 sauvage au sein d'une cellule différenciée comportant de la p53 sauvage endogène ait peu ou pas d'effet sur la viabilité, il est néanmoins avantageux de pouvoir disposer d'une protéine qui ne serait fonctionnelle qu'au sein de la cellule ciblée. Cette spécificité de la cellule tumorale versus la cellule normale est actuellement beaucoup travaillée au niveau de la spécificité du ciblage du vecteur viral ou de la conception de systèmes d'expression spécifiques. La présente invention décrit maintenant des dérivés de p53 dont un des domaines fonctionnels est éteint en l'absence d'un activateur cellulaire présent essentiellement dans les cellules transformées.

Ainsi, un autre objet de l'invention concerne un variant de la protéine p53 actif préférentiellement dans les cellules transformées, dans lequel un au moins des domaines fonctionnels de p53 est délété en tout ou en partie et est substitué par un domaine hétérologue actif préférentiellement dans les cellules transformées. Préférentiellement, le domaine fonctionnel de p53 concerné est le domaine transactivateur. Ainsi, un objet particulièrement préféré de l'invention concerne un variant de la protéine p53 actif préférentiellement dans les cellules transformées, dans lequel le domaine transactivateur naturel est délété en tout ou en partie et est substitué par un domaine transactivateur actif préférentiellement dans les cellules transformées. Avantageusement, le domaine transactivateur naturel est délété par suppression des résidus 1-74 inclus de p53.

L'invention concerne plus particulièrement des variants de p53 qui sont fonctionnels spécifiquement en présence d'une protéine Ras oncogénique ou d'un mutant de p53. Ces molécules sont obtenues notamment par remplacement du domaine transactivateur de la protéine p53 sauvage par un domaine protéique capable de lier spécifiquement un transactivateur ou un complexe transactivateur présent dans une cellule transformée.

Le domaine protéique capable de lier spécifiquement le transactivateur transcriptionnel ou le complexe transactivateur transcriptionnel présent dans les molécules de l'invention peut être de différents types. Il peut s'agir en particulier d'un domaine d'oligomérisation dans le cas où le transactivateur ou le complexe transactivateur ciblé comporte également un tel domaine. Il peut également s'agir de tout domaine synthétique ou naturel connu pour interagir avec ledit transactivateur ou complexe transactivateur.

En outre, le terme actif préférentiellement indique que ces variants exercent leur activité essentiellement lorsqu'ils sont exprimés dans des cellules transformées. Une activité résiduelle peut toutefois exister dans les cellules non transformées, mais inférieure à celle observée dans les cellules transformées.

Selon une manière plus générale, l'invention concerne toute protéine chimère comprenant un domaine transactivateur, un domaine de liaison à l'ADN, un domaine d'adressage au noyau et un domaine d'oligomérisation, dans laquelle les domaines de liaison à l'ADN et d'adressage au noyau sont constitués par les acides aminés 75 à 325 de la protéine p53 sauvage humaine (SEQ ID n° 4). La demanderesse a en effet montré que cette région de la protéine p53, couplée à des domaines transactivateur et d'oligomérisation appropriés, permet la création de molécules de type p53 ayant des propriétés particulièrement avantageuses en terme de stabilité, de résistance aux effets négatifs des mutants de p53 et de sensibilité à l'inactivation par certains facteurs cellulaires.

Selon une variante, les domaines de liaison à l'ADN et d'adressage au noyau sont constitués par les acides aminés 75 à 336 de la protéine p53 sauvage humaine (SEQ ID n° 5).

Les protéines chimères selon l'invention peuvent comporter différents types de domaines transactivateur. Il peut s'agir du domaine transactivateur de la protéine p53. Préférentiellement, il s'agit d'un domaine transactivateur hétérologue, choisi par exemple parmi le domaine transactivateur de VP16 ou un domaine protéique capable de lier spécifiquement un transactivateur ou un complexe transactivateur présent dans une cellule transformée.

Concernant le domaine d'oligomérisation, il s'agit préférentiellement d'un domaine artificiel, et donc spécifique, tel que par exemple un leucine zipper artificiel, en particulier de séquence SEQ ID n° 1.

Les protéines chimères selon l'invention peuvent en outre comporter un résidu histidine en position 182.

Des exemples précis de protéines chimères telles que décrites dans la présente demande sont notamment pEC114, pEC116, pEC147 et pEC149.

La présente invention a également pour objet tout acide nucléique codant pour un variant ou une protéine chimère tel que défini ci-avant.

L'acide nucléique selon l'invention peut être un acide ribonucléique (ARN) ou désoxyribonucléique (ADN). En outre, il peut s'agir d'un ADN complémentaire (ADNc) éventuellement comprenant un ou plusieurs introns du gène p53. Il peut être d'origine humaine, animale, virale, synthétique ou semi-synthétique. Il peut être obtenu de différentes manières et notamment par synthèse chimique en utilisant les séquences présentées dans la demande et par exemple un synthétiseur d'acides nucléiques. Il peut également être obtenu par criblage de banques au moyen de sondes spécifiques, notamment telles que décrites dans la demande. II peut encore être obtenu par des techniques mixtes incluant la modification chimique (élongation, délétion, substitution, etc) de séquences criblées à partir de banques. D'une manière générale, les acides nucléiques de l'invention peuvent être préparés selon toute technique connue de l'homme du métier.

Préférentiellement, l'acide nucléique selon l'invention est un ADNc ou un ARN.

L'acide nucléique selon l'invention est avantageusement choisi parmi :
(a) tout ou partie des séquences SEQ ID n° 25, 26,27,28,29, 31, 32, 33 et 34 ou de leur brin complémentaire,
(b) toute séquence hybridant avec les séquences (a) et codant pour un dérivé selon l'invention,
(c) les variants de (a) et (b) résultant de la dégénérescence du code génétique.

Comme indiqué précédemment, la demanderesse a maintenant construit de nouvelles séquences d'acide nucléique codant pour des polypeptides variants de p53, ayant des propriétés antiprolifératives et apoptotiques tout à fait remarquables. Ces acides nucléiques peuvent être utilisés comme agents thérapeutiques pour produire dans les cellules des dérivés selon l'invention capables de détruire ou de corriger des dysfonctionnements cellulaires. A cet effet, la présente invention concerne également toute cassette d'expression comprenant un acide nucléique tel que défini ci-avant, un promoteur permettant son expression et un signal de terminaison de la transcription. Le promoteur est avantageusement choisi parmi les promoteurs fonctionnels dans les cellules mammifères, de préférence humaines. Plus préférentiellement, il s'agit d'un promoteur permettant l'expression d'un acide nucléique dans une cellule hyperproliférative (cancéreuse, resténose, etc). A cet égard, différents promoteurs peuvent être utilisés. Il peut s'agir par exemple du propre promoteur du gène p53. Il peut également s'agir de régions d'origine différente (responsables de l'expression d'autres protéines, ou même synthétiques). Il peut ainsi s'agir de tout promoteur ou séquence dérivée stimulant ou réprimant la transcription d'un gène de façon spécifique ou non, inductible ou non, forte ou faible. On peut citer notamment les séquences promotrices de gènes eucaryotes ou viraux. Par exemple, il peut s'agir de séquences promotrices issues du génome de la cellule cible. Parmi les promoteurs eucaryotes, on peut utiliser en particulier de promoteurs ubiquitaires (promoteur des gènes HPRT, PGK, a-actine, tubuline, etc), de promoteurs des filaments intermédiaires (promoteur des gènes GFAP, desmine, vimentine, neurofilaments, kératine, etc), de promoteurs de gènes thérapeutiques (par exemple le promoteur des gènes MDR, CFTR, Facteur VIII, ApoAI, etc), de promoteurs spécifiques de tissus (promoteur du gène pyruvate kinase, villine, protéine intestinale de liaison des acides gras, a-actine du muscle lisse, etc) ou encore de promoteurs répondant à un stimulus (récepteur des hormones stéroïdes, récepteur de l'acide rétinoïque, etc). De même, il peut s'agir de séquences promotrices issues du génome d'un virus, tel que par exemple les promoteurs des gènes E1A et MLP d'adénovirus, le promoteur précoce du CMV, ou encore le promoteur du LTR du RSV, etc. En outre, ces régions promotrices peuvent être modifiées par addition de séquences d'activation, de régulation, ou permettant une expression tissu-spécifique ou majoritaire.

La présente invention fournit maintenant de nouveaux agents thérapeutiques permettant, par leurs propriétés antiprolifératives et/ou apoptotiques d'interférer avec de nombreux dysfonctionnements cellulaires. Dans ce but, les acides nucléiques ou cassettes selon l'invention peuvent être injectés tels quels au niveau du site à traiter, ou incubés directement avec les cellules à détruire ou traiter. Il a en effet été décrit que les acides nucléiques nus pouvaient pénétrer dans les cellules sans vecteur particulier. Néanmoins, on préfère dans le cadre de la présente invention utiliser un vecteur d'administration, permettant d'améliorer (i) l'efficacité de la pénétration cellulaire, (ii) le ciblage (iii) la stabilité extra- et intracellulaires.

Dans un mode de mise en oeuvre particulièrement préféré de la présente invention l'acide nucléique ou la cassette est incorporé dans un vecteur. Le vecteur utilisé peut être d'origine chimique (liposome, nanoparticule, complexe peptidique, lipides ou polymères cationiques, etc) virale (rétrovirus, Adénovirus, virus de l'herpès, AAV, virus de la vaccine, etc) ou plasmidique.

L'utilisation de vecteurs viraux repose sur les propriétés naturelles de transfection des virus. Il est ainsi possible d'utiliser par exemple les adénovirus, les herpès virus, les rétrovirus et les virus adéno associés. Ces vecteurs s'avèrent particulièrement performants sur le plan de la transfection. A cet égard, un objet préféré selon l'invention réside dans un rétrovirus recombinant défectif dont le génome comprend un acide nucléique tel que défini ci-avant. Un autre objet particulier de l'invention réside dans un adénovirus recombinant défectif dont le génome comprend un acide nucléique tel que défini ci-avant.

Le vecteur selon l'invention peut également être un agent non viral capable de promouvoir le transfert et l'expression d'acides nucléiques dans des cellules eucaryotes. Les vecteurs chimiques ou biochimiques, synthétiques ou naturels, représentent une alternative intéressante aux virus naturels en particulier pour des raisons de commodité, de sécurité et également par l'absence de limite théorique en ce qui concerne la taille de l'ADN à transfecter. Ces vecteurs synthétiques ont deux fonctions principales, compacter l'acide nucléique à transfecter et promouvoir sa fixation cellulaire ainsi que son passage à travers la membrane plasmique et, le cas échéant, les deux membranes nucléaires. Pour pallier à la nature polyanionique des acides nucléiques, les vecteurs non viraux possèdent tous des charges polycationiques.

L'acide nucléique ou le vecteur utilisé dans la présente invention peut être formulé en vue d'administrations par voie topique, orale, parentérale, intranasale, intraveineuse, intramusculaire, sous-cutanée, intraoculaire, transdermique, etc. De préférence, l'acide nucléique ou le vecteur est utilisé sous une forme injectable. Il peut donc être mélangé à tout véhicule pharmaceutiquement acceptable pour une formulation injectable, notamment pour une injection directe au niveau du site à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Une injection directe de l'acide nucléique dans la tumeur du patient est intéressante car elle permet de concentrer l'effet thérapeutique au niveau des tissus affectés. Les doses d'acide nucléique utilisées peuvent être adaptées en fonction de différents paramètres, et notamment en fonction du gène, du vecteur, du mode d'administration utilisé, de la pathologie concernée ou encore de la durée du traitement recherchée.

L'invention concerne également toute composition pharmaceutique comprenant au moins un acide nucléique tel que défini ci-avant.

Elle concerne également toute composition pharmaceutique comprenant au moins un vecteur tel que défini ci-avant.

Elle concerne aussi toute composition pharmaceutique comprenant au moins un variant de p53 tel que défini ci-avant.

En raison de leurs propriétés antiprolifératives, les compositions pharmaceutiques selon l'invention sont tout particulièrement adaptées pour le traitement des désordres hyperprolifératifs, tels que notamment les cancers et la resténose. La présente invention fournit ainsi une méthode particulièrement efficace pour la destruction de cellules, notamment de cellules hyperprolifératives. Elle peut être utilisée in vitro ou ex vivo. Ex vivo, elle consiste essentiellement à incuber les cellules en présence d'un ou plusieurs acides nucléiques (ou d'un vecteur, ou cassette ou directement du dérivé). In vivo, elle consiste à administrer à l'organisme une quantité active d'un vecteur (ou d'une cassette) selon l'invention, de préférence directement au niveau du site à traiter (tumeur notamment). A cet égard, l'invention a également pour objet une méthode de destruction de cellules hyperprolifératives comprenant la mise en contact desdites cellules ou d'une partie d'entre-elles avec un acide nucléique tel que défini ci-avant.

La présente invention est avantageusement utilisée in vivo pour la destruction de cellules en hyperprolifération (i.e. en prolifération anormale). Elle est ainsi applicable à la destruction des cellules tumorales ou des cellules de muscle lisse de la paroi vasculaire (resténose). Elle est tout particulièrement appropriée au traitement des cancers dans lesquels un mutant de p53 est observé. A titre d'exemple, on peut citer les adénocarcinomes du colon, les cancers de la thyroïde, les carcinomes du poumon, les leucémies myéloïdes, les cancers colorectaux, les cancers du sein, les cancers du poumon, les cancers gastriques, les cancers de l'oesophage, les lymphômes B, les cancers ovariens, les cancers de la vessie, les glioblastomes, les hépatocarcinomes, les cancers des os, de la peau, du pancréas ou encore les cancers du rein et de la prostate, les cancers de l'oesophage, les cancers du larynx, les cancers tête et cou, les cancers ano-génitaux HPV positifs, les cancers du nasopharynx EBV positifs, les cancers dans lesquels la protéine cellulaire mdm2 est surexprimée, etc.

Les variants de l'invention sont particulièrement efficaces pour le traitement des cancers dans lesquels la protéine MDM2 est en outre surexprimée, ainsi que des cancers liés au virus HPV tels que les cancers ano-génitaux HPV positifs.

La présente invention est décrite plus en détail dans les exemples qui suivent, qui doivent être considérés comme illustratifs et non limitatifs.

### Légende des Figures

Figure 1 : Domaines fonctionnels de la protéine p53 sauvage. TA : Domaine activateur de la transcription; DNB : domaine de liaison à l'ADN; NLS : signal de localisation nucléaire; OL : domaine d'oligomérisation; REG : domaine de régulation.
Figure 2 : Construction d'un ADNc codant pour la forme AS de la p53.
Figure 3 : Clonage des ADNc codant pour les constructions pEC104, pEC106, pEC131, pEC132 et pEC133 et pour leur variant H182.
Figure 4 : Construction des variants pEC107, pEC110, pEC139 et pEC140 par fusion avec le domaine d'oligomérisation artificiel.
Figure 5 : Construction des variants pEC114, pEC116, pEC141, pEC143, pEC145, pEC147, pEC149, pEC151, pEC153 et pEC155.

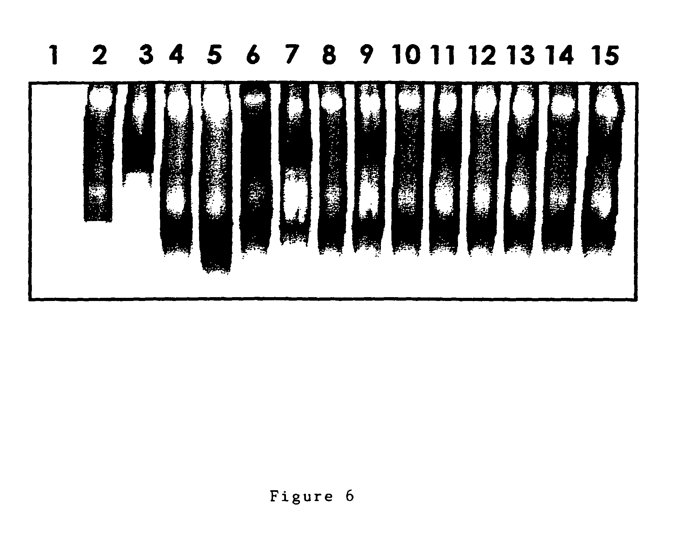

Figure 6 : Reconnaissance de séquences d'ADN double brin spécifiques par les molécules hybrides de l'invention. Expérience de retard sur gel: compétition entre HisV325 et p53 sauvage. colonne 1: incubation en l'absence de HisV325 et p53 sauvage, colonne 2: 30 ng p53 sauvage, colonne 3: idem 2 + pAb421, colonne 4: 30 ng HisV325, colonne 5: idem 4 + pAb421, colonne 6: 30 ng HisV325 + 30 ng p53 sauvage, colonne 7: idem 6 + pAb421, colonne 8: 30 ng HisV325 + 15 ng p53 sauvage, colonne 9: idem 8 + pAb421, colonne 10: 30 ng HisV325 + 7.5 ng p53 sauvage, colonne 11: idem 10 + pAb421, colonne 12: 30 ng HisV325 + 4.5 ng p53 sauvage, colonne 13: idem 12 + pAb421, colonne 14: 30 ng HisV325 + 3 ng p53 sauvage, colonne 15: idem 14 + pAb421
Figure 7 : Activité transactivatrice de la protéine p53 sauvage et des variants AS, V-325 et V-336.
Figure 8 : Activité transactivatrice de la protéine p53 sauvage et des variants V-325, V-336 et V-343.
Figure 9 : Expression des variants de l'invention dans les cellules SAOS-2
Figure 10 : Induction des gènes hdm2 et WAF1 dans les cellules EB, EB-1 et EB-V325
Figure 11 : Effet de la protéine E6 sur la fonction transactivatrice de la protéine p53 et des variants de l'invention dans les cellules SAOS-2. Quantité des vecteurs CMV-construction = 100ng
Figure 12 : Effet de la protéine E6 sur la fonction transactivatrice de la protéine p53 et des variants de l'invention dans les cellules HeLa.
Figure 13: Sensibilité de la protéine p53 sauvage et des variants de l'invention à la dégradation induite par la protéine E6.
Figure 14 : Effet du mutant dominant-négatif de p53 H175 sur la fonction transactivatrice des variants de l'invention. Quantité des vecteurs CMV-construction = 100ng
Figure 15 : Effet de la protéine hdm2 sur la fonction transactivatrice de la protéine p53 et des variants de l'invention dans les cellules SAOS-2. Quantité des vecteurs CMV-construction = 100ng
Figure 16 : Effet des protéines p53 sauvage et V-325 sur la croissance de cellules surexprimant la protéine hdm2.
Figure 17 : Induction de l'apoptose par les protéines p53 sauvage et V-325.
Figure 18 : Cinétique d'induction de l'apoptose dans les cellules EB, EB-1 et EB-V325

### Exemples

### Exemple A. Construction de differents fragments nucleotidiques necessaires a la realisation des genes codant pour les variants de la proteine p53

### A1. Construction du cDNA codant pour la p53 sauvage humaine

Le gène de la p53 humaine a été cloné par réaction d'amplification en chaine (PCR) sur de l'ADN d'une banque de placenta humain (Clontech) en utilisant les oligonucléotides 5'-1 et 3'-393.

Ce produit a ensuite été cloné directement après PCR dans le vecteur pCRII (Invitrogène).

### A2 - Construction d'un ADNc codant pour la forme AS de la p53

La forme AS de la p53 comprend un fragment codant pour les acides aminés 1 à 366 de la protéine p53 humaine additionné des 19 derniers acides aminés du produit d'épissage alternatif de la protéine p53 murine.

La forme AS de la p53 a été obtenue en deux étapes:
- amplification par PCR d'un fragment codant pour les acides aminés 1 à 367 de la protéine p53 en utilisant les oligonucléotides 5'-1 (Cf exemple A1) et 3'-367.
   Le fragment PCR ainsi obtenu a ensuite été cloné dans le vecteur pCRII (Invitrogène). Le fragment ainsi cloné possède un site de reconnaissance pour l'enzyme de restriction Xho I (fragment 1-367).
- les oligonucléotides 5'-AS1, 5'-AS2, 3'-AS1 et 3'-AS2 ont été phosphorylés puis hybridés ensemble pour constituer le fragment codant pour les 19 derniers acides aminés du produit d'épissage alternatif de la protéine p53 murine.

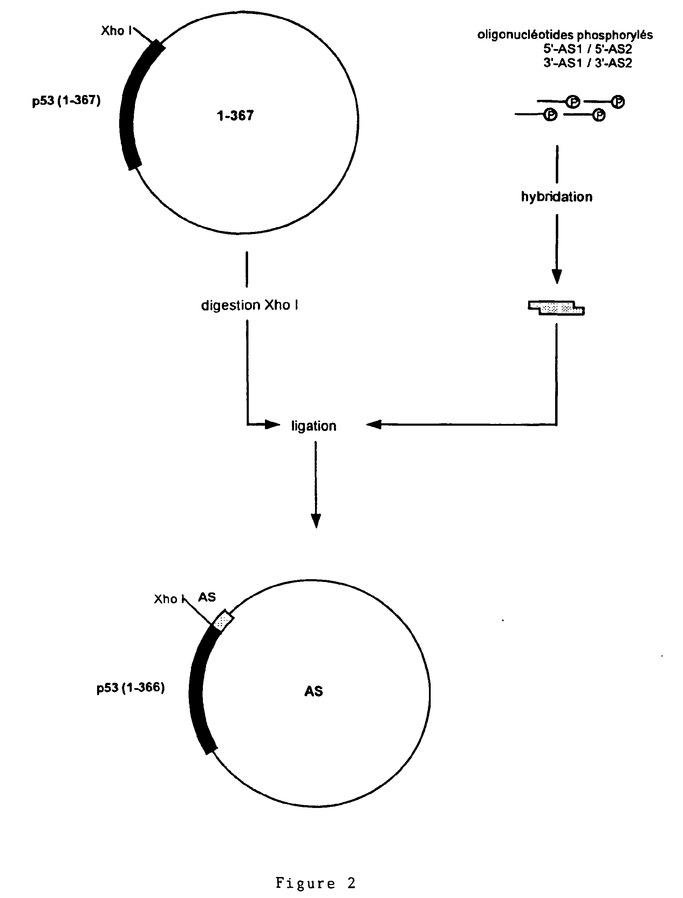

Ce fragment a ensuite été inseré au niveau du site Xho I du fragment 1-367 (voir figure 2). Le gène ainsi construit code pour le variant humain du produit d'épissage alternatif de la protéine p53 murine (AS).

La séquence protéique ainsi modifiée est la suivante:

### A3 - Construction d'ADNc codant pour différents fragments de la protéine p53 portant le domaine de liaison à l'ADN.

Cet exemple décrit la construction de différents ADNc codant pour différents fragments de la protéine p53 humaine, portant tout ou partie du domaine de liaison à l'ADN de p53. Ces fragments sont ensuite utilisés dans la construction des variants de p53. Ces fragments ont été obtenus par réaction d'amplification par la polymérase sur les matrices décrites dans les exemples A1 et A2 au moyen de différents oligonucléotides. Les réactions d'amplification ont été réalisées dans les conditions décrites dans l'exemple A4.1.

### A3.1 - Construction d'un ADNc codant pour la région 75-325 de p53 et de son dérivé H182

Cet exemple décrit la construction d'un ADNc codant pour les acides aminés 75 à 325 de la protéine p53 humaine sauvage (75-325).

Cet ADNc a été obtenu par réaction d'amplification en chaine (PCR) sur l'ADN de p53 (décrit dans l'exemple A1) avec les oligonucléotides 5'-75 et 3'-325 suivants :

Un dérivé de ce fragment portant une mutation ponctuelle sur l'acide aminé 182 de la protéine p53 humaine (cystéine -> Histidine) a été obtenu par mutagénèse dirigée au moyen du kit Amersham, en utilisant l'oligonucléotide H182 de séquence :

Ce fragment a été désigné 75-325(H182).

### A3.2 - Construction d'un ADNc codant pour la région 75-336 de p53 et de son dérivé H 182

Cet exemple décrit la construction d'un ADNc codant pour les acides aminés 75 à 336 de la protéine p53 humaine sauvage (75-336).

Cet ADNc a été obtenu par réaction d'amplification en chaine (PCR) sur l'ADN de p53 (décrit dans l'exemple A1) avec les oligonucléotides 5'-75 (SEQ ID n° 13) et 3'-336 suivant :

Un dérivé de ce fragment portant une mutation ponctuelle sur l'acide aminé 182 de la protéine p53 humaine (cystéine -> Histidine) a été obtenu par mutagénèse dirigée au moyen du kit Amersham, en utilisant l'oligonucléotide H182 (SEQ ID n° 15). Ce fragment a été désigné 75-336(H 182).

### A3.3 - Construction d'un ADNc codant pour la région 75-343 de p53

Cet exemple décrit la construction d'un ADNc codant pour les acides aminés 75 à 343 de la protéine p53 humaine sauvage (75-343).

Cet ADNc a été obtenu par réaction d'amplification en chaine (PCR) sur l'ADN de p53 (décrit dans l'exemple A1) avec les oligonucléotides 5'-75 (SEQ ID n°13) et 3'-343 suivant :

### A3.4 - Construction d'un ADNc codant pour la région 75-367 de p53 et de son dérivé H 182

Cet exemple décrit la construction d'un ADNc codant pour les acides aminés 75 à 367 de la protéine p53 humaine sauvage (75-367).

Ce fragment a été obtenu par réaction d'amplification en chaine (PCR) sur l'ADN de p53 décrit dans l'exemple A1 avec les oligonucléotides 5'-75 (SEQ ID n° 13) et 3'-367 (SEQ ID n° 8).

Le fragment ainsi obtenu inclut un site de reconnaissance par l'endonucléase Xhol (75-367).

Un dérivé de ce fragment portant une mutation ponctuelle sur l'acide aminé 182 de la protéine p53 humaine (cystéine -> Histidine) a été obtenu par mutagénèse dirigée au moyen du kit Amersham, en utilisant l'oligonucléotide H182 (SEQ ID n° 15). Ce fragment a été désigné 75-367(H182).

### A3.5 - Construction d'un ADNc codant pour le fragment 75-AS et de son dérivé H 182

Cet exemple décrit la construction d'un ADNc codant pour les acides aminés 75 à 366 de la protéine p53 humaine sauvage (75-366) additioné des 19 derniers acides aminés du produit de splicing alternatif de la protéine p53 murine.

Ce fragment a été obtenu par réaction d'amplification en chaine (PCR) sur l'ADN du fragment AS décrit dans l'exemple A2 avec les oligonucléotides 5'-75 (SEQ ID n° 13) et 3'-AS2 (SEQ ID n°12).

Un dérivé de ce fragment portant une mutation ponctuelle sur l'acide aminé 182 de la protéine p53 humaine (cystéine -> Histidine) a été obtenu par mutagénèse dirigée au moyen du kit Amersham, en utilisant l'oligonucléotide H182 (SEQ ID n° 15). Ce fragment a été désigné 75-AS(H182).

### A3.6 - Construction d'un ADNc codant pour le fragment 75-393 de p53 et de son dérivé H 182

Cet exemple décrit la construction d'un ADNc codant pour les acides aminés 75 à 393 de la protéine p53 humaine (75-393). Ce fragment a été obtenu par réaction d'amplification en chaine (PCR) sur l'ADN p53 décrit dans l'exemple A1 avec les oligonucléotides 5'-75 (SEQ ID n° 13) et 3'-393 (SEQ ID n° 7).

Un dérivé de ce fragment portant une mutation ponctuelle sur l'acide aminé 182 de la protéine p53 humaine (cystéine -> Histidine) a été obtenu par mutagénèse dirigée au moyen du kit Amersham, en utilisant l'oligonucléotide H182 (SEQ ID n° 15). Ce fragment a été désigné 75-393(H 182).

### A4 - Construction d'ADNc codant pour différents fragments portant un domaine activateur de la transcription (domaine transactivateur).

Cet exemple décrit la construction de différents ADNc codant pour différents fragments portant un domaine transactivateur. Ces fragments sont ensuite utilisés dans la construction des variants de p53.

### A4.1 - Réactions de PCR

Les différents fragments ont été obtenus par réaction d'amplification par la polymérase sur différentes matrices au moyen de différents oligonucléotides. Les réactions d'amplification ont été réalisées dans les conditions suivantes : Enzyme Amplitaq DNA polymerase (Perkin-Elmer) dans le tampon fourni par le fournisseur, avec une concentration de dNTP de 0,2 mM, 100 ng de matrice et 500 ng de chacun des deux oligonucléotides.
- cycle: 2 min à 91°C
- cycles: 1 min à 91°C
   1 min à 55°C
   1 min à 72°C
- cycle: 5 min à 72°C

### A4.2 - Construction d'un ADNc codant pour la région 411-490 de la protéine virale VP16 (VP16 TA)

Cet exemple décrit la construction d'un ADNc codant pour les acides aminés 411-490 de la protéine virale VP16 (VP16 TA). Cette région porte le domaine transactivateur de cette protéine.

Le fragment transactivateur dérivé de la protéine virale VP16 (411-490) du virus de l'herpès simplex a été obtenu par réaction d'amplification en chaine (PCR) en utilisant les conditions précédemment définies (Cf A4.1) et les oligonucléotides 5'-VP16 et 3'-VP16 suivants: et 100 ng du plasmide pUHD15-1 (Gossen & Bujard, Proc. Natl. Acad. Sci. USA 89 (1992) 5547)

Le fragment ainsi obtenu comprend 334 paires de bases dont la séquence est donnée SEQ ID n° 2. Il comprend dans sa partie N-terminale un résidu méthionine apporté par le site d'initiation de la transcription (ATG), ajouté lors de l'étape de clonage par PCR.

A4.2 - Construction d'ADNc codant pour différents fragments capables de recruter le domaine activateur de la transcription (domaine transactivateur) d'une protéine p53 endogène.

A4.2.1 - Construction d'un ADNc codant pour un anticorps simple chaine capable de lier le protéine p53 (ScFv 421)

Cet exemple décrit la construction d'un ADNc codant pour un anticorps simple chaine capable de lier la protéine p53 (ScFv 421). Cette construction exprimée au niveau intracellulaire, doit être capable de fixer une protéine p53 endogène sauvage ou mutante pour recruter son domaine transactivateur.

Le cDNA codant pour le ScFv 421 (demande de brevet PCT/FR96/00477) peut-être extrait sous la forme d'un fragment Nco I / Not I qui comprend un site d'initiation de la traduction (ATG) et aucune séquence d'arrêt de la traduction.

Le fragment ainsi obtenu comprend 766 paires de bases dont la séquence est donnée SEQ ID n° 36.

A4.2.2 - Construction d'un ADNc codant pour la région 325-360 de la protéine p53 sauvage (325-360)

Cet exemple décrit la construction d'un ADNc codant pour les acides aminés 325-360 de la protéine p53 sauvage (325-360). Cette région porte le domaine d'oligomérisation de cette protéine. Cette construction exprimée au niveau intracellulaire, doit être capable de fixer une protéine p53 endogène sauvage ou mutante pour recruter son domaine transactivateur.

Ce domaine d'oligomérisation dérivé de la protéine p53 sauvage humaine (325-360) a été obtenu par réaction d'amplification en chaine (PCR) en utilisant les conditions précédemment définies (Cf A4.1) et les oligonucléotides 5'-325 et 3'-360 suivants: sur 100 ng de l'ADN de p53 (décrit dans l'exemple A1).

Le fragment ainsi obtenu comprend 141 paires de bases dont la séquence est donnée SEQ ID n° 39. Il comprend dans sa partie N-terminale un résidu méthionine apporté par le site d'initiation de la traduction (ATG), ajouté lors de l'étape de clonage par PCR et aucune séquence d'arrêt de la traduction.

### A4.2.3 - Construction d'un ADNc codant pour la région 325-393 de la protéine p53 sauvage (325-393)

Cet exemple décrit la construction d'un ADNc codant pour les acides aminés 325-393 de la protéine p53 sauvage (325-393). Cette région porte le domaine d'oligomérisation de cette protéine. Cette construction exprimée au niveau intracellulaire, doit être capable de fixer une protéine p53 endogène sauvage ou mutante pour recruter son domaine transactivateur.

Ce domaine d'oligomérisation dérivé de la protéine p53 sauvage humaine (325-360) a été obtenu par réaction d'amplification en chaine (PCR) en utilisant les conditions précédemment définies (Cf A4.1) et les oligonucléotides 5'-325 (SEQ ID n° 37) et 3'-393.2 suivant: sur 100 ng de l'ADN de p53 (décrit dans l'exemple A1).

Le fragment ainsi obtenu comprend 243 paires de bases dont la séquence est donnée SEQ ID n° 41. Il comprend dans sa partie N-terminale un résidu méthionine apporté par le site d'initiation de la traduction (ATG), ajouté lors de l'étape de clonage par PCR et aucune séquence d'arrêt de la traduction.

### A5 - Construction d'ADNc codant pour différents fragments portant un domaine d'oligomérisation.

Cet exemple décrit la construction de différents ADNc codant pour différents fragments portant un domaine d'oligomérisation. Ces fragments sont ensuite utilisés dans la construction des variants de p53. Ces fragments ont été obtenus par réaction d'amplification par la polymérase sur différentes matrices (p53 pour le domaine homologue et matrices d'origine différentes pour les domaines d'oligomérisation hétérologues, notamment artificiels) au moyen de différents oligonucléotides. Les réactions d'amplification ont été réalisées dans les conditions décrites en A4.1 ci-dessus.

### A5.1 - Construction d'un ADNc comprenant un domaine d'oligomérisation artificiel.

Cet exemple décrit la construction d'un ADNc comprenant un domaine d'oligomérisation artificiel, constitué d'un leucine zipper artificiel. Cet ADNc est ensuite utilisé pour la construction de variants à partir des fragments 75-325 (exemple A3.1) et 75-336 (exemple A3.2.) de la protéine p53 humaine et de leurs dérivés modifiés au niveau de la cystéine 182.

Cet ADNc a été construit à partir des 6 oligonucléotides suivants.

Ces oligonucléotides ont été synthétisés au moyen d'un synthétiseur automatique d'ADN, en utilisant la chimie des phosphoramidites. Ces six oligonucléotides présentent des complémentarités deux à deux (Iz1-5'/Iz1-3', Iz2-5'/Iz2-3', Iz3-5'/Iz3-3') et des complémentarités chevauchantes (Iz1-3'/Iz2-5', Iz2-3'/Iz3-5') permettant l'obtention du domaine d'oligomérisation par simple hybridation et ligation. La séquence LZ résultante est donnée SEQ ID n° 1.

### A5.2 - Construction d'un ADNc comprenant le domaine d'oligomérisation naturel de la p53 humaine.

Cet exemple décrit la construction d'un ADNc comprenant le domaine d'oligomérisation naturel de la p53 humaine. Cet ADNc est représenté par le fragment codant pour les acides aminés 325 à 356 de p53 contenu dans les constructions 75-367 (exemple A3.4), 75-AS (exemple A3.5), 75-393 (exemple A3.6) et de leurs dérivés modifiés au niveau de la cystéine 182.

### Exemple B - Construction des genes codant pour differents variants de la protéine p53

### B1 - Clonage des differents fragments de p53

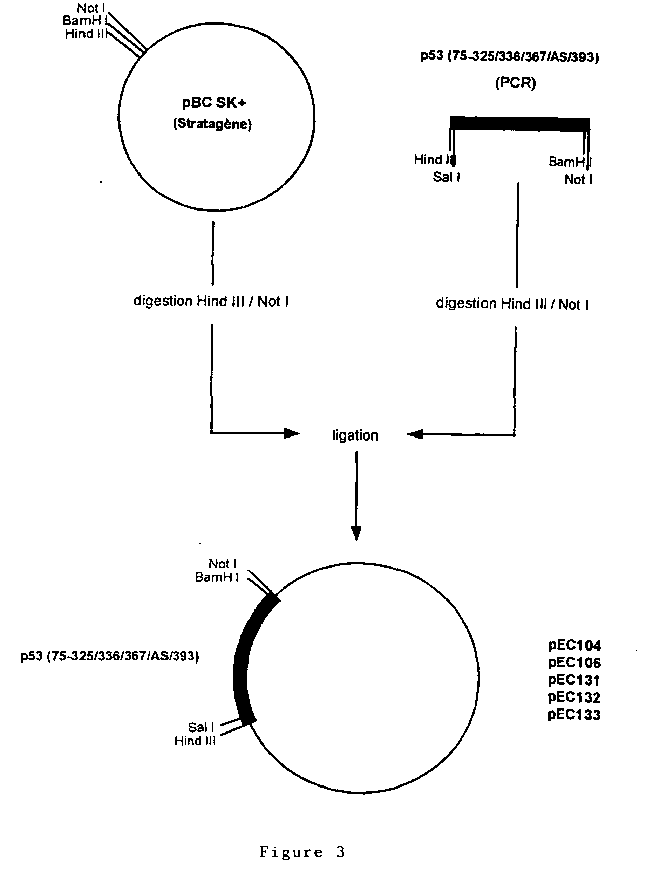

Chacun des différents fragments obtenus par PCR décrit dans l'exemple A a été cloné après PCR dans le vecteur pBC SK+ (Stratagène) en utilisant les sites de reconnaissance par les enzymes de restriction Hind III et Not I (Figure 3).

Les produits de ces constructions portent les numéros suivants:

| | |
|---|---|
| 75-325 | --> pEC 104 |
| 75-336 | --> pEC 106 |
| 75-343 | --> pEC 171 |
| 75-367 | --> pEC 131 |
| 75-AS | --> pEC 132 |
| 75-393 | --> pEC 133 |

A partir de ces produits et par mutagénèse dirigée en utilisant l'oligonucleotide-directed in vitro mutagenesis system (Amersham) et l'oligonucléotide H182, ont été obtenues les constructions correspondantes portant une histidine en position 182. Ces constructions portent les numéros suivants:

| | |
|---|---|
| 75-325(H182) | --> pEC 134 |
| 75-336(H182) | --> pEC 135 |
| 75-367(H182) | --> pEC 136 |
| 75-AS(H182) | --> pEC 137 |
| 75-393(H182) | --> pEC 138 |

### B2 - Fusion du leucine-zipper aux fragment 75-325, 75-336 et 75-343 et à leur variant H182

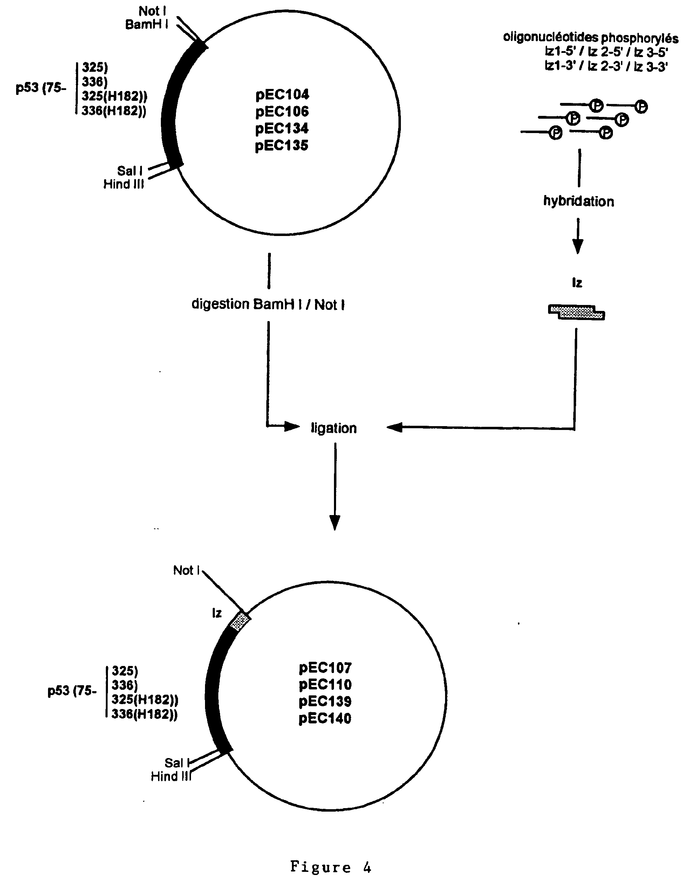

Les oligonucléotides constituant le leucine-zipper (Iz1-5', Iz1-3', Iz2-5', Iz2-3', Iz3-5' et Iz3-3') ont été phosphorylés à l'aide de la T4 kinase, puis hybridés tous ensemble et insérés dans les vecteurs pEC 104, 106, 134 et 135 et 171 préalablement digérés par les enzymes de restriction BamHI et NotI (Figure 4).

Les produits de ces constructions portent les numéros suivants:

| | |
|---|---|
| 75-325-Iz | --> pEC 107 |
| 75-336-Iz | --> pEC 110 |
| 75-343-Iz | ---> pEC 174 |
| 75-325(H182)-Iz | --> pEC 139 |
| 75-336(H182)-Iz | --> pEC 140 |

### B3 - Fusion du domaine activateur de la transcription à l'ensemble des fragments p53

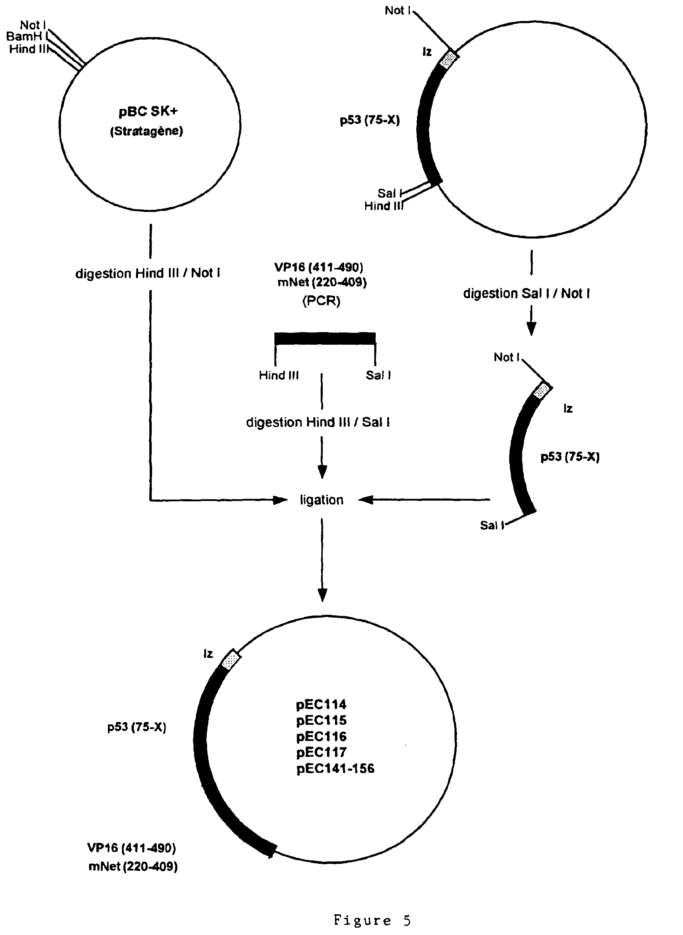

Les produits finaux ont été obtenus par une ligation à trois partenaires effectuée de la façon suivante (Figure 5):
Le domaine activateur de la transcription dérivé de VP16 décrit dans l'exemple A3 a été préparé par digestion enzymatique des produits de PCR par les enzymes de restriction Hind III et Sal I.
Les différents fragments p53 précédement obtenus (75-325-lz, 75-336-lz, 75-343-lz, 75-AS, 75-367, 75-393 et leurs variants H182) ont été isolés après digestion enzymatique des plasmides les contenant par les enzymes de restriction SalI et NotI.

Les combinaisons possibles (domaine activateur/p53) ont été constituées et insérées simultanément dans le vecteur pBC SK+ (Stratagène) préalablement digéré par les enzymes de restriction Hind III et Not I.

Les produits de ces constructions portent les numéros suivants:

| | | |
|---|---|---|
| VP16-75-325-lz | V-325 | --> pEC 114 (SEQ ID n° 25) |
| VP16-75-336-lz | V-336 | --> pEC 116 (SEQ ID n° 26) |
| VP16-75-367 | V-367 | --> pEC 141 (SEQ ID n° 27) |
| VP16-75-AS | V-AS | --> pEC 143 (SEQ ID n° 28) |
| VP16-75-393 | V-393 | --> pEC 145 (SEQ ID n° 29) |
| VP16-75-343-Iz | V-343 | --> pEC 175 (SEQ ID n° 30) |
| VP16-75-325(H182)-lz | V-325H | --> pEC 147 |
| VP16-75-336(H182)-lz | V-336H | --> pEC 149 |
| VP16-75-367(H182) | V-367H | --> pEC 151 |
| VP16-75-AS(H182) | V-ASH | --> pEC 153 |
| VP16-75-393(H182) | V-393H | --> pEC 155 |

Les produits correspondants portant un domaine liant spécifiquement un transactivateur ou un complexe transactivateur à la place du domaine VP16 sont construits de la même façon. Ces constructions sont désignées ci-dessous :

| | | |
|---|---|---|
| ScFv-75-325-Iz | S-325 | --> pEC 176 (SEQ ID n° 31) |
| ScFv-75-336-Iz | S-336 | |
| ScFv-75-367 | S-367 | |
| ScFv-75-AS | S-AS | |
| ScFv-75-393 | S-393 | |
| ScFv-75-325(H182)-lz | S-325H | |
| ScFv-75-336(H182)-lz | S-336H | |
| ScFv-75-367(H182) | S-367H | |
| ScFv-75-AS(H182) | S-ASH | |
| ScFv-75-393(H182) | S-393H | |
| (325-393)-75-325-lz | 393-325 | --> pEC 177 (SEQ ID n° 32) |
| (325-393)-75-336-lz | 393-336 | |
| (325-393)-75-367 | 393-367 | |
| (325-393)-75-AS | 393-AS | |
| (325-393)-75-393 | 393-393 | |
| (325-393)-75-325(H182)-Iz | 393-325H | |
| (325-393)-75-336(H182)-Iz | 393-336H | |
| (325-393)-75-367(H182) | 393-367H | |
| (325-393)-75-AS(H182) | 393-ASH | |
| (325-393)-75-393(H182) | 393-393H | |
| (325-360)-75-325-Iz | 360-325 | --> pEC 178 (SEQ ID n° 33) |
| (325-360)-75-336-Iz | 360-336 | |
| (325-360)-75-367 | 360-367 | |
| (325-360)-75-AS | 360-AS | |
| (325-360)-75-393 | 360-393 | |
| (325-360)-75-325(H182)-lz | 360-325H | |
| (325-360)-75-336(H182)-lz | 360-336H | |
| (325-360)-75-367(H182) | 360-367H | |
| (325-360)-75-AS(H182) | 360-ASH | |
| (325-360)-75-393(H182) | 360-393H | |

Les produits contenant le domaine 325-360 de la p53 en remplacement du domaine transactivateur (1-74) peuvent se voir additionner un séparateur synthétique (Hinge) obtenu par insertion au site Sal I d'un fragment d'ADN obtenu par hybridation de la paire d'oligonucléotides synthétiques complémentaires Hinge-up et Hinge-down suivants:

La séquence d'ADN double brin Hinge résultante est la suivante et la séquence protéique correspondante est (SEQ ID n° 44): Les produits correspondants sont désignées ci-dessous :

| | |
|---|---|
| (325-360)-Hinge-75-325-Iz | 360h-325--> pEC 179 (SEQ ID n° 34) |
| (325-360)-Hinge-75-336-Iz | 360h-336 |
| (325-360)-Hinge-75-367 | 360h-367 |
| (325-360)-Hinge-75-AS | 360h-AS |
| (325-360)-Hinge-75-393 | 360h-393 |
| (325-360)-Hinge-75-325(H182)-Iz | 360h-325H |
| (325-360)-Hinge-75-336(H182)-Iz | 360h-336H |
| (325-360)-Hinge-75-367(H182) | 360h-367H |
| (325-360)-Hinge-75-AS(H182) | 360h-ASH |
| (325-360)-Hinge-75-393(H182) | 360h-393H |

### Exemple C - Construction de vecteurs d'expression des variants de p53

Cet exemple décrit la construction de vecteurs utilisable pour le transfert des acides nucléiques de l'invention in vitro ou in vivo.

### C1 - Construction de vecteurs plasmidiques

Pour la construction de vecteurs plasmidiques, 2 types de vecteurs ont été utilisés.
- Le vecteur pSV2, décrit dans DNA Cloning, A practical approach Vol.2, D.M. Glover (Ed) IRL Press, Oxford, Washington DC, 1985. Ce vecteur est un vecteur d'expression eucaryote. Les acides nucléiques codant pour les variants ont été insérés dans ce vecteur sous forme de fragments Hpal-EcoRV. Ils sont ainsi placés sous le controle du promoteur de l'enhancer du virus SV40.
- Le vecteur pCDNA3 (Invitrogen). Il s'agit également d'un vecteur d'expression eucaryote. Les acides nucléiques codant pour les variants de l'invention sont ainsi placés, dans ce vecteur, sous le controle du promoteur précoce du CMV. Toutes les constructions décrites dans l'exemple B3 ont été introduites dans ce vecteur sous forme d'un fragment Hind III / Not I pour être testées dans les différents systèmes d'évaluation *in vivo*.

### C2 - Construction de vecteurs viraux

Selon un mode particulier, l'invention réside dans la construction et l'utilisation de vecteurs viraux permettant le transfert et l'expression in vivo des acides nucléiques tels que définis ci-avant.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprenent les ITR, une séquence permettant l'encapsidation et un acide nucléique selon l'invention. Encore plus préférentiellement, dans le génome des adénovirus de l'invention, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697) et L5 (WO95/02697). Selon un mode préféré de mise en oeuvre, l'adénovirus selon l'invention comprend une délétion dans les régions E1 et E4. Selon un autre mode de réalisation préféré, il comprend une délétion dans région E1 au niveau de laquelle sont insérés la région E4 et l'acide nucléique de l'invention (Cf FR94 13355). Dans les virus de l'invention, la délétion dans la région E1 s'étend préférentiellement des nucléotides 455 à 3329 sur la séquence de l'adénovirus Ad5.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN d'intérêt. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 203 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697.

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant une séquence nucléique de l'invention d'intérêt bordée de deux régions répétées inversées (ITR) d'AAV, et d'un plasmide portant les gènes d'encapsidation (gènes rep et cap) d'AAV. Une lignée cellulaire utilisable est par exemple la lignée 293. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants selon l'invention comportant un acide nucléique selon l'invention, un plasmide comportant notamment les LTR, la séquence d'encapsidation et ledit acide nucléique est construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

Pour la mise en oeuvre de la présente invention, il est tout particulièrement avantageux d'utiliser un adénovirus ou un rétrovirus recombinant défectif. Ces vecteurs possèdent en effet des propriétés particulièrement intéressantes pour le transfert de gènes dans les cellules tumorales.

### C3 - Vecteurs chimiques

Parmi les vecteurs synthétiques développés, on préfère utiliser dans le cadre de l'invention les polymères cationiques de type polylysine, (LKLK)n, (LKKL)n, polyéthylène immine et DEAE dextran ou encore les lipides cationiques ou lipofectants. Ils possèdent la propriété de condenser l'ADN et de promouvoir son association avec la membrane cellulaire. Parmi ces derniers, on peut citer les lipopolyamines (lipofectamine, transfectam, etc) différents lipides cationiques ou neutres (DOTMA, DOGS, DOPE, etc) ainsi que des peptides d'origine nucléaire. En outre, le concept de la transfection ciblée a été développé, médiée par un récepteur, qui met à profit le principe de condenser l'ADN grâce au polymère cationique tout en dirigeant la fixation du complexe à la membrane grâce à un couplage chimique entre le polymère cationique et le ligand d'un récepteur membranaire, présent à la surface du type cellulaire que l'on veut greffer. Le ciblage du récepteur à la transferrine, à l'insuline ou du récepteur des asialoglycoprotéines des hépatocytes a ainsi été décrit. La préparation d'un composition selon l'invention utilisant un tel vecteur chimique est réalisée selon toute technique connue de l'homme du métier, généralement par simple mise en contact des différents composants.

### Exemple D - Evaluation fonctionnelle des variants de p53

Les variants de p53 selon l'invention ont été évalués en test cellulaire pour les critères suivants:
- liaison à une séquence d'ADN double brin spécifique
- fonction transactivatrice
- activité antiproliférative
- activité apoptotique
- potentiel oncogénique associé à certaines mutations de p53

Les constructions utilisées plus particulièrement pour cette évaluation sont les constructions V-325, V-336 , V-343 et AS décrites dans l'exemple B.

### D1 Reconnaissance de séquences d'ADN double brin spécifiques par les molécules hybrides de l'invention

### D1.1 Production des molécules hybrides

Le cDNA de la p53 sauvage a été cloné dans le vecteur pBlueBacIII (Invitrogen) au site BamHI. Par insertion dans le plasmide pAcHLT-A (Pharmingen) du fragment contenant le cDNA de V325 obtenu par digestion du plasmide pEC114 par les enzymes EcoR I et Not I a été généré un vecteur permettant l'obtention d'un baculovirus recombinant ayant pour but l'expression d'une protéine V325 étiquetée en N-terminale par une séquence peptidique contenant entre autres un enchainement de 6 résidus histidine. A partir de ces vecteurs, des baculovirus recombinants ont été produits et purifiés suivant les instructions des fabricants (Invitrogen, Pharmingen). Les deux protéines ont été purifiées à homogénéité à partir d'extraits nucléaires de cellules d'insectes SF9 infectées par leur baculovirus respectif, les extraits nucléaires étant obtenus en suivant la procédure décrite par Delphin et al. (C. Delphin, Eur. J. Biochem., 223, 683-692, 1994).

La p53 sauvage est purifiée par immuno-affinité sur l'anticorps monoclonal pAb421 (Oncogene Sciences, Ab-1) suivant le protocole suivant: l'extrait nucléaire des cellules infectées est incubé 3 h à 4 °C avec un gel de protéine A-agarose sur lequel a été couplé covalemment l'anticorps pAb421. Après lavage extensif du gel par un tampon 50 mM TrisHCl pH 7,8 contenant 1 M KCl et des inhibiteurs de protéases, la protéine p53 est éluée par le peptide correspondant à l'épitope reconnu par cet anticorps sur p53 (KKGQSTSRHK), ce peptide étant utilisé à une concentration de 5 mg/ml dans la solution utilisée pour le lavage. Après concentration sur Centricon-30 (Amicon Grace), la p53 éluée est séparée du peptide et purifiée à homogénéité par perméation sur gel sur une colonne de Superose 6 HR10/30 équilibrée par 50 mM TrisHCl pH 7.5, 0,2 M NaCl, 0,1 mM EGTA, 0,1 mM ZnCl₂ , 10 mM DTT, 0,1 mM PMSF, 0,1 % NP-40, 5 % Glycérol. Les fractions contenant p53 sont aliquotées et immédiatement congelées à - 80 °C jusqu'à utilisation.

La protéine V325 étiquetée en N-terminale par une séquence peptidique contenant entre autres un enchainement de 6 résidus histidine appelée désormais HisV325 a été purifiée par une procédure adaptée de Hochuli et al. (Bio/Technology Vol 6 (1988) 1321). Avant d'être appliquée sur le gel de Nickel-NTA agarose, l'extrait nucléaire des cellules infectées est dessalé sur une colonne PD10 (Pharmacia) équilibrée en tampon 50 mM phosphate de sodium pH 8 contenant 5 mM β-mercaptoéthanol, 0,1 % NP-40 et un cocktail d'inhibiteurs de protéases. L'incubation de l'extrait nucléaire avec le gel de Nickel-NTA agarose a été réalisée dans ce tampon pendant 1 h à 4 °C sous agitation. Le gel est ensuite lavé extensivement par le même tampon à pH 6. La protéine HisV325 est éluée par 0,3 M imidazole dans ce dernier tampon après lavage du gel en 0,1 M imidazole. Les fractions contenant HisV325 sont aliquotées et immédiatement congelées à - 80 °C jusqu'à utilisation.

### D1.2 Construction de la séquence d'ADN double brin spécifique

La séquence d'ADN double brin spécifique utilisée dans cette expérience est constituée de deux oligonucléotides de synthèse dont la séquence est la suivante:

Ces deux oligonucléotides de synthèse ont été marqués au phosphore 33 par incubation de 30min à 37°C de 5 pmoles de chaque oligonucléotide dans 20 µl du milieu réactionnel suivant:

| | |
|---|---|
| Tris-HCl pH7,6 | 50 mM |
| MgCl₂ | 10 mM |
| dithiothréitol | 5 mM |
| Spermidine | 100 µM |
| EDTA | 100 µM |
| ATP-γ-³³P (Amersham) | 50µ Ci (1000-3000 Ci/mmole) |
| T4 kinase (Boehringer) | 10 U |

Puis les deux oligonucléotides ainsi marqués ont été hybridés en présence de 100 mM NaCl pour reconstituer la séquence double brin WAF-RE suivante contenant la séquence spécifique reconnue par p53 dans la région promotrice du gène WAF-1 (W.S. El-Deiry, Cell Vol75 (1993) 817):

D1.3 Reconnaissance de la séquence double brin WAF-RE par les molécules hybrides de l'invention.

Pour mettre en évidence une reconnaissance spécifique de la séquence double brin WAF-RE par les molécules hybrides de l'invention, des expériences de retard sur gel ont été réalisées sur le principe décrit ci-après. La réaction de liaison à l'ADN est effectuée dans 25 µl de milieu réactionnel (Tris-HCl 20 mM pH 7,5, 5 mM MgCl₂, 0,05 mM ZnCl₂, 5 mM dithiotreitol, 0,1 mg/ml BSA, 10 % glycérol, 1% Nonidet P-40, 0,1M NaCl, 2 µg/ml aprotinine, 2 µg/ml E-64, 2 µg/ml leupeptine, 2 µg/ml pepstatine) par addition de la séquence WAF-RE (2,4 10⁻⁹M) préparé selon l'exemple précédent, de 1,2 10 ⁶M de l'oligonucléotide compétiteur froid AP2 (Promega) utilisé pour éliminer la fixation non spécifique et de 30 ng de molécules hybrides à tester en présence ou non de p53 sauvage (entre 3 et 30 ng), la p53 sauvage pouvant être activée pour son activité de fixation spécifique à l'ADN par 300 ng d'anticorps pAb421 (T. R. Hupp, Cell Vol 71 (1992) 875). Les mélanges réactionnels sont incubés 30 minutes sur glace et les mélanges finaux sont soumis à une électrophorèse native sur gel de polyacrylamide à 4 % avec migration à 200V et 16°C. Le gel est ensuite séché et autoradiographié.

Le résultat d'une expérience représentative de compétition entre p53 sauvage et HisV325 en retard sur gel est présenté sur la Figure 6. Ce résultat montre que His-V325 reconnaît la séquence double brin WAF-RE avec une affinité comparable à celle de la p53 sauvage. On notera que HisV325 donne une bande majoritaire en gel retard qui migre plus vite que celle obtenue avec la p53 sauvage. Ceci pourrait indiquer que HisV325 se fixe sous forme de dimère. De plus, comme attendu, cette bande n'est ni déplacée, ni amplifiée par la présence de pAb421. Enfin, en l'absence de pAb421, la p53 sauvage fixe beaucoup moins de RE-WAF que ne le fait V325.

### D2 - Evaluation de la fonction transactivatrice

La fonction transactivatrice des constructions a été évaluée dans un système de transactivation in vivo dans les cellules SAOS-2 (ostéosarcome humain) déficientes pour les deux allèles de la protéine p53 (cellules accessibles à l'ATCC sous le numéro HTB85) et dans des lignées tumorales H358 (Maxwell & Roth, Oncogene 8 (1993), 3421) et HeLa (ATCC CCL 2). Ce sytème repose sur l'utilisation d'un gène rapporteur dosable enzymatiquement et placé sous la dépendance d'un promoteur contenant les motifs nucléotidiques de reconnaissance spécifique par la forme sauvage de la p53 (cf protocoles expérimentaux).

Dans ce test, le gène rapporteur est le gène CAT (chloramphénicol-acétyl transférase) et la séquence de reconnaissance par la p53 est la séquence consensus (p53RE) définie par Funk et collaborateurs (Mol. Cell. Biol. 12 (1992) 2866).

L'évaluation de cette fonction a été effectuée en comparaison avec celle de la protéine sauvage pour trois types de critères différents.

### D2.1 - Activité transactivatrice en dose réponse

Les cellules (3,5 10⁵) sont ensemencées dans des boites de Pétri de 6 cm de diamètre contenant 3 ml de milieu DMEM (Gibco BRL) additioné de 10% de sérum de veau foetal inactivé à la chaleur, et cultivées sur la nuit dans un incubateur à CO₂ (5%) à 37°C. Les différentes constructions sont alors transfectées en utilisant la lipofectAMINE (Gibco BRL) comme agent de transfection de la façon suivante: 3 µg de plasmide total sont incubés (dont 0,5 µg du plasmide reporter) avec 10 µl de lipofectAMINE pendant 30 min avec 3 ml de milieu Opti-MEM (Gibco BRL) sans sérum (mélange de transfection). Pendant ce temps, les cellules sont rincées deux fois au PBS puis incubées 4 h à 37°C avec le mélange de transfection, après quoi celui-ci est aspiré et remplacé par 3 ml de milieu DMEM additioné de 10% de sérum de veau foetal inactivé à la chaleur et les cellules remises à pousser pendant 48 h à 37°C.

### Protocole de dosage de l'activité CAT

48h après la transfection, les cellules sont lavées une fois en PBS puis gratées et récupérées dans 100 µl de tampon Tris 0,25 M pH 8 et à lysées par trois cycles de congélation - décongélation dans un bain éthanol/carboglace. L'extrait cellulaire total ainsi obtenu est mis à centrifuger 15 min à 10000 rpm et le surnageant récupéré pour le dosage de l'activité. Celui-ci est effectué en additionnant 20 µl d'extrait cellulaire à 130 µl d'un mélange réactionnel dont la composition finale est la suivante:
- Acétyl-Coenzyme A 0,4 mM
- Chloramphénicol, D-thréo-(dichloroacétyl-1,2-¹⁴C) 23 µM (200 nCi)
- Tris 0,18 M pH 8

Après 1 heure d'incubation à 37°C, les produits de la réaction sont extraits par 250 µl d'acétate d'éthyle dont 20 µl sont déposés sur une plaque de silice (chromatographie en couche mince) mise à migrer dans un mélange contenant 95% de chloroforme et 5% de méthanol. La plaque de chromatographie ainsi obtenue est enfin révélée à l'aide d'un instantimager (Packard instruments) qui permet de calculer le rapport des différents produits d'acétylation, rapport qui reflète l'activité de l'enzyme Chloramphénicol-Acétyl-Transférase et donc l'activité transactivatrice des différentes constructions.

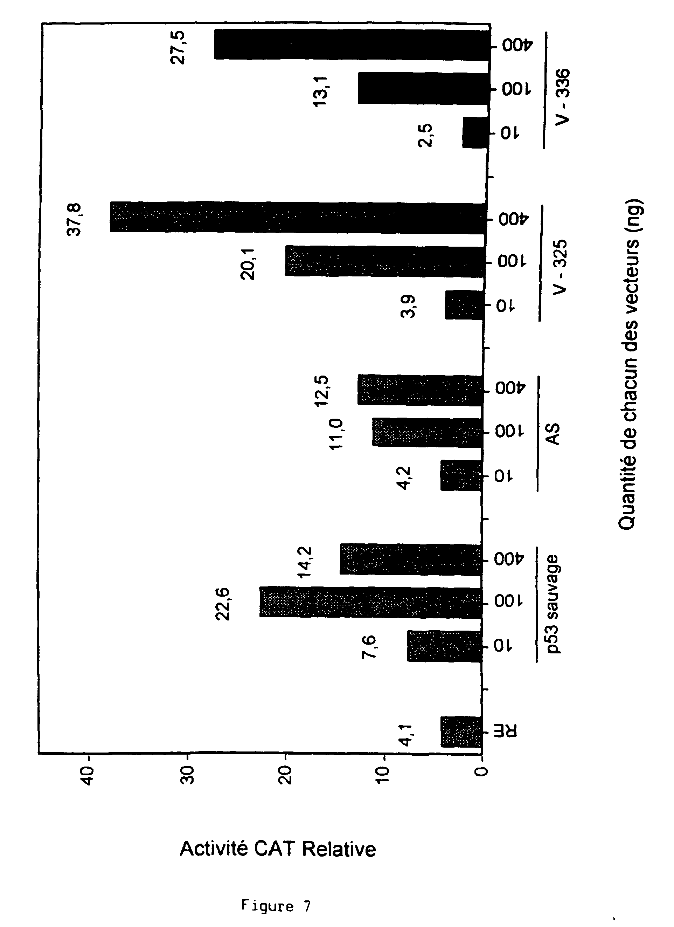

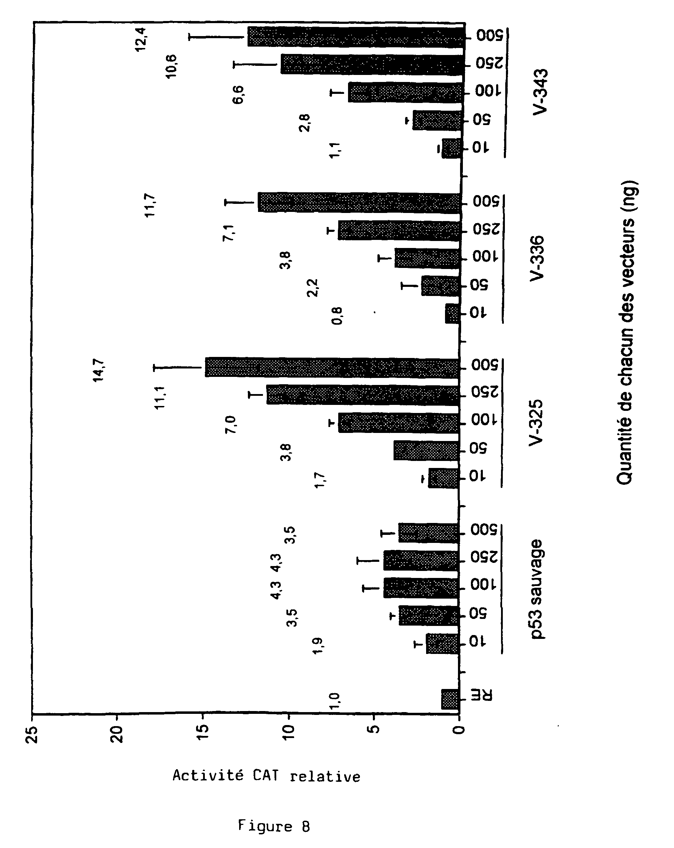

Les résultats obtenus dans la lignée SAOS-2 avec les constructions placées sous contrôle du promoteur CMV (pCDNA3) et présentés dans les Figures 7 et 8 montrent les propriétés suivantes pour chacune des constructions:
- la protéine p53 présente une activité dose-dépendante qui tend vers la saturation pour des doses élevées (à partir de 100ng de plasmide). Cette saturation peut traduire la nécessité de cofacteurs qui seraient limitants dans ces conditions.
- la protéine AS conserve la capacité d'activer la transcription de la protéine sauvage
- les protéines V-325, V-336 et V-343 présentent, tout comme la protéine p53, une activité transactivatrice qui ne semble pas quant à elle saturable aux fortes doses. Il est donc possible que cette absence apparente de saturation puisse conduire à une augmentation globale de l'activité. Il apparait en outre que la construction V-325 est plus active que ses homologues V-336 et V-343, ce qui suggère que les protéines chimères portant la région 75-325 sont particulièrement avantageuses.

Dans le but de confirmer ces propriétés une expérience similaire a été effectuée dans la lignée tumorale H 358 qui est tout comme la lignée SAOS-2, déficiente pour les deux allèles du gène p53. Dans cette expérience, chaque transfection a été effectuée avec 50 ng de chacune des construction placée sous dépendance du promoteur CMV. Les résultats présentés sur le Tableau 1 montrent clairement que les deux variants V-325 et V-336 présentent une activité transactivatrice améliorée par rapport à celle de la protéine p53 sauvage, avec de nouveau une meilleure activité du variant V-325.

**Tableau 1: Activité transactivatrice dans les cellules de la lignée tumorale H 358.**

| | pCDNA 3 | p53 sauvage | V-325 | V-336 |
|---|---|---|---|---|
| Activité CAT Relative | 1 | 6 | 25 | 16 |

Ces deux essais confirment que les variants de l'invention possèdent au moins une des propriétés de la p53 améliorée.

Afin de vérifier que cette différence d'activité n'est pas due à une différence d'expression mais bien à une activité accrue des variants de l'invention, le niveau d'expression de la protéine p53 sauvage et des variants V-325, V-336 et V-343 a été analysé dans les cellules SAOS-2. Pour ce faire les cellules sont transfectées par 3µg de chacun des plasmides utilisés dans l'expérience précédente, et récupérées 24 heures et 48 heures après la transfection. Après deux lavages en tampon PBS (Gibco BRL), les cellules sont lysées 15 minutes à 4°C dans 50µl de tampon RIPA (Tris-Hcl 10 mM pH 8,0, 150 mM NaCl, 1 mM EDTA, 1%,Nonidet-P40, 1% déoxycholate de sodium, 0,1% dodécyl sulfate de sodium) additionné de 2 mM PMSF, 20 µg/ml aprotinine, 2 µg/ml E64, 2 µg/ml leupeptine et 2 µg/ml pepstatine. Après 15 min de centrifugation à 15000 rpm, les surnageants sont prélevés, additionnés de tampon de migration (Laemmli U.K., Nature, 227, 680-685, 1970) et soumis à électrophorèse sur gel de polyacrylamide à 10% en milieu dénaturant à 200V suivant le protocole précédemment décrit (Laemmli U.K., Nature, 227, 680-685, 1970). Puis les protéines sont transférées sur membrane de PVDF (NEN Research Products) en utilisant le système de transfert semi-sec NOVEX suivant les recommandations du frabiquant, et révélées à l'aide de l'anticorps monoclonal pAb 240 (Oncogene Sciences, Ab-3) et d'un anticorps secondaire (lapin anti-souris) couplé à la peroxydase (Nordic Immunology) en utilisant le kit ECL (Amersham).

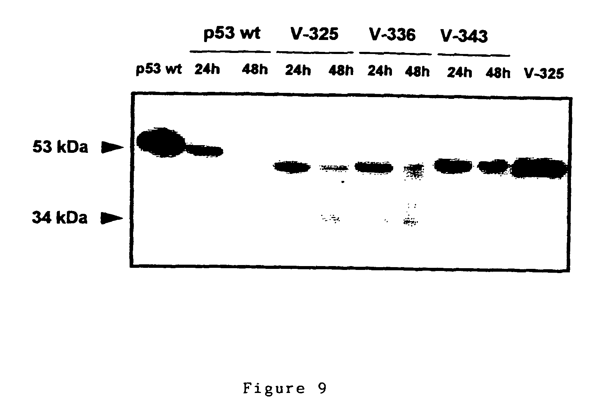

Le résultat de cette expérience présenté dans la Figure 9 montre que les variants V-325 et V-336 sont exprimés à un niveau comparable à celui de la protéine p53 sauvage et que le variant V-343 semble légèrement mieux exprimé que les précédents. De plus la comparaison des niveaux d'expression à 24 et 48 heures semble indiquer que la stabilité relative de chacune des constructions est similaire. Ce résultat montre donc que l'activité accrue des variants de l'invention V-325 et V-336 n'est pas due à une meilleure expression mais probablement à un potentiel d'activateur transcriptionnel accru, contrairement au variant V-343.

Par la suite, et dans le but de confirmer cette capacité des variants de l'invention d'activer un gène placé sous la dépendance d'un élément de reconnaissance de la p53 sauvage, l'étude de l'activation de gènes endogènes a été effectuée en regardant l'expression des gènes hdm2 et WAF1, normalement induits par la protéine p53.

Cette expérience a été effectuée dans la lignée cellulaire EB (cancer du colon) déficiente pour les deux allèles codant pour la protéine p53 (Shaw et al., PNAS 89 (1992) 4495). Un clone stable exprimant la protéine p53 sous contrôle du promoteur inductible de la métallothionéine a été construit à partir de cette lignée (clone EB-1 (Shaw et al., PNAS 89 (1992) 4495)). De la même façon un autre clone stable exprimant la protéine V-325 sous contrôle du promoteur inductible de la métallothionéine a été construit en utilisant un plasmide dérivé du vecteur pmIMT1i (obtenu de P. Shaw) par insertion du cDNA codant pour la protéine V-325 aux sites EcoR I - Not I (pmIMT1i-V325). Pour ce faire, les cellules EB (3.5 10⁵ cellules) ont été transfectées par 1,3 µg du plasmide pmIMT1i-V325 et 200 ng de plasmide pcDNA3 suivant le protocole précédement décrit et les clones stables ont été sélectionés après transfection par croissance dans un milieu contenant 800µg/ml de généticine. Un clone exprimant V-325 de façon inductible à un niveau comparable à l'expression de la protéine p53 dans le clone EB-1 a été sélectioné (clone EB-V325).

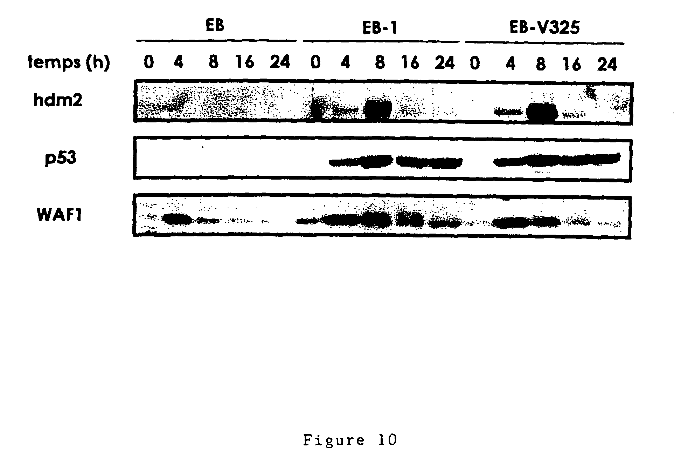

Les clones EB-1 et EB-V325 ainsi que les cellules parentales EB (10⁶ cellules) ont été soumis à un traitement au ZnCl₂ (200 µM) et des extraits cellulaires ont été efféctués à différents temps et soumis à électrophorèse et transfert sur membrane comme décrit précédement. Les protéines transférées ont été révélées par trois anticorps différents; l'anticorps monoclonal pAb240 dirigé contre la protéine p53, et deux anticorps polyclonaux dirigés l'un contre la protéine hdm2 et l'autre contre la protéine WAF1. Les résultats de cette expérience présentés dans la Figure 10, montrent que: 1) la protéine p53, absente dans les cellules EB, EB-V325 et EB-1 en l'absence d'induction, est exprimée dans le clone EB-1 dès 4 heures après le début du traitement au zinc, et le variant V-325 est exprimé de la même façon dans le clone EB-V325, 2) la protéine WAF1 dont l'expression semble être induite dans les cellules EB par le traitement au zinc 4 heures après le début de celui-çi, voit son expression prolongée jusqu'à 16 heures dans les clones EB-1 et EB-V325, et 3) l'induction de la protéine hdm2 n'est observable que dans les clones EB-1 et EB-V325, avec une expression accrue dans le clone EB-V325.

Ces résultats montrent que l'activation de la transcription par le variant V-325 se traduit par l'induction de l'expression de gènes normalement induits par la protéine p53 sauvage, et que ce variant présente bien une activité accrue dans un contexte physiologique par rapport à la protéine p53 sauvage.

### D2.2 - Effet de la protéine E6 (HPV18) sur la fonction transactivatrice

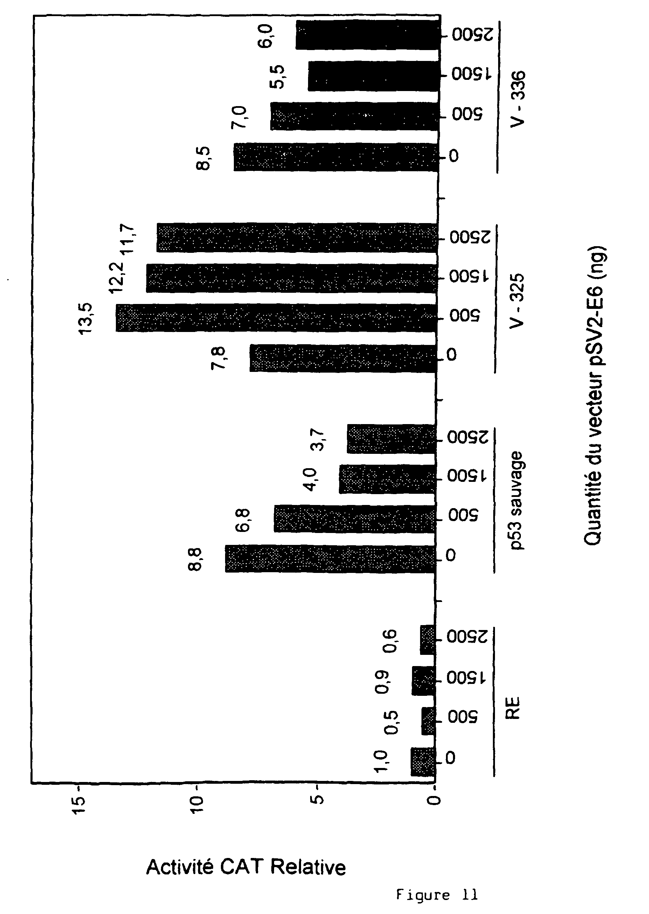

Les protocoles utilisés sont identiques à ceux décrits dans l'exemple D1.1. Dans cette expérience de transfection, les constructions placées sous contrôle du promoteur CMV (pCDNA3) ont été co-transfectées avec des concentrations croissantes d'un plasmide exprimant E6 sous contrôle du promoteur SV40 (pSV2). Les résultats obtenus dans la lignée SAOS-2 et présentés dans la Figure 11 montrent les propriétes suivantes pour chacune des constructions:
- l'activité de la p53 décroit au fur et à mesure que l'on augmente la concentration de E6, cette décroissance étant très probablement le reflet de la dégradation de la p53 induite par E6.
- la protéine V-336 présente une absence de sensibilité à E6.
- la protéine V-325 semble pouvoir être légèrement activée par E6. La protéine V-325 est toujours, et dans toutes les situations observées, plus active que la p53. Pour confirmer cette différence de comportement vis-à-vis de la protéine E6, l'activité transactivatrice des constructions V-325 et V-336 dans des cellules HPV18 positives (HeLa) et donc exprimant la protéine E6, a été testée et comparée à celle de la p53 sauvage.

Dans cette expérience de transfection effectuée selon le protocole décrit précédemment, les différentes constructions ont été placées sous contrôle du promoteur CMV (pCDNA3).

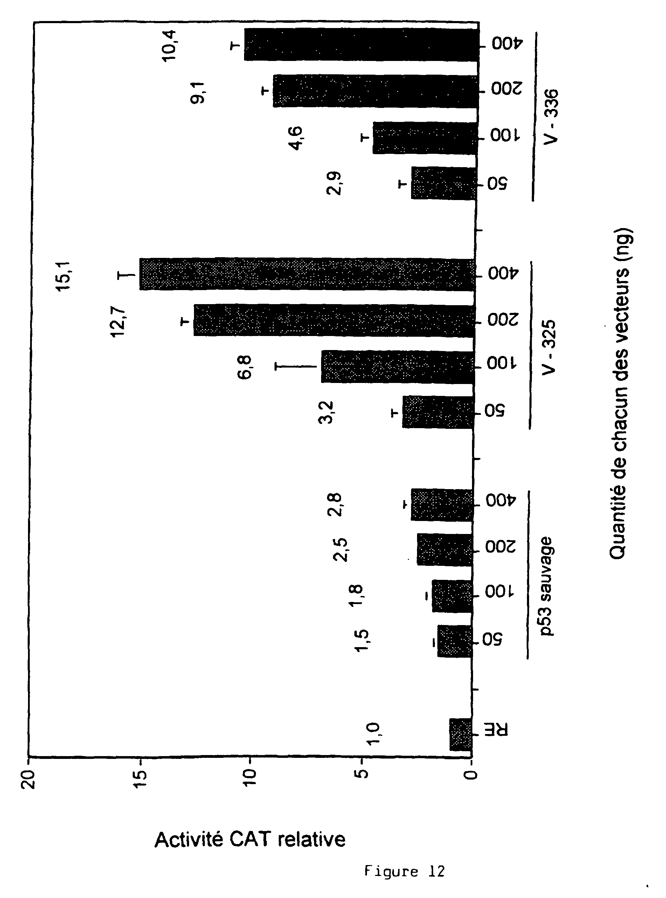

Les résultats présentés dans la Figure 12 montrent une très nette activité transcriptionnelle des deux constructions V-325 et V-336, dans un contexte ou la protéine p53 sauvage est très peu active, laissant supposer à nouveau que ces deux constructions sont insensibles à E6 contrairement à la protéine sauvage.

Pour tester si cette absence de sensibilité à la protéine E6 est le reflet d'une meilleure stabilité en réponse à la dégradation induite par cette protéine, une expérience de dégradation *in vitro* a été effectuée.

Les différentes molécules utilisées dans cette expérience ont été obtenues par traduction in vitro en lysat de réticulocytes des molécules décrites dans l'exemple C1 (vecteur pcDNA3) en utilisant le kit TNT Coupled Reticulocyte lysate Systems (Promega) suivant le protocole expérimental décrit par le fournisseur pour un volume réactionnel total de 50 µl.

Pour cette expérience, les molécules hybrides de l'invention V-325 et V-336 ainsi que la protéine p53 sauvage sont produites par traduction *in vitro* en présence de 44 µCi de ³⁵S-methionine (Amersham) (1175 Ci/mmole) pour générer ces molécules hybrides radioactivement marquées. La protéine E6 (HPV18), quand à elle est produite dans les mêmes conditions mais en absence de ³⁵S-methionine.

Puis 2 µl de chacun des produits radiomarqués (p53, V-325 et V-336) sont mis à incuber à 30°C avec 2 µl de protéine E6 non radiomarquée et 10 µl de lysat de réticulocyte dans un volume final de 40 µl de tampon Tris-HCl 25mM, pH 7,5, 100 mM NaCl, 3 mM DTT. La réaction est ensuite arrétée à différents temps par prélèvement de 7,5 µl du milieu réactionnel et addition de 7,5 µl de tampon de migration (Laemmli U.K., Nature, 227, 680-685, 1970) et les échantillons ainsi préparés sont soumis à électrophorèse sur gel de polyacrylamide à 10% en milieu dénaturant à 200 V suivant le protocole précédemment décrit (Laemmli U.K., Nature, 227, 680-685, 1970). Le gel est ensuite séché et révélé à l'aide d'un instantimager (Packard instruments) qui permet d'estimer les quantités de variants de l'invention n'ayant pas été dégradés au cours de la réaction.

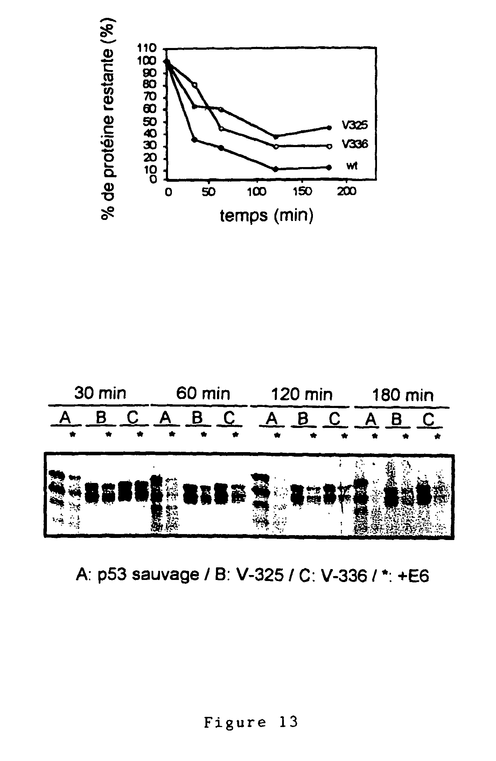

Le résultat de cette expérience présenté sur la figure 13 montre clairement que les variants V-325 et V-336 sont beaucoup plus résistants que la protéine p53 sauvage à la dégradation induite par E6, avec de nouveau de meilleures propriétés pour le variant V-325 en terme de resistance à la dégradation. Ces résultats reflètent bien les différences de sensibilité de la protéine p53 sauvage et des variants V-325 et V-336 à la protéine E6 observés au niveau de l'activité transcriptionnelle dans les expériences précédentes (Figures 11 et 12).

Ce comportement fait de ces deux constructions des candidats super-sauvage particulièrement avantageux pour le traitement de pathologies liées à l'infection par HPV16 ou HPV18.

D2.3 - Effet d'un mutant p53 dominant-négatif sur la fonction transactivatrice

Dans cette expérience, le mutant H175, décrit comme dominant oncogénique et dominant négatif vis à vis de la protéine p53 sauvage, a été utilisé. Dans cette expérience de transfection effectuée selon le protocole décrit précédemment, les différentes constructions ainsi que le mutant H175 ont été placés sous contrôle du promoteur CMV (pCDNA3). Chacune des constructions a été co-transfectée avec des concentrations croissantes du plasmide exprimant le mutant H175.

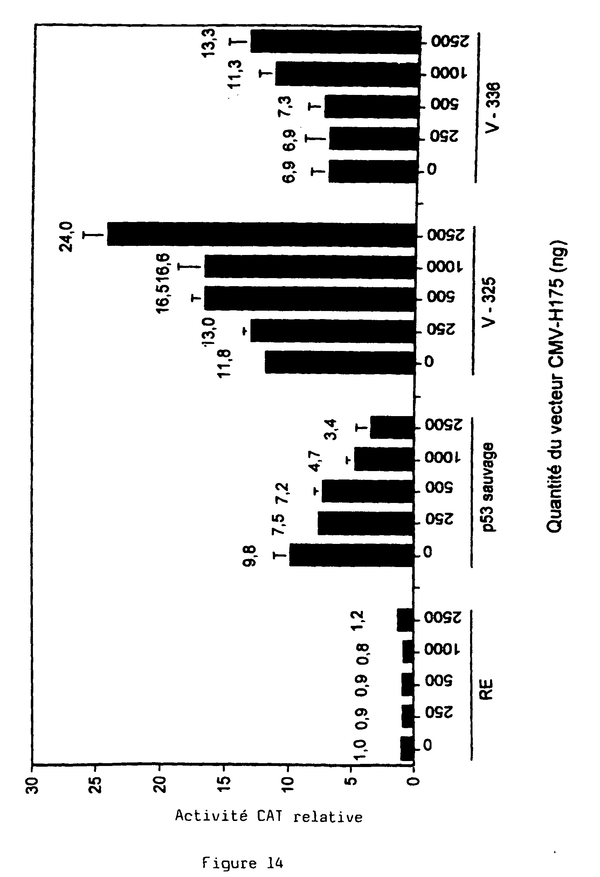

Les résultats présentés dans la Figure 14 montrent les propriétes suivantes pour chacune des constructions:
- la protéine p53 voit son activité transactivatrice diminuer lorsqu'elle est en présence d'un excès de forme mutée H175, ce qui correspond bien à une situation physiologique puisque l'on sait que ce type de forme mutante est beaucoup plus stable que la p53 sauvage et donc toujours en excès. On mesure donc bien là l'effet dominant-négatif de ce mutant.
- la protéine AS présente une sensibilité accrue à l'effet dominant négatif du mutant H175, puisque sensible à des concentrations plus faibles de celui-ci.
- au contraire, les protéines V-325 et V-336 ne sont non seulement plus sensibles à l'effet dominant-négatif mais voient même leur activité augmentée de façon dose-dépendante en présence de la forme mutante H175. De nouveau, dans cet essai la protéine V-325 présente un effet accru par rapport à la protéine V-336 la confirmant un peu plus dans son possible statut de super-sauvage.

### D2.4 - Effet de la protéine hdm2 sur la fonction transactivatrice

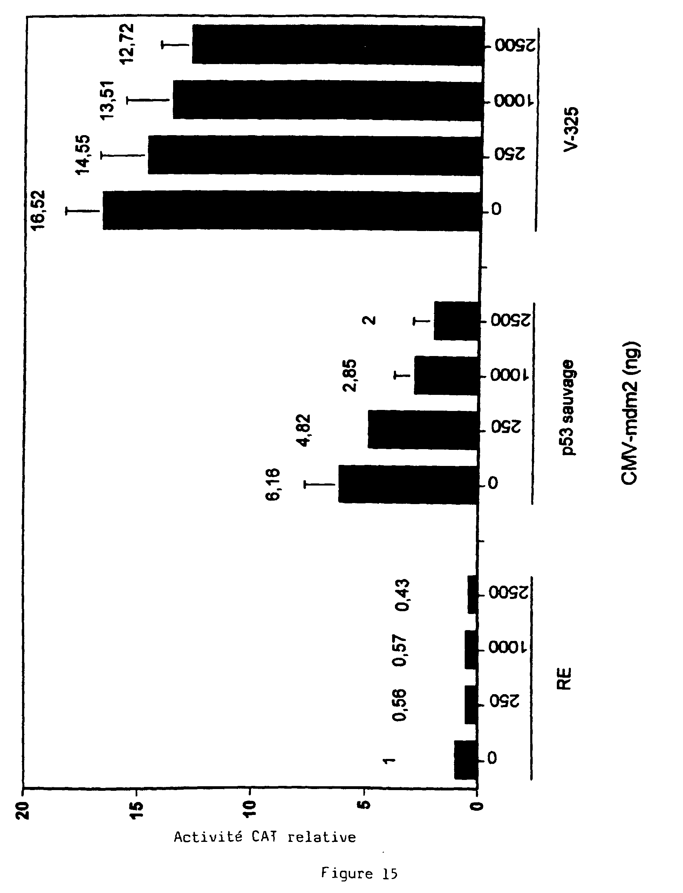

Les protocoles utilisés sont identiques à ceux décrits dans l'exemple D1.1. Dans cette expérience de transfection, les constructions placées sous contrôle du promoteur CMV (pcDNA3) sont co-transfectées avec des concentrations croissantes d'un plasmide exprimant hdm2 (fragment 1-134) sous contrôle du promoteur CMV (pcDNA3). Les résultats obtenus dans la lignée SAOS-2 et présentés dans la Figure 15 montrent les propriétes suivantes pour chacune des constructions:
- l'activité de la protéine p53 décroit au fur et à mesure que l'on augmente la concentration de hdm2, ce qui correspond bien à une situation physiologique.
- la protéine V-325 semble être insensible à cette inhibition par hdm2.

Ce comportement fait de la protéine V-325 un candidat super-sauvage particulièrement avantageux pour le traitement de pathologies liées à a surexpression de hdm2, et plus particulièrement, au traitement des pathologies liées à la surexpression de protéines cellulaires interagissant avec le domaine N-terminal de la protéine p53.

Les résultats de ces quatre expériences montrent clairement que les variants selon l'invention, notamment les variants contenant la région 75-325-lz ou 75-336-lz, présentent 1) une activité transactivatrice accrue, 2) une sensibilité moindre à l'effet de la protéine E6 de HPV18, 3) l'absence de sensibilité à l'effet dominant-négatif de certains mutants de la p53 et même l'accroissement de son activité dans un tel contexte, et 4) une absence de sensibilité à la protéine hdm2. Ces différentes propriétés sont tout à fait remarquables et inattendues et confèrent aux variants de l'invention des avantages thérapeutiques considérables.

### D3 - Effet sur la croissance cellulaire

L'effet des constructions V-325 et V-336 sur la croissance cellulaire a été testé en parallèle avec la p53 sur différents types de lignées cellulaires dans une expérience de formation de colonies résistantes à la néomycine suite à la transfection par des plasmides exprimant ces trois protéines.

Dans cette expérience de transfection effectuée selon le protocole décrit précédemment, les différentes constructions ont été placées sous contrôle du promoteur CMV (pCDNA3).

### Protocole de formation de colonies résistantes à la néomycine

48h après transfection, les cellules sont gratées et transferées dans des boites de Pétri de 10 cm de diamètre et remises à pousser avec 10 ml de milieu DMEM additioné de 10% de sérum de veau foetal inactivé à la chaleur et contenant 400 µg/ml de généticine (G418). Suite à une séléction de 15 jours en présence de G418, le nombre de colonies Neo^{R} est déterminé par comptage après coloration à la fuchsine.

Cette expérience a été effectuée sur différents types cellulaires dont le statut des protéines p53 et Ras est présenté dans le Tableau 2

**Tableau 2: Statut des lignées cellulaires utilisées dans le test de formation de colonies Néo^{R}**

| lignée | p53 | Ras | surexpression hdm2 | n° ATCC |
|---|---|---|---|---|
| SAOS-2 | - / - | ? | - | HTB 85 |
| HCT 116 | ? | Ki-Ras muté | - | CCL 247 |
| H 322 | L 248 | ? | - | (*) |
| H 460 | sauvage | Ki-Ras muté | - | HTB 177 |
| HeLa | sauvage | ? | - | CCL 2 |
| OsA-CL | ? | ? | + | (**) |

| | | | | |
|---|---|---|---|---|
| (*) Putnam et al., Surg. Oncol., 1 (1993), 49 | | | | |
| (**) Oliner et al., Nature, 358, (1992), 80 | | | | |

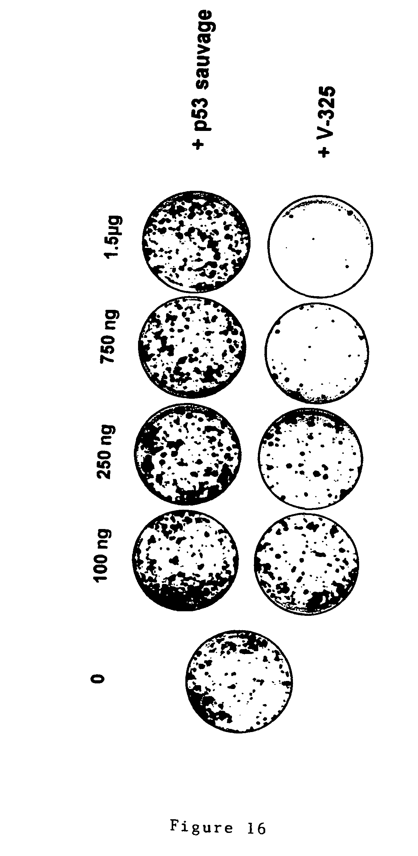

Les résultats de ces expériences sont présentés dans le Tableau 3 et sur la Figure 16.

**Tableau 3: Formation de colonies Néo^{R}**

| lignée | vecteur | p53 sauvage | V-325 | V-336 |
|---|---|---|---|---|
| SAOS-2 | 253 | 17 | 12 | 13 |
| HCT116 | 112 | 62 | 58 | 61 |
| H 322 | 93 | 5 | 2 | 3 |
| H 460 | 153 | 110 | 87 | 92 |
| HeLa | 172 | 151 | 31 | 47 |

Ces résultats montrent que les constructions V-325 et V-336 possèdent la capacité de bloquer la croissance cellulaire de façon au moins aussi efficace que la protéine p53 sauvage dans des contextes cellulaire où celle-ci peu fonctionner normalement (p53 sauvage ou double délétant), mais surtout qu'elles conservent cette activité même dans des contextes cellulaires ou la protéine p53 sauvage est très peu active (cellules HeLa exprimant la protéine E6 de HPV18 et cellules OsA-CL présentant une surexpression de la protéine hdm2). Cette propriété confère aux variants de l'invention des avantages thérapeutiques considérables.

### D4 - Activité apoptotique des variants de l'invention

L'activité apoptotique des variants de l'invention a été étudiée en utilisant les cellules EB, EB-1 et EB-V325 et les conditions d'induction précédement décrites (exemple D1).

Les cellules ainsi induites (10⁶ cellules) sont fixées et perméabilisées par une incubation de 40 minutes dans 1 ml de Permeafix (Ortho Diagnostic Systems Inc.), puis lavées deux fois en tampon A (PBS (Gibco BRL) additionné de 0,5% de Tween 20), avant d'être resuspendues et incubées une heure à température ambiante dans 100 µl de tampon A additionné de 2% BSA (PBS-BSA) et 1 µg de l'anticorps monoclonal pAb240. Après deux nouveaux lavages en tampon PBS-BSA, les cellules sont incubées 1 heure à température ambiante dans 100 µl du même tampon additioné de 1 µg d'un anticorps polyclonal secondaire couplé à la fluorescéine (GAM-FITC (Immunotech)). Puis, les cellules sont lavées deux fois dans le tampon A, resuspendues dans 1 ml du même tampon contenant 5 µg d'iodure de propidium et 1 mg de RNase (DNase-free), et incubées 30 min à température ambiante avant d'être analysées par cytométrie de flux.

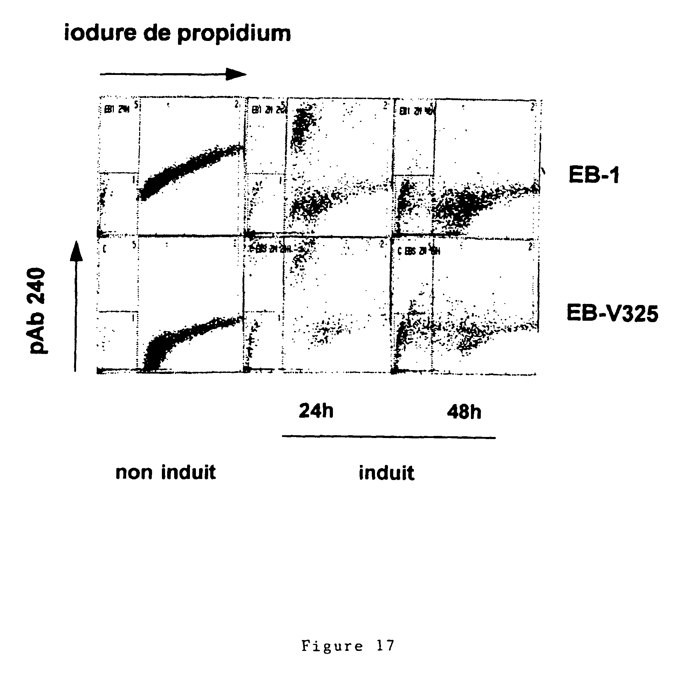

Les résultats d'une expérience d'induction de 24 et 48 heures effectuée sur les cellules EB-1 et EB-V325 sont présentés dans la Figure 17. Dans ces conditions les cellules exprimant la protéine p53 sauvage ou son variant V-325 (détectés par l'anticorps pAb240) sont majoritairement réparties en phase G1 et sub-G1 (apoptose) après 24 heures d'induction, puis essentiellement en sub-G1 après 48 heures. Ce résultat indique clairement que la protéine V-325 est capable, tout comme la protéine p53 sauvage, d'induire l'apoptose.

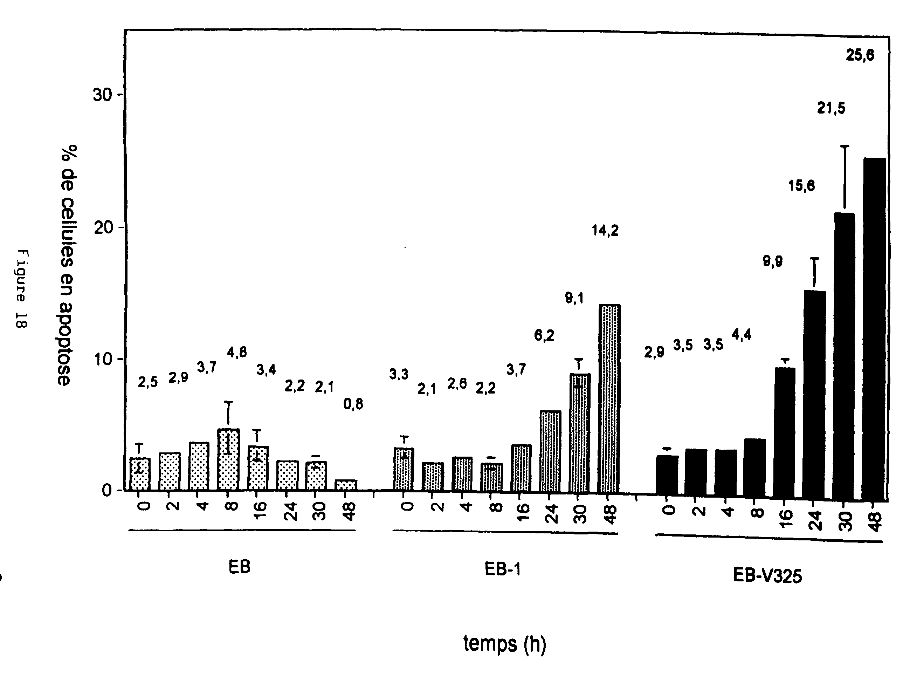

Les résultats d'une expérience cinétique d'induction effectuée sur les cellules EB et les clones EB-1 et EB-V325 présentés dans la figure 18 montrent que le variant V-325 semble induire plus rapidement et plus massivement l'apoptose que la protéine p53 sauvage. En tenant compte du fait que les deux protéines semblent être exprimées à des niveaux comparables dans ces clones (cf § D1), ce résultat conforte l'idée d'une activité améliorée du variant V-325 par rapport à celle de la protéine p53 sauvage.

## Revendications

1. Variant de la protéine p53 dans lequel :
(a) une partie du domaine d'oligomérisation ainsi que tout le domaine de régulation sont délétés et remplacés par un domaine leucine zipper artificiel **caractérisé en ce que** la délétion de la partie C-terminale est effectuée à partir du résidu 326 ou à partir du résidu 337 par référence à la protéine p53 humaine et
(b) tout ou partie du domaine transactivateur est délété et remplacé par le domaine transactivateur de VP16.

2. Variant selon la revendication 1 **caractérisé en ce que** le domaine leucine zipper artificiel est un domaine non présent à l'état naturel assurant une sélectivité d'oligomérisation.

3. Variant selon la revendication 2 **caractérisé en ce que** le domaine leucine zipper possède la séquence SEQ ID n° 1.

4. Variant selon l'une des revendications 1 à 3 **caractérisé en ce que** le résidu arginine en position 182 de la protéine p53 est remplacé par une histidine.

5. Variant selon l'une des revendications précédentes **caractérisé en ce qu'**il comporte une délétion des résidus 1 à 74.

6. Variant selon la revendication 5, **caractérisé en ce que** le domaine transactivateur comporte la séquence SEQ ID n° 2.

7. Composé V-325 de séquence SEQ ID n° 25 et son variant V-325H comportant une histidine en position 182 de la p53.

8. Composé V-336 de séquence SEQ ID n° 26 et son variant V-336H comportant une histidine en position 182 de la p53.

9. Acide nucléique codant pour un variant ou une protéine chimère selon l'une des revendications 1 à 8.

10. Acide nucléique selon la revendication 9, **caractérisé en ce qu'**il s'agit d'un ADNc, d'un ARN, d'un acide synthétique ou semi-synthétique.

11. Acide nucléique selon la revendication 9, **caractérisé en ce qu'**il est choisi parmi les acides nucléiques de séquence SEQ ID n° 25 et 26.

12. Cassette d'expression comprenant un acide nucléique selon la revendication 9, un promoteur permettant son expression et un signal de terminaison de la transcription.

13. Vecteur comprenant un acide nucléique selon la revendication 9 ou une cassette selon la revendication 12.

14. Vecteur selon selon la revendication 13, **caractérisé en ce qu'**il s'agit d'un vecteur viral.

15. Vecteur selon selon la revendication 14, **caractérisé en ce qu'**il s'agit d'un adénovirus recombinant défectif.

16. Vecteur selon selon la revendication 14, **caractérisé en ce qu'**il s'agit d'un rétrovirus recombinant défectif.

17. Vecteur selon selon la revendication 14, **caractérisé en ce qu'**il s'agit d'un AAV recombinant défectif.

18. Vecteur selon selon la revendication 14, **caractérisé en ce qu'**il s'agit d'un HSV recombinant défectif.

19. Vecteur selon selon la revendication 13, **caractérisé en ce qu'**il s'agit d'un vecteur chimique ou biochimique.

20. Composition pharmaceutique comprenant un acide nucléique et ou un vecteur selon l'une des revendications 10 à 19.

21. Composition pharmaceutique comprenant un variant ou une protéine chimère selon l'une des revendications 1 à 9.

22. Composition pharmaceutique selon l'une des revendications 20 ou 21 pour le traitement des désordres hyperprolifératifs.

## Claims

1. Variant of the p53 protein in which
(a) part of the oligomerization domain and all of the regulatory domain are deleted and replaced by an artificial leucine zipper domain **characterized in that** the deletion of the C-terminal part is carried out from residue 326 or from residue 337 with reference to the human P53 protein, and
(b) all or part of the transactivating domain is deleted and replaced by the transactivating domain of VP16.

2. Variant according to Claim 1, **characterized in that** the artificial leucine zipper domain is a domain not present in the natural state ensuring selectivity of oligomerization.

3. Variant according to Claim 2, **characterized in that** the leucine zipper domain has the sequence SEQ ID No. 1.

4. Variant according to one of Claims 1 to 3, **characterized in that** the arginine residue in position 182 of the p53 protein is replaced by a histidine.

5. Variant according to one of the preceding claims, **characterized in that** it comprises a deletion of residues 1 to 74.

6. Variant according to Claim 5, **characterized in that** the transactivating domain comprises the sequence SEQ ID No. 2.

7. Compound V-325 of sequence SEQ ID No. 25 and its variant V-325H comprising a histidine in position 182 of p53.

8. Compound V-336 of sequence SEQ ID No. 26 and its variant V-336H comprising a histidine in position 182 of p53.

9. Nucleic acid encoding a variant or a chimeric protein according to one of Claims 1 to 8.

10. Nucleic acid according to Claim 9, **characterized in that** it is a cDNA, an RNA, a synthetic or semisynthetic acid.

11. Nucleic acid according to Claim 9, **characterized in that** it is chosen from the nucleic acids of sequence SEQ ID No. 25 and 26.

12. Expression cassette comprising a nucleic acid according to Claim 9, a promoter allowing its expression and a signal for termination of transcription.

13. Vector comprising a nucleic acid according to Claim 9 or a cassette according to Claim 12.

14. Vector according to Claim 13, **characterized in that** it is a viral vector.

15. Vector according to Claim 14, **characterized in that** it is a defective recombinant adenovirus.

16. Vector according to Claim 14, **characterized in that** it is a defective recombinant retrovirus.

17. Vector according to Claim 14, **characterized in that** it is a defective recombinant AAV.

18. Vector according to Claim 14, **characterized in that** it is a defective recombinant HSV.

19. Vector according to Claim 13, **characterized in that** it is a chemical or biochemical vector.

20. Pharmaceutical composition comprising a nucleic acid and or a vector according to one of Claims 10 to 19.

21. Pharmaceutical composition comprising a variant or a chimeric protein according to one of Claims 1 to 19.

22. Pharmaceutical composition according to either of Claims 20 and 21, for the treatment of hyperproliferative disorders.

## Patentansprüche

1. Variante des Proteins p53, in der
(a) ein Teil der Oligomerisationsdomäne sowie die Regulationsdomäne deletiert und durch eine künstliche Leucin-Zipper-Domäne ersetzt ist, **dadurch gekennzeichnet, dass** die Deletion des C-terminalen Teils ab dem Rest 326 oder ab dem Rest 337 bezüglich des humanen Proteins p53 erfolgt ist, und
(b) die Transaktivatordomäne ganz oder teilweise deletiert und durch die Transaktivatordomäne von VP16 ersetzt ist.

2. Variante nach Anspruch 1, **dadurch gekennzeichnet, dass** die künstliche Leucin-Zipper-Domäne eine Domäne ist, die im natürlichen Zustand nicht vorhanden ist und eine Oligomerisationsselektivität sicher stellt.

3. Variante nach Anspruch 2, **dadurch gekennzeichnet, dass** die Leucin-Zipper-Domäne die Sequenz SEQ ID NO: 1 besitzt.

4. Variante nach einem der Ansprüche 1 bis 3, **dadurch gekennzeichnet, dass** der Agininrest in Position 182 des Proteins p53 durch ein Histidin ersetzt ist.

5. Variante nach einem der vorangehenden Ansprüche, **dadurch gekennzeichnet, dass** sie eine Deletion der Reste 1 bis 74 enthält.

6. Variante nach Anspruch 5, **dadurch gekennzeichnet, dass** die Transaktivatordomäne die Sequenz SEQ ID NO: 2 enthält.

7. Verbindung V-325 mit der Sequenz SEQ ID NO: 25 und ihre Variante V-325H, die ein Histidin in Position 182 des p53 enthält.

8. Verbindung V-336 mit der Sequenz SEQ ID NO: 26 und ihre Variante V-336H, die ein Histidin in Position 182 des p53 enthält.

9. Nucleinsäure, die für eine Variante oder ein chimäres Protein nach einem der Ansprüche 1 bis 8 codiert.

10. Nucleinsäure nach Anspruch 9, **dadurch gekennzeichnet, dass** es sich um eine cDNA, eine RNA, eine synthetische oder halbsynthetische Säure handelt.

11. Nucleinsäure nach Anspruch 9, **dadurch gekennzeichnet, dass** sie unter den Nucleinsäuren der Sequenz SEQ ID NO: 25 und 26 ausgewählt ist.

12. Expressionskassette, die eine Nucleinsäure nach Anspruch 9, einen Promotor, der ihre Expression erlaubt und ein Terminationssignal der Transkription umfasst.

13. Vektor, der eine Nucleinsäure nach Anspruch 9 oder eine Kassette nach Anspruch 12 umfasst.

14. Vektor nach Anspruch 13, **dadurch gekennzeichnet, dass** es sich um einen viralen Vektor handelt.

15. Vektor nach Anspruch 14, **dadurch gekennzeichnet, dass** es sich um ein defektes rekombinantes Adenovirus handelt.

16. Vektor nach Anspruch 14, **dadurch gekennzeichnet, dass** es sich um ein defektes rekombinantes Retrovirus handelt.

17. Vektor nach Anspruch 14, **dadurch gekennzeichnet, dass** es sich um ein defektes rekombinantes AAV handelt.

18. Vektor nach Anspruch 14, **dadurch gekennzeichnet, dass** es sich um ein defektes rekombinantes HSV handelt.

19. Vektor nach Anspruch 13, **dadurch gekennzeichnet, dass** es sich um einen chemischen oder biochemischen Vektor handelt.

20. Pharmazeutische Zusammensetzung, die eine Nucleinsäure und/oder einen Vektor nach einem der Ansprüche 10 bis 19 umfasst.

21. Pharmazeutische Zusammensetzung, die eine Variante oder ein chimäres Protein nach einem der Ansprüche 1 bis 9 umfasst.

22. Pharmazeutische Zusammensetzung nach einem der Ansprüche 20 oder 21 für die Behandlung von hyperproliferativen Störungen.
